# EUROPEAN PATENT APPLICATION

(11) **EP 2 157 090 A1**
(43) Date of publication of application: **24.02.2010**
(21) Application number: 08765775.5
(22) Date of filing: 20.06.2008
(51) Int. Cl.: C07D 403/12, A61K 31/497, A61K 31/501, A61P 3/04, A61P 3/10, A61P 43/00, C07D 401/14, C07D 403/14, C07D 413/14, C07D 417/14

(54) **PYRAZINAMIDE COMPOUND**

(30) Priority: 21.06.2007 JP 2007163860
(71) Applicant: Taisho Pharmaceutical Co. Ltd., Tokyo 170-8633 (JP); Nissan Chemical Industries, Ltd., Tokyo 101-0054 (JP)
(72) Inventor: UMEMIYA, Hiroki, Tokyo 170-8633 (JP); WATATANI, Kengo, Tokyo 170-8633 (JP); KAWAGUCHI, Takanori, Tokyo 170-8633 (JP); KAWABE, Kenichi, Tokyo 170-8633 (JP); OKADA, Takumi, Funabashi-shi Chiba 274-8507 (JP); SASAKO, Shigetada, Funabashi-shi Chiba 274-8507 (JP); SAITO, Noriko, Funabashi-shi Chiba 274-8507 (JP)
(74) Representative: HOFFMANN EITLE
(86) International application number: PCT/JP2008/061355
(87) International publication number: WO 2008/156174

(57) **Abstract**

Disclosed is a pyrazinamide compound represented by the formula (1), a tautomer, stereoisomer or pharmaceutically acceptable salt thereof, or a solvate of the compound or the tautomer, stereoisomer or pharmaceutically acceptable salt thereof, which has an excellent GK-activating activity and is therefore useful as a medicinal agent. (1) wherein R¹ represents a hydrogen atom, a C₁₋₈ alkyl group, a C₃₋₈ cycloalkyl group, a C₂₋₉ heterocyclyl group, a phenyl group, a C₁₋₉ heteroaryl group, a C₇₋₁₄ arylalkyl group or the like; R² represents a C₁₋₈ alkyl group, a C₂₋₉ heterocyclyl group, a phenyl group, a C₁₋₉ heteroaryl group or the like; Ar represents a monocyclic or fused polycyclic C₁₋₉ heteroaryl group represented by the formula (2) or a C₂₋₉ heterocyclyl group represented by formula (2); Y represents -S-, -O-or -NH-; and Z represents -S-, -O- or -CH₂-.

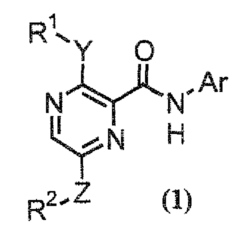

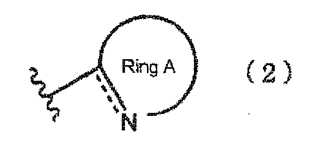

## Description

### TECHNICAL FIELD

The present invention relates to a novel pyrazinamide compound having a glucokinase activating effect and a pharmaceutical containing the compound as an active ingredient.

### BACKGROUND ART

Glucokinase (hereinafter described as GK) belongs to the hexokinase family and catalyzes phosphorylation of glucose taken in a cell such as a pancreatic beta cell and a hepatic cell. GK in the liver differs from that in pancreatic beta cells in the sequence of the N-terminal 15 amino acids due to a difference in splicing, but they are enzymatically identical. GK has a high affinity, as high as S_{0.5} = around 10 mM, for glucose. Additionally, the affinity of GK for glucose is not suppressed by a product, glucose-6-phosphate. Therefore, the reaction rate thereof sensitively responds to a blood glucose level within the physiological variation range. In pancreatic beta cells, GK controls insulin secretion in a glucose dependent manner; whereas, in the liver, GK controls the glycolysis system or glycogen synthesis to maintain/control the glucose level. It is therefore postulated that GK acts as a glucose sensor for maintaining homeostasis of a blood glucose level (see Non-Patent Document 1).
The postulation that GK acts as a biological glucose sensor is supported by findings of gene mutation in gene manipulated mice and humans. A mouse with homologousdeletion of GK died shortly after birth with elevated blood glucose; on the other hand, in a mouse with heterologousdeletion, elevated blood glucose and glucose tolerance insufficiency have been observed (see Non-Patent Document 2). On the other hand, in a mouse excessively expressing GK, a low blood glucose level has been confirmed (see Non-Patent Document 3). Furthermore, human MODY 2 (maturity onset diabetes of the young), in which a GK gene mutation is detected, develops diabetes at a young age (see Non-Patent Document 4). In this gene mutation, low GK activity has been confirmed. On the other hand, a family having a gene mutation which accelerates GK activity has been reported (see Non-Patent Document 5). In this gene mutation, the affinity of GK for glucose is accelerated and a symptom of fasting hypoglycemia accompanying an elevation of a blood insulin level is observed.
As described, GK is found to act as a glucose sensor in mammalians including humans.

It is considered that a substance enhancing GK activity (hereinafter, described as a GK activating substance) can increase glucose metabolism and glycogen synthesis in the liver, and glucose inducible insulin secretion from the pancreatic beta cell, thereby correcting elevated blood glucose. It is expected that, if elevated blood glucose is corrected, diabetic chronic complications, such as retinopathy, nephropathy, neurosis, ischemic heart disease and arteriosclerosis, can be treated and prevented, and further, diabetes-related disorders, such as obesity, hyperlipemia, hypertension and metabolic syndrome, can be treated and prevented. Therefore, a compound enhancing GK activity is expected to serve an effective therapeutic agent for diabetes.

On the other hand, it is reported that GK is expressed not only in the pancreas and the liver but also in the feeding center and plays an important role in suppression of food intake by glucose (see Non-Patent Document 6). Therefore, it is considered that the GK activating substance also acts upon the feeding center to suppress food intake. GK is thus expected to serve as a therapeutic agent not only for diabetes but also for obesity.
Conventionally, as the GK activating substance, a certain type of propionamide compound, a certain type of picolinamide compound, a certain type of benzamide compound and a certain type of benzimidazole compound have been reported; however, no disclosure is made of a compound of the present invention (see Patent Documents 1, 2, 3 and 4). Furthermore, pyrazinamide compounds structurally resemble have been reported; however, no disclosure is made of a compound of the present invention. Uses thereof are a schizophrenia therapeutic agent and a herbicide, respectively. These compounds differ in uses from a compound of the present invention (see Patent Documents 5 and 6).

Non-Patent Document 1: Matschinsky F. M. and Magnuson M. A., Frontiers in Diabetes, 16, 2004
Non-Patent Document 2: Grupe A. et al., Cell, 83, 1, 69-78, 1995
Non-Patent Document 3: Ferre T. et al., Proc. Natl. Acad. Sci., 93, 14, 7225-7230, 1996
Non-Patent Document 4: Vioneet N. et al., Nature, 356, 6371, 721-722, 1992
Non-Patent Document 5: Glaser B. et al., N. Engl. J. Med. 338, 4, 226-230, 1998
Non-Patent Document 6: Kang L. et al., Diabetes, 55, 2, 412-420, 2006
Patent Document 1: WO01/085707
Patent Document 2: WO04/081001
Patent Document 3: WO05/044801
Patent Document 4: WO07/007910
Patent Document 5: WO05/079802
Patent Document 6: WO94/027974

### DISCLOSURE OF THE INVENTION

### PROBLEMS TO BE SOLVED BY THE INVENTION

An object of the present invention is to provide a compound having an excellent GK activation action and useful as a pharmaceutical.

### MEANS FOR SOLVING THE PROBLEMS

The present inventors have conducted intensive studies with a view toward finding a compound having a GK activation action. As a result, they found that a pyrazinamide compound represented by the general formula (1) or a pharmaceutically acceptable salt thereof achieves this object. Based on the finding, the present invention was accomplished.

Accordingly, the present invention provides (I) a pyrazinamide compound represented by formula (1), a tautomer or stereo isomer of the compound or a pharmaceutically acceptable salt thereof or a solvate thereof 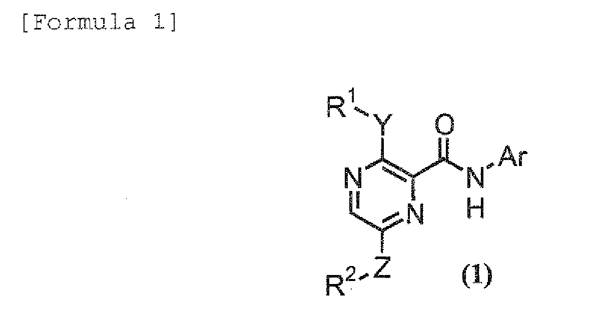 (in the formula (1),
R¹ represents a hydrogen atom, a C₁₋₈ alkyl group, a C₃₋₈ cycloalkyl group (the C₃₋₈ cycloalkyl group is unsubstituted or substituted with one hydroxy group), a C₂₋₉ heterocyclyl group (the C₂₋₉ heterocyclyl group is unsubstituted or substituted with one to three groups which may be the same or different and selected from the group consisting of a C₁₋₆ alkyl group and an oxo group), a phenyl group (the phenyl group is unsubstituted or substituted with one to three groups which may be the same or different and selected from the group consisting of a halogen atom, a hydroxy group, a trifluoromethyl group, a C₁₋₆ alkyl group, a hydoxy C₁₋₈ alkyl group, a C₁₋₆ alkoxy group, a C₁₋₆ alkylthio group, a C₁₋₆ alkylsulfonyl group, a carboxy group and a C₂₋₆ alkoxycarbonyl group), a C₁₋₉ heteroaryl group (the C₁₋₉ heteroaryl group is unsubstituted or substituted with one to three groups which may be the same or different and selected from the group consisting of a halogen atom, a C₁₋₆ alkyl group and a hydoxy C₁₋₈ alkyl group) or a C₇₋₁₄ arylalkyl group (the C₇₋₁₄ arylalkyl group is unsubstituted or substituted with one to three groups which may be the same or different and selected from the group consisting of a halogen atom, a C₁₋₆ alkyl group, a hydroxy group and a C₁₋₆ alkoxy group),
R² represents a C₁₋₈ alkyl group (the C₁₋₈ alkyl group is unsubstituted or substituted with one to three groups which may be the same or different and selected from the group consisting of a halogen atom, a hydroxy group and a C₁₋₆ alkoxy group), a C₂₋₉ heterocyclyl group (the C₂₋₉ heterocyclyl group is unsubstituted or substituted with one or two oxo groups), a phenyl group (the phenyl group is unsubstituted or substituted with one to three halogen atoms) or a C₁₋₉ heteroaryl group (the C₁₋₉ heteroaryl group is unsubstituted or substituted with one to three groups which may be the same or different and selected from the group consisting of a hydroxy group, an amino group and a C₁₋₆ alkyl group),
Ar represents a monocyclic or fused polycyclic C₁₋₉ heteroaryl group represented by formula (2) or a C₂₋₉ heterocyclyl group represented by formula (2): 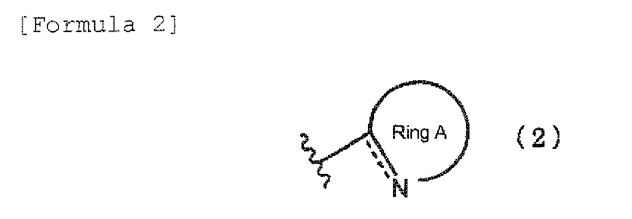 (in the formula (2), ring A is unsubstituted or substituted with one to three groups which may be the same or different and selected from the group consisting of a C₁₋₆ alkyl group, a carboxy group, a carboxy C₁₋₆ alkyl group, a carbamoyl group, a carbamoyl C₁₋₆ alkyl group, a hydroxy C₁₋₈ alkyl group, a C₁₋₆ alkoxy C₁₋₈ alkyl group, a halogen atom, a C₂₋₆ alkoxycarbonyl group, a C₂₋₆ alkoxycarbonyl C₁₋₆ alkyl group and a di (C₁₋₆ alkyl) amino C₁₋₈ alkyl group),
Y represents -S-, -O- or -NH-, and
Z represents -S-, -O- or -CH₂-).
(II) Another aspect of the present invention provides the pyrazinamide compound represented by the formula (1), a tautomer or stereo isomer of the compound or a pharmaceutically acceptable salt thereof or a solvate thereof according to (I) 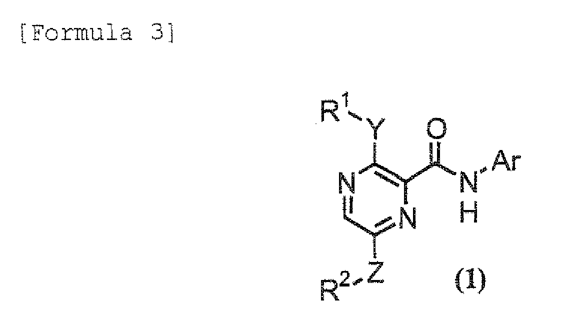 (in the formula (1),
R¹ represents a hydrogen atom, a C₁₋₈ alkyl group, a C₃₋₈ cycloalkyl group, a C₂₋₉ heterocyclyl group (the C₂₋₉ heterocyclyl group is unsubstituted or substituted with one C₁₋₆ alkyl group), a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group or a C₇₋₁₄ arylalkyl group (the C₇₋₁₄ arylalkyl group is unsubstituted or substituted with one C₁₋₆ alkyl group),
R² represents a C₂₋₉ heterocyclyl group (the C₂₋₉ heterocyclyl group is unsubstituted or substituted with one oxo group), a phenyl group (the phenyl group is unsubstituted or substituted with one halogen atom) or a C₁₋₉ heteroaryl group (the C₁₋₉ heteroaryl group is unsubstituted or substituted with one group selected from the group consisting of a hydroxy group, an amino group and a C₁₋₆ alkyl group),
Ar represents a monocyclic C₁₋₆ heteroaryl group represented by the formula (2) or a C₂₋₉ heterocyclyl group represented by formula (2): 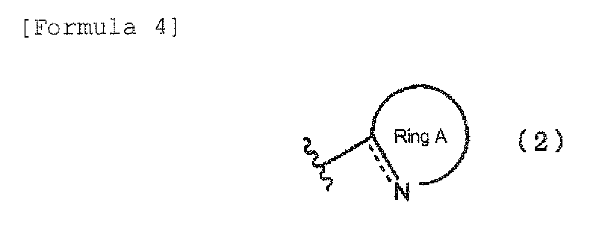 (in the formula (2), ring A is unsubstituted or substituted with one or two groups which may be the same or different and selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl group, a C₂₋₆ alkoxycarbonyl C₁₋₆ alkyl group and a di(C₁₋₆ alkyl)amino C₁₋₈ alkyl group),
Y represents -S-, -O- or -NH-, and
Z represents -S- or -CH₂-).
(III) Another aspect of the present invention provides the pyrazinamide compound represented by the formula (1), a tautomer or stereo isomer of the compound or a pharmaceutically acceptable salt thereof or a solvate thereof according to (I) 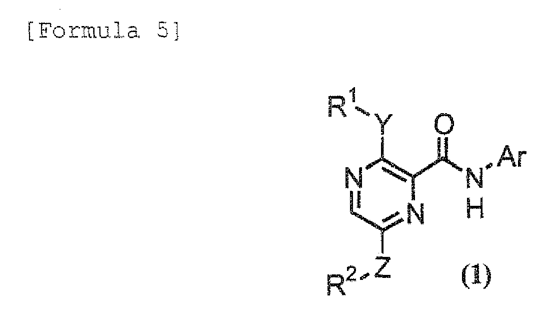 (in the formula (1),
R¹ represents a C₁₋₈ alkyl group, a C₃₋₈ cycloalkyl group, a C₂₋₉ heterocyclyl group (then C₂₋₉ heterocyclyl group is unsubstituted or substituted with one C₁₋₆ alkyl group), a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group (the C₁₋₉ heteroaryl group is unsubstituted or substituted with one C₁₋₆ alkyl group) or a C₇₋₁₄ arylalkyl group (the C₁₋₁₄ arylalkyl group is unsubstituted or substituted with one C₁₋₆ alkyl group),
R² represents a C₂₋₉ heterocyclyl group (the C₂₋₉ heterocyclyl group is unsubstituted or substituted with one oxo group), a phenyl group (the phenyl group is unsubstituted or substituted with one halogen atom) or a C₁₋₉ heteroaryl group (the C₁₋₉ heteroaryl group is unsubstituted or substituted with one group selected from the group consisting of a hydroxy group, an amino group and a C₁₋₆ alkyl group),
Ar represents a monocyclic C₁₋₆ heteroaryl group represented by formula (2) or a C₂₋₉ heterocyclyl group represented by formula (2): 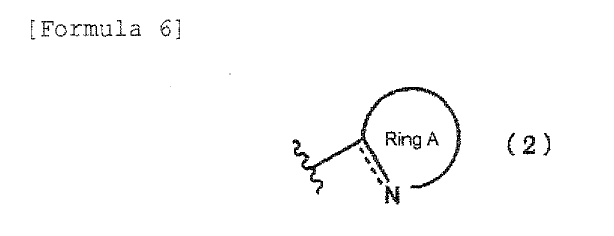 (in the formula (2), ring A is unsubstituted or substituted with one or two groups which may be the same or different and selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl group, a C₂₋₆ alkoxycarbonyl C₁₋₆ alkyl group and a di (C₁₋₆ alkyl)amino C₁₋₈ alkyl group),
Y represents -S- or -O-, and
Z represents -S- or -CH₂-,
with the proviso that, when Z represents -CH₂-, the monocyclic C₁₋₆ heteroaryl group represented by the formula (2) does not represent include a pyridin-2-yl group).
(IV) Another aspect of the present invention provides the pyrazinamide compound, a tautomer or stereo isomer of the compound or a pharmaceutically acceptable salt thereof or a solvate thereof according to (II), wherein R² is a C₁₋₉ heteroaryl group (the C₁₋₉ heteroaryl group is unsubstituted or substituted with one group selected from the group consisting of a hydroxy group, an amino group and a C₁₋₆ alkyl group), and Z is -S- in the formula (1).
(V) Another aspect of the present invention provides the pyrazinamide compound, a tautomer or stereo isomer of the compound or a pharmaceutically acceptable salt thereof or a solvate thereof according to (III), wherein R² is a C₁₋₉ heteroaryl group (the C₁₋₉ heteroaryl group is unsubstituted or substituted with one group selected from the group consisting of a hydroxy group, an amino group and a C₁₋₆ alkyl group), and Z is -S- in the formula (1).
(VI) Another aspect of the present invention provides the pyrazinamide compound, a tautomer or stereo isomer of the compound or a pharmaceutically acceptable salt thereof or a solvate thereof according to (IV), wherein R² is a triazolyl group (the triazolyl group is unsubstituted or substituted with one group selected from the group consisting of an amino group and a C₁₋₆ alkyl group) in the formula (1).
(VII) Another aspect of the present invention provides the pyrazinamide compound, a tautomer or stereo isomer of the compound or a pharmaceutically acceptable salt thereof or a solvate thereof according to (V), wherein R² is a triazolyl group (the triazolyl group is unsubstituted or substituted with one group selected from the group consisting of an amino group and a C₁₋₆ alkyl group) in the formula (1).
(VIII) Another aspect of the present invention provides the pyrazinamide compound, a tautomer or stereo isomer of the compound or a pharmaceutically acceptable salt thereof or a solvate thereof according to (II), wherein R² is a 1,6-dihydropyridazinyl group (the 1,6-dihydropyridazinyl group is substituted with one oxo group), and Z is -CH₂- in the formula (1).
(IX) Another aspect of the present invention provides the pyrazinamide compound, a tautomer or stereo isomer of the compound or a pharmaceutically acceptable salt thereof or a solvate thereof according to (III), wherein R² is a 1,6-dihydropyridazinyl group (the 1,6-dihydropyridazinyl group is substituted with one oxo group), and Z is -CH₂- in the formula (1).
(X) Another aspect of the present invention provides the pyrazinamide compound, a tautomer or stereo isomer of the compound or a pharmaceutically acceptable salt thereof or a solvate thereof according to any one of (II), (IV), (VI) and (VIII), wherein
R¹ is a C₁₋₈ alkyl group, a C₃₋₈ cycloalkyl group, a C₂₋₉ heterocyclyl group, a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₂₋₆ alkylsulfonyl group), a C₁₋₉ heteroaryl group or a C₁₋₁₄ arylalkyl group (the C₇₋₁₄ arylalkyl group is unsubstituted or substituted with one C₁₋₆ alkyl group), and

Y is -S- or -NH-

in the formula (1).
(XI) Another aspect of the present invention provides the pyrazinamide compound, a tautomer or stereo isomer of the compound or a pharmaceutically acceptable salt thereof or a solvate thereof according to any one of (III), (V), (VII) and (IX), wherein
R¹ is a C₁₋₈ alkyl group, a C₃₋₈ cycloalkyl group, a C₂₋₉ heterocyclyl group, a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group (the C₁₋₉ heteroaryl group is unsubstituted or substituted with one C₁₋₆ alkyl group) or a C₇₋₁₄ arylalkyl group (the C₇₋₁₄ arylalkyl group is unsubstituted or substituted with one C₁₋₆ alkyl group), and

Y is -S-

in the formula (1).
(XII) Another aspect of the present invention provides the pyrazinamide compound, a tautomer or stereo isomer of the compound or a pharmaceutically acceptable salt thereof or a solvate thereof according to (X), wherein Y is -S- in the formula (1).
(XIII) Another aspect of the present invention provides the pyrazinamide compound, a tautomer or stereo isomer of the compound or a pharmaceutically acceptable salt thereof or a solvate thereof according to any one of (II) to (XII), wherein Ar is a monocyclic C₁₋₆ heteroaryl group represented by the formula (2) 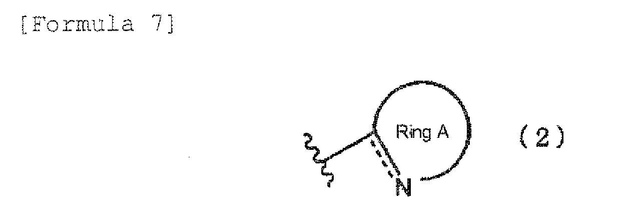 (in the formula (2), ring A is unsubstituted or substituted with one group selected from the group consisting of a C₁₋₆ alkyl group, a carboxy group, a carboxy C₁₋₆ alkyl group, a carbamoyl group, a hydoxy C₁₋₉ alkyl group, a C₁₋₆ alkoxy C₁₋₈ alkyl group, a halogen atom, a C₂₋₆ alkoxycarbonyl group and a C₂₋₆ alkoxycarbonyl C₁₋₆ alkyl group) or a C₂₋₉ heterocyclyl group
in the formula (1).
(XIV) Another aspect of the present invention provides the pyrazinamide compound, a tautomer or stereo isomer of the compound or a pharmaceutically acceptable salt thereof or a solvate thereof according to (XIII), wherein
Ar is a 1H-pyrazol-3-yl group, a 1,3-thiazol-2-yl group, a pyridin-2-yl group, a pyrazin-2-yl group, an isoxazol-3-yl group (the 1H-pyrazol-3-yl group, 1,3-thiazol-2-yl group, pyridin-2-yl group, pyrazin-2-yl group and isoxazol-3-yl group are unsubstituted or substituted with one group selected from the group consisting of a C₁₋₆ alkyl group, a carboxy group, a carboxy C₁₋₆ alkyl group, a carbamoyl group, a hydroxy C₁₋₈ alkyl group, a C₁₋₆ alkoxy C₁₋₈ alkyl group, a halogen atom, a C₂₋₆ alkoxycarbonyl group and a C₂₋₆ alkoxycarbonyl C₁₋₆ alkyl group) or a 4,5-dihydro-1,3-thiazol-2-yl group in the formula (1).
(XV) Another aspect of the present invention provides the following pyrazinamide compounds, a tautomer or stereo isomer of the compound or a pharmaceutically acceptable salt thereof or a solvate thereof:
3-[(4-fluorophenyl)sulfanyl]-N-(1-methyl-1H-pyrazol-3-yl)-6-(4H-1,2,4-triazol-3-ylsulfanyl)pyrazine-2-carboxamide,
3-[(4-fluorophenyl)sulfanyl]-N-(5-methyl-1,3-thiazol-2-yl)-6-(4H-1,2,4-triazol-3-ylsulfanyl)pyrazine-2-carboxamide,
3-[(1-methylethyl)sulfanyl]-N-(1-methyl-1H-pyrazol-3-yl)-6-(4H-1,2,4-triazol-3-ylsulfanyl)pyrazine-2-carboxamide,
3-(cyclohexylsulfanyl)-N-(1-methyl-1H-pyrazol-3-yl)-6-(4H-1,2,4-triazol-3-ylsulfanyl)pyrazine-2-carboxamide,
3-[(4-methylphenyl)sulfanyl]-N-(1-methyl-1H-pyrazol-3-yl)-6-(4H-1,2,4-triazol-3-ylsulfanyl)pyrazine-2-carboxamide,
3-[(4-methoxyphenyl)sulfanyl]-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-{[4-(methylsulfanyl)phenyl]sulfanyl}-6-(4H-1,2,4-triazol-3-ylsulfanyl)pyrazine-2-carboxamide,
3-(benzylsulfanyl)-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-{[4-(methylsulfonyl)phenyl]sulfanyl}-6-(4H-1,2,4-triazol-3-ylsulfanyl)pyrazine-2-carboxamide,
3-{[(1S)-1-methyl-2-phenylethyl]sulfanyl}-N-(1-methyl-1H-pyrazol-3-yl)-6-(4H-1,2,4-triazol-3-ylsulfanyl)pyrazine-2-carboxamide,
6-[({3-[(4-fluorophenyl)sulfanyl]-6-(4H-1,2,4-triazol-3-ylsulfanyl)pyrazin-2-yl}carbonyl)amino]pyridine-3-carboxylic acid methyl ester,
{3-[({3-[(4-fluorophenyl)sulfanyl]-6-(4H-1,2,4-triazol-3-ylsulfanyl)pyrazin-2-yl}carbonyl)amino]-1H-pyrazol-1-yl}acetic acid ethyl ester,
3-[(4-fluorophenyl)sulfanyl]-N-[1-(2-hydroxyethyl)-1H-pyrazol-3-yl]-6-(4H-1,2,4-triazol-3-ylsulfanyl)pyrazine-2-carboxamide,
3-[(4-fluorophenyl)sulfanyl]-N-[1-(2-methoxyethyl)-1H-pyrazol-3-yl]-6-(4H-1,2,4-triazol-3-ylsulfanyl)pyrazine-2-carboxamide,
N-(1,5-dimethyl-1H-pyrazol-3-yl)-3-[(4-fluorophenyl)sulfanyl]-6-(4H-1,2,4-triazol-3-ylsulfanyl)pyrazine-2-carboxamide,
N-(1-methyl-1H-pyrazol-3-yl)-6-(4H-1,2,4-triazol-3-ylsulfanyl)-3-{[4-(trifluoromethyl)phenyl]sulfanyl}pyrazine-2-carboxamide,
N-(1-methyl-1H-pyrazol-3-yl)-3-(tetrahydro-2H-pyran-4-ylsulfanyl)-6-(4H-1,2,4-triazol-3-ylsulfanyl)pyrazine-2-carboxamide,
3-[(3,4-dimethoxyphenyl)sulfanyl]-N-(1-methyl-1H-pyrazol-3-yl)-6-(4H-1,2,4-triazol-3-ylsulfanyl)pyrazine-2-carboxamide,
3-[(3-methoxyphenyl)sulfanyl]-N-(1-methyl-1H-pyrazol-3-yl)-6-(4H-1,2,4-triazol-3-ylsulfanyl)pyrazine-2-carboxamide,
3-[(2-methoxyphenyl)sulfanyl]-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-[(6-methylpyridin-3-yl)sulfanyl]-6-(4H-1,2,4-triazol-3-ylsulfanyl)pyrazine-2-carboxamide,
N-(1-methyl-1H-pyrazol-3-yl)-3-(pyridin-4-ylsulfanyl)-6-(4H-1,2,4-triazol-3-ylsulfanyl)pyrazine-2-carboxamide,
N-(4,5-dihydro-1,3-thiazol-2-yl)-3-[(4-fluorophenyl)sulfanyl]-6-(4H-1,2,4-triazol-3-ylsulfanyl)pyrazine-2-carboxamide,
N-(5-chloro-1,3-thiazol-2-yl)-3-[(4-fluorophenyl)sulfanyl]-6-(4H-1,2,4-triazol-3-ylsulfanyl)pyrazine-2-carboxamide,
3-[(4-fluorophenyl)sulfanyl]-N-(5-methylisoxazol-3-yl)-6-(4H-1,2,4-triazol-3-ylsulfanyl)pyrazine-2-carboxamide,
N-(5-carbamoylpyridin-2-yl)-3-[(4-fluorophenyl)sulfany]-]-6-(4H-1,2,4-triazol-3-ylsulfanyl)pyrazine-2-carboxamide,
3-[(4-fluorophenyl)sulfanyl]-N-(pyrazin-2-yl)-6-(4H-1,2,4-triazol-3-ylsulfanyl)pyrazine-2-carboxamide,
3-[(4-fluorophenyl)sulfanyl]-N-[1-(2-methoxyethyl)-1H-pyrazol-3-yl]-6-[(5-methyl-4H-1,2,4-triazol-3-yl)sulfanyl]pyrazine-2-carboxamide,
3-[(4-methoxyphenyl)sulfanyl]-N-(5-methyl-1,3-thiazol-2-yl)-6-(4H-1,2,4-triazol-3-ylsulfanyl)pyrazine-2-carboxamide,
3-[(4-hydroxyphenyl)sulfanyl]-N-(5-methyl-1,3-thiazal-2-yl)-6-(4H-1,2,4-triazol-3-ylsulfanyl)pyrazine-2-carboxamide,
6-[({3-[(4-fluorophenyl)sulfanyl]-6-(4H-1,2,4-triazol-3-ylsulfanyl)pyrazin-2-yl}carbonyl)amino]pyridine-3-carboxylic acid,
{3-[({3-[(4-fluorophenyl)sulfanyl]-6-(4H-1,2,4-triazol-3-ylsulfanyl)pyrazin-2-yl}carbonyl)amino]-1H-pyrazol-1-yl}acetic acid,
3-[(4-fluorophenyl)sulfanyl]-N-(1-methyl-1H-pyrazol-3-yl)-6-[(4-methyl-4H-1,2,4-triazol-3-yl)sulfanyl]pyrazine-2-carboxamide,
3-[(4-flucrophenyl)sulfanyl]-N-(1-methyl-1H-pyrazol-3-yl)-6-[(5-me-thyl-4H-1,2,4-triazol-3-yl)sulfanyl]pyrazine-2-carboxamide,
3-[(4-fluorophenyl)sulfanyl]-6-[(5-hydroxy-4H-1,2,4-triazol-3-yl)sulfanyl]-N-(1-methyl-1H-pyrazol-3-yl)pyrazine-2-carboxamide,
6-[(5-amino-4H-1,2,4-triazol-3-yl)sulfanyl]-3-[(4-fluorophenyl)sulfanyl]-N-(1-methyl-1H-pyrazol-3-yl)pyrazine-2-carboxamide,
3-[(4-fluorophenyl)sulfanyl]-N-(1-methyl-1H-pyrazol-3-yl)-6-(1,3,4-thiadiazol-2-ylsulfanyl)pyrazine-2-carboxamide,
3-[(4-fluorophenyl)sulfanyl]-N-(1-methyl-1H-pyrazol-3-yl)-6-(pyridin-2-ylsulfanyl)pyrazine-2-carboxamide,
3-[(4-methoxyphenyl)sulfanyl]-N-(1-methyl-1H-pyrazol-3-yl)-6-[(5-methyl-4H-1,2,4-triazol-3-yl)sulfanyl]pyrazine-2-carboxamide,
6-[(5-ethyl-4H-1,2,4-triazol-3-yl)sulfanyl]-3-[(4-methoxyphenyl)sulfanyl]-N-(1-methyl-1H-pyrazol-3-yl)pyrazine-2-carboxamide,
6-[(5-amino-4H-1,2,4-triazol-3-yl)sulfanyl]-3-[(4-methoxyphenyl)sulfanyl]-N-(1-methyl-1H-pyrazol-3-yl)pyrazine-2-carboxamide,
3-[(4-methoxyphenyl)sulfanyl]-N-pyrazin-2-yl-6-(4H-1,2,4-triazol-3-ylsulfanyl)pyrazine-2-carboxamide,
3-[(4-methoxyphenyl)sulfanyl]-6-[(5-methyl-4H-1,2,4-triazol-3-yl)sulfanyl]-N-pyrazin-2-ylpyrazine-2-carboxamide,
6-[(5-amino-4H-1,2,4-triazol-3-yl)sulfanyl]-3-[(4-methoxyphenyl)sulfanyl)-N-pyrazin-2-ylpyrazine-2-carboxamide,
3-{[4-(methylsulfanyl)phenyl]sulfanyl}-N-pyrazin-2-yl-6-(4H-1,2,4-triazol-3-ylsulfanyl)pyrazine-2-carboxamide,
3-{[4-(methylsulfanyl)phenyl]sulfanyl}-6-[(5-methyl-4H-1,2,4-triazol-3-yl)sulfanyl]-N-pyrazin-2-ylpyrazine-2-carboxamide,
6-[(5-amino-4H-1,2,4-triazol-3-yl)sulfanyl]-3-{[4-(methylsulfanyl)phenyl]sulfanyl}-N-pyrazin-2-ylpyrazine-2-carboxamide,
N-(1-methyl-1H-pyrazol-3-yl)-3-{[4-(methylsulfonyl)phenyl]sulfanyl)-6-[(5-methyl-4H-1,2,4-triazol-3-yl)sulfanyl]pyrazine-2-carboxamide,
6-[(5-amino-4H-1,2,4-triazol-3-yl)sulfanyl]-N-(1-methyl-1H-pyrazol-3-yl)-3-{[4-(methylsulfonyl)phenyl]sulfanyl}pyrazine-2-carboxamide,
N-(1-methyl-1H-pyrazol-3-yl)-6-[(5-methyl-4H-1,2,4-triazol-3-yl)sulfanyl]-3-(pyridin-4-ylsulfanyl)pyrazine-2-carboxamide,
6-[(5-amino-4H-1,2,4-triazol-3-yl)sulfanyl]-N-(1-methyl-1H-pyrazol-3-yl)-3-(pyridin-4-ylsulfanyl)pyrazine-2-carboxamide,
3-(4-fluorophenoxy)-N-(1-methyl-1H-pyrazol-3-yl)-6-(4H-1,2,4-triazol-3-ylsulfanyl)pyrazine-2-carboxamide,
3-[(4-fluorophenyl)amino]-N-(1-methyl-1H-pyrazol-3-yl)-6-(4H-1,2,4-triazol-3-ylsulfanyl)pyrazine-2-carboxamide,
3-[(4-fluorophenyl)sulfanyl]-N-(5-methyl-1,3-thiazol-2-yl)-6-[(6-oxopyridazin-1(6H)-yl)methyl]pyrazine-2-carboxamide,
3-[(4-fluorophenyl)sulfanyl]-N-(1-methyl-1H-pyrazol-3-yl)-6-[(6-oxopyridazin-1(6H)-yl)methyl]pyrazine-2-carboxamide,
3-[(4-fluorophenyl)sulfanyl]-N-(1-methyl-1H-pyrazol-3-yl)-6-(1H-1,2,4-triazol-1-ylmethyl)pyrazine-2-carboxamide.
(XVI) Another aspect of the present invention provides a pharmaceutical having, as an active ingredient, a pyrazinamide compound, a tautomer or stereo isomer of the compound or a pharmaceutically acceptable salt thereof or a solvate thereof according to any of (I) to (XV).
(XVII) Another aspect of the present invention provides the pharmaceutical according to (XVI) for preventing or treating a disorder or state that would be improved by a glucokinase activating effect.
(XVIII) Another aspect of the present invention provides the pharmaceutical according (XVII) being a prophylactic/therapeutic agent for diabetes or obesity.

### ADVANTAGE OF THE INVENTION

A compound having an excellent GK activation action has been successfully provided by the present invention.

### BEST MODE FOR CARRYING OUT THE INVENTION

The present invention will be morse specifically described below; however, it is not particularly limited to exemplified ones.
In the present invention, "n" represents normal, "i" iso, "s" secondary, "t" tertiary, "c" cyclo, "o" ortho, "m" meta and "p" para.
The "C₁₋₆ alkyl group" represents a straight or branched alkyl group having 1 to 6 carbon atoms. For example, a methyl group, an ethyl group, a n-propyl group, a n-butyl group, a n-pentyl group, a n-hexyl group, an i-propyl group, an i-butyl group, a t-butyl group, a s-butyl group, an i-pentyl group, a neopentyl group and a t-pentyl group can be mentioned. A methyl group and an ethyl group are more preferable.
The "C₁₋₈ alkyl group" represents a straight or branched alkyl group having 1 to carbon atoms. For example, a methyl group, an ethyl group, a n-propyl group, a n-butyl group, a n-pentyl group, a n-hexyl group, a n-heptyl group, a n-octyl group, an i-propyl group, an i-butyl group, a t-butyl group, a s-butyl group, an i-pentyl group, a neopentyl group and a t-pentyl group can be mentioned. A methyl group, an ethyl group and an i-propyl group are more preferable.
The "hydroxy C₁₋₈ alkyl group" represents a "C₁₋₈ alkyl group" having a hydroxy group as a substituent. For example, a hydroxymethyl group, a 2-hydroxyethyl group, a 3-hydroxy-n-propyl group, a 2-hydroxy-n-propyl group, a 4-hydroxy-n-butyl group and a 2-hydroxy-i-propyl group can be mentioned. A 2-hydroxyethyl group is more preferable.
The "halogen atom" represents a fluorine atom, a chlorine atom, a bromine atom or an iodine atom. A fluorine atom and a chlorine atom are more preferable.
The "C₃₋₈ cycloalkyl group" represents a cycloalkyl group having 3 to 8 carbon atoms. For example, a c-propyl group, a c-butyl group, a c-pentyl group, a c-hexyl group, a c-heptyl group and a c-octyl group can be mentioned. A c-hexyl group is more preferable.
The "C₁₋₆ alkoxy group" represents a straight or branched alkoxy group having 1 to 6 carbon atoms. For example, a methoxy group, an ethoxy group, a n-propoxy group, an i-propoxy group, a n-butoxy group, an i-butoxy group, a s-butoxy group, a t-butoxy group, a n-pentyloxy group, an i-pentyloxy group and a n-hexyloxy group can be mentioned. A methoxy group is more preferable.
The "C₁₋₆ alkoxy C₁₋₈ alkyl group" represents a "C₁₋₈ alkyl group" having a "C₁₋₆ alkoxy group" as a substituent. For example, a 2-methoxyethyl group, a 2-ethoxyethyl group and a 2-i-propoxy-2-methyl-n-propyl group can be mentioned. A 2-methoxyethyl group is more preferable.
The "C₂₋₆ alkoxycarbonyl group" represents a carbonyl group having a straight or branched alkoxy group having 1 to 5 carbon atoms. For example, a methoxycarbonyl group, an ethoxycarbonyl group and an i-propoxycarbonyl group can be mentioned. A methoxycarbonyl group is more preferable.

The "C₁₋₆ alkylthio group" represents a straight or branched alkylthio group having 1 to 6 carbon atoms. For example, a methylthio group, an ethylthio group, a n-propylthio group, an i-propylthio group, a n-butylthio group, an i-butylthio group, a s-butylthio group and a t-butylthio group can be mentioned. A methylthio group is more preferable.
The "C₁₋₆ alkylsulfonyl group" represents a straight or branched alkylsulfonyl group having 1 to 6 carbon atoms. For example, a methylsulfonyl group, a n-propylsulfonyl group, an i-butylsulfonyl group and a n-hexylsulfonyl group can be mentioned. A methylsulfonyl group is more preferable.
The "C₂₋₉ heterocyclyl group" represents a 5 to 7-membered monocyclic heterocyclyl group or a fused polycyclic heterocyclyl group constituted of 10 to 14 atoms, constituted of at least one atom selected from the group consisting of an oxygen atom, a sulfur atom and a nitrogen atom and 2 to 9 carbon atoms. For example, a tetrahydrofuranyl group, a tetrahydropyranyl group, an isothiazolidinyl group, a pyrrolidinyl group, an imidazolidinyl group, a pyrazolinyl group, a piperidyl group, a piperazinyl group, a morpholinyl group, a 1,3-oxazolidinyl group, a 1,6-dihydropyridazinyl group, a thiazolidinyl group, an indolinyl group, a 1,6-dihydropyrimidinyl group, a 1,2,3,4-tetrahydropyrimidinyl group and a 4,5-dihydro-1,3-thiazol-2-yl group can be mentioned. A tetrahydropyranyl group, a 1,6-dihydropyridazinyl group and a 4,5-dihydro-1,3-thiazol-2-yl group are more preferable.

The "C₇₋₁₄ arylalkyl group" represents a straight or branched alkyl group having a total number of carbon atoms of 7 to 14 and having a monocyclic or polycyclic aromatic hydrocarbon group as a substituent or a group having a total number of carbon atoms of 7 to 14 and obtained by fusing a monocyclic or polycyclic aromatic hydrocarbon group with a straight or branched alkyl group. For example, a benzyl group, a phenethyl group, a phenylpropyl group, a naphthylmethyl group, an indanyl group, a (2H)-indenyl group and a tetrahydronaphthyl group can be mentioned. A benzyl group and a phenethyl group are more preferable.
The "C₁₋₉ heteroaryl group" represents a 5 to 7-membered monocyclic aromatic heterocyclyl group constituted of at least one atom selected from the group consisting of an oxygen atom, a sulfur atom and a nitrogen atom and 1 to 9 carbon atoms, or a fused polycyclic aromatic heterocyclyl group constituted of 10 to 14 atoms. For example, an imidazolyl group, a pyrazolyl group, a thiazolyl group, an oxazolyl group, an isoxazolyl group, a pyrrolyl group, a triazolyl group, a pyridyl group, a pyrimidinyl group, a pyrazinyl group, an indolyl group, a quinolyl group and a thiadiazolyl group can be mentioned. A pyrazolyl group, a thiazolyl group, a triazolyl group, a pyridyl group, an isoxazolyl group, a pyrazinyl group and a thiadiazolyl group are more preferable.
The "carboxy C₁₋₆ alkyl group" represents a "C₁₋₆ alkyl group" having a carboxy group as a substituent. For example, a carboxymethyl group, a 2-carboxyethyl group, a 3-carboxy-n-propyl group, a 2-carboxy-n-propyl group, a 2-carboxy-i-propyl group and a 4-carboxy-n-butyl group can be mentioned. A carboxymethyl group is more preferable. The "C₂₋₆ alkoxycarbonyl C₁₋₆ alkyl group" represents a "C₁₋₆ alkyl group" having a "C₂₋₆ alkoxycarbonyl group" as a substituent. For example, a methoxycarbonylmethyl group, a 2-(methoxycarbonyl)ethyl group, a 3-(methoxycarbonyl)-n-propyl group, a 2-(methoxycarbonyl)-n-propyl group, a 2-(methoxycarbonyl)-i-propyl group, an ethoxycarbonylmethyl group and a 2-(ethoxycarbonyl)ethyl group can be mentioned. An ethoxycarbonylmethyl group is more preferable.
The "carbamoyl C₁₋₆ alkyl group" represents a "C₁₋₆ alkyl group" having a carbamoyl group as a substituent. For example, a carbamoylmethyl group, a 2-carbamoylethyl group, a 3-carbamoyl-n-propyl group, a 2-carbamoyl-n-propyl group, a 2-carbamoyl-i-propyl group and a 4-carbamoyl-n-butyl group can be mentioned. A carbamoylmethyl group is more preferable.
The "di(C₁₋₆ alkyl) amino group" represents an amino group having two "C₁₋₆ alkyl groups" which may be the same or different, as a substituent. For example, a dimethylamino group, a diethylamino group, a di-n-propylamino group, a di-i-propylamino group, a di-i-butylamino group, a di-s-butylamino group, a di-t-butylamino group, an ethyl(methyl)amino group and a methyl(n-propyl)amino group can be mentioned.
The "di(C₁₋₆ alkyl) amino C₁₋₈ alkyl group" represents a "C₁₋₈ alkyl group" having a "di(C₁₋₆ alkyl)amino group" as a substituent. For example, a (dimethylamino)methyl group, a 2-(dimethylamino)ethyl group, a 2-{di(n-propyl)amino}ethyl group, a di(i-propyl)aminomethyl group, a 2-(ethylmethylamino)ethyl group and a 2-{(methyl)-(n-propyl)amino}ethyl group can be mentioned. A 2-(dimethylamino)ethyl group is more preferable.
The monocyclic or fused polycyclic C₁₋₉ heteroaryl group represented by the formula (2): 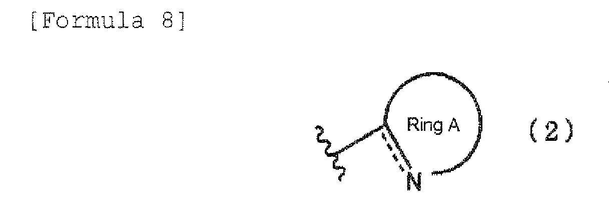 represents a 5 to 7-membered monocyclic aromatic heterocyclyl group constituted of at least one atom selected from the group consisting of an oxygen atom, a sulfur atom and a nitrogen atom and 1 to 9 carbon atoms, or a fused polycyclic aromatic heterocyclyl group constituted of 10 to 14 atoms, in which a nitrogen atom of the amide group positioned next to this group in the formula (1) is bonded to a carbon atom forming the following bond in this group. 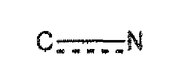 For example, a pyrazolyl group, a thiazolyl group, a pyridyl group, a pyrazinyl group, a thiadiazolyl group, a pyridothiazolyl group, an isoxazolyl group and an oxazolyl group can be mentioned.
The monocyclic C₁₋₆ heteroaryl group represented by formula (2): 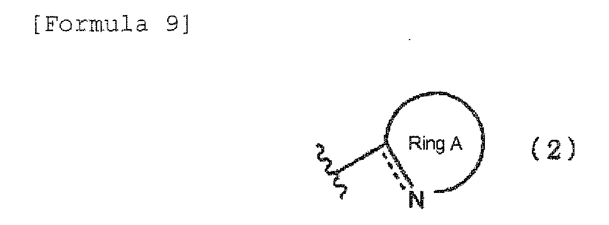 represents a 5 to 7-membered monocyclic aromatic heterocyclyl group constituted of at least one atom selected from the group consisting of an oxygen atom, a sulfur atom and a nitrogen atom and 1 to 6 carbon atoms, in which a nitrogen atom of the amide group positioned next to this group in the formula (1) is bonded to a carbon atom forming the following bond in this group. 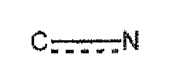 For example, a pyrazolyl group, a thiazolyl group, a pyridyl group, a pyrazinyl group, a thiadiazolyl group, an isoxazolyl group and an oxazolyl group can be mentioned.
The C₂₋₉ heterocyclyl group represented by formula (2): 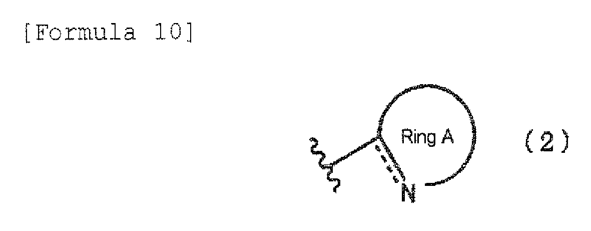 represents a 5 to 7-membered monocyclic heterocyclyl group constituted of at least one atom selected from the group consisting of an oxygen atom, a sulfur atom and a nitrogen atom and 2 to 9 carbon atoms or a fused polycyclic aromatic heterocyclyl group formed of 10 to 14 atoms, in which a nitrogen atom of the amide group positioned next to this group in the formula (1) is bonded to a carbon atom forming the following bond in this group. 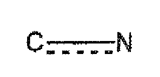 For example, an isothiazolidinyl group, a pyrrolidinyl group, an imidazolidinyl group, a pyrazolinyl group, a piperidyl group, a piperazinyl group, a morpholinyl group, a 1,3-oxazolidinyl group, a 1,6-dihydropyridazinyl group, a thiazolidinyl group, an indolinyl group, a 1,6-dihydropyrimidinyl group, a 1,2,3,4-tetrahydropyrimidinyl group, a 4,5-dihydro-1,3-thiazol-2-yl group, a 4H-3,1-benzothiazinyl group and a 2H-1,4-henzothiazinyl group can be mentioned. A 4,5-dihydro-1,3-thiazol-2-yl group is more preferable.

Preferred embodiments of the compound of the present invention are as follows:
Preferable R¹ is a hydrogen atom, a C₁₋₈ alkyl group, C₃₋₈ cycloalkyl group, a C₂₋₉ heterocyclyl group (the C₂₋₉ heterocyclyl group is unsubstituted or substituted with one C₁₋₆ alkyl group), a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group (the C₁₋₉ heteroaryl group is unsubstituted or substituted with one C₁₋₆ alkyl group) or a C₁₋₁₄ arylalkyl group (the C₇₋₁₄ arylalkyl group is unsubstituted or substituted with one C₁₋₆ alkyl group). Further preferable R¹ is a C₁₋₈ alkyl group, a C₃₋₈ cycloalkyl group, a C₂₋₉ heterocyclyl group, a phenyl group (the phenyl group is unsubstituted or substituted with one group selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group) or a C₁₋₉ heteroaryl group (the C₁₋₉ heteroaryl group is unsubstituted or substituted with one C₁₋₆ alkyl group).
Preferable R² is a phenyl group (the phenyl group is unsubstituted or substituted with one halogen atom) or a C₁₋₉ heteroaryl group (the C₁₋₉ heteroaryl group is unsubstituted or substituted with one group selected from the group consisting of a hydroxy group, an amino group and a C₁₋₆ alkyl group). Further preferable R² is a C₁₋₉ heteroaryl group (the C₁₋₉ heteroaryl group is unsubstituted or substituted with one group selected from the group consisting of a hydroxy group, an amino group and a C₁₋₆ alkyl group). Further preferable R² is a triazolyl group (the thiazolyl group is unsubstituted or substituted with one group selected from the group consisting of an amino group and a C₁₋₆ alkyl group) or a thiadiazolyl group.
Another preferable R² is a C₂₋₉ heterocyclyl group (the C₂₋₉ heterocyclyl group is unsubstituted or substituted with one oxo group) and further preferably a 1,6-dihydropyridazinyl group (the 1,6-dihydropyridazinyl group is substituted with one oxo group).
Preferable Ar is a monocyclic C₁₋₆ heteroaryl group represented by the formula (2) (in the formula (2), ring A is unsubstituted or substituted with one or two groups which may be the same or different and selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl group, a C₂₋₆ alkoxycarbonyl C₁₋₆ alkyl group and a di (C₁-₆ alkyl) amino C₁₋₈ alkyl group). Further preferable Ar is a monocyclic C₁₋₆ heteroaryl group represented by the formula (2) (in the formula (2), ring A is unsubstituted or substituted with one group selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl group and a C₂₋₆ alkoxycarbonyl C₁₋₆ alkyl group. Further preferable Ar is a 1H-pyrazol-3-yl group, a 1,3-thiazol-2-yl group, a pyridin-2-yl group, a pyrazin-2-yl group or an isoxazol-3-yl group (the 1H-pyrazol-3-yl group, 1,3-thiazol-2-yl group, pyridin-2-yl group, pyrazin-2-yl group and isoxazol-3-yl group are unsubstituted or substituted with one group selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl group and a C₂₋₆ alkoxycarbonyl C₁₋₆ alkyl group).
Another preferable Ar is a C₂₋₉ heterocyclyl group and further preferably a 4,5-dihydro-1,3-thiazol-2-yl group.
Preferable Y is -NH- or -S- and further preferably -S-.
Preferable Z is -CH₂- or -S- and further preferably, -S-.

In the present invention, a pharmaceutically acceptable salt is, for example, a mineral acid salt such as a hydrochloride, a hydrobromide, a hydroiodide, a phosphate, a sulfate and a nitrate; a sulfonate such as methane sulfonate, ethane sulfonate, benzene sulfonate and p-toluene sulfonate; a carboxylate such as an oxalate, a tartrate, a citrate, a maleate, a succinate, an acetate, a benzoate, a mandelate, an ascorbate, a lactate, a gluconate and a palate; an amino acid salt such as a glycine salt, a lysine salt, an arginine salt, an ornithine salt, a glutamic acid salt and an aspartic acid salt; or an inorganic salt such as a lithium salt, a sodium salt, a potassium salt, a calcium salt and a magnesium salt; or a salt with an organic base, such as an ammonium salt, a triethylamine salt, a diisopropylamine salt and a cyclohexylamine salt. Preferably, a hydrochloride, a, hydrobromide, a phosphate, a sulfate, a methane sulfonate, a p-toluene sulfonate, an oxalate, a tartrate, a citrate, an acetate, a lactate, a glutamic acid salt, an aspartic acid salt, a sodium salt, a potassium salt, an ammonium salt or a triethylamine salt is mentioned.
In the present invention, a solvate represents a pharmaceutically acceptable solvate of a compound of the present invention or a salt thereof. When a compound of the present invention and a salt thereof are exposed to the air or recrystallized, they may absorb moisture and turn into compounds with absorption water and hydrates. The compound of the present invention includes such a hydrate.
Some of the compounds of the present invention have an asymmetric center. In this case, various types of optical isomers are present. Accordingly, a compound of the present invention can be present in the form of a (+) configuration (single substance), a (-) configuration (single substance), a racemic mixture thereof or a (±) mixture containing optically active substances of both configurations in an arbitrary ratio. Furthermore, in the case of a compound having two or more asymmetric centers, diastereomers derived from individual optical isomerism are also present. A compound having all types of isomers in an arbitrary ratio is included in the compound of the present invention. For example, a diastereomer can be separated by a well-known method to one skilled in the art, for example, fractional crystallization. Furthermore, an optically active substance can be obtained by a well known organic chemical method developed for this purpose. Furthermore, some of the compounds of the present invention have geometric isomers such as a cis-isomer and a trans isomer. A compound containing these isomers in an arbitrary ratio is also included in the compound of the present invention.
A pyrazinamide compound of the present invention may be a pharmaceutically acceptable salt thereof or a solvate of these. Hereinafter, a pyrazinamide compound of the present invention including a tautomer, a stereo isomer or a pharmaceutically acceptable salt thereof or a solvate thereof will be referred to as "the compound of the present invention".
Furthermore, a compound generally called prodrug which have a group that can be chemically or metabolically decomposed and is capable of forming a pharmacologically active compound of the present invention by solvolysis or in vivo under physiological conditions are included in "the compound of the present invention".

The compound of the present invention has a GK activation action. Therefore, the compound of the present invention enhances glucose metabolism and glycogen synthesis in the liver, glucose inductive insulin secretion from the pancreatic beta cell, thereby correcting elevated blood glucose. Thus, the compound of the present invention can be used as a new drug that differs from a conventional therapeutic agent for diabetes in mechanism of action. Diabetes includes Type I diabetes, Type II diabetes and other types of diabetes caused by specific causes. Furthermore, the compound of the present invention is effective also for treating and preventing diabetic complications such as ketoacidosis, microangiopathy (retinopathy, nephropathy), arteriosclerosis (atheroma arteriosclerosis symptom, myocardial infarction, cerebral infarction, peripheral artery confinement), neuropathy (of sensory nerves, motor nerves, autonomous nerve), foot gangrene and an infectious disease.
Furthermore, the compound of the present invention can be used for treating and preventing diabetes-related disorders such as obesity, hyperlipemia, hypertension, metabolic syndrome, edema, hyperuricemia and gout.
Furthermore, the compound of the present invention can be used in combination with a therapeutic agent for diabetes, a therapeutic agent for diabetes complication, a therapeutic agent for hyperlipemia and a therapeutic agent for hypertension, etc. whose mechanism of action differs from that of the GK activation action. If the compound of the present invention is used in combination with other pharmaceutical(s), a synergistic effect, which is higher than the sum of the effects obtained by single agents on the aforementioned disorders, can be expected.
Examples of the therapeutic agents for diabetes and therapeutic agents for diabetes complications that can be used in combination include an insulin preparation, an insulin-resistance improving agent (PPARγ agonist, PPARα/γ agonist, PPARδ agonist, PPARα/γ/δ agonist, etc.) (e.g., Pioglitazone, Rosiglitazone, GW-501516, GW-590735, ABT-335, AZD-6,610, AVE-8133), α-glucosidase inhibitor (e.g., Voglibose, Acarbose, Miglitol), a biguanide agent (e.g., Metformin, Buformin, Phenformin), an insulin secretion accelerator (e.g., Glibenclamide, Glimepiride, Repaglinide, Nateglinide, Mitiglinide), an insulin preparation, a glucagon receptor antagonist, an insulin receptor kinase accelerator, dipeptidyl-peptidase IV inhibitor (e.g., Vildagliptin, TS-021, Sitagliptin, BMS-477118), an SGLT inhibitor (e.g., Sergliflozin, Dapagliflozin, KGT-1681, YM543, TS-033, AVE-2268), a PTP1b inhibitor (e.g., sodium vanadate), a glucose-6-phosphatase inhibitor, a glycogen phosphorylase inhibitor (e.g., PSN-357, FR-258900), an FBPase inhibitor (e.g., MB-07803), a PECK inhibitor, a pyruvic acid dehydrogenase kinase inhibitor, a D-chiro inositol, a GSK3 inhibitor, a GLP-1 agonist (e.g., Liraglutide), an amylin agonist (e.g. Pramlintide), a glucocorticoid receptor antagonist, a 11 beta HSD1 inhibitor (e.g., AMG-221, INCB-13739), a protein kinase C inhibitor (e.g., Ruboxistaurin), a Beta-3 adrenaline receptor agonist (e.g., AJ-9677), a phosphatidyl inositol kinase inhibitor, a phosphatidyl inositol phosphatase inhibitor, an ACC inhibitor, a GPR40 receptor agonist, a GPR119 receptor agonist (e.g., APD-668), TGR5 receptor agonist, an AMPS activating agent (e.g., DRL-16536), an aldose reductase inhibitor and an AGE inhibitor.
Examples of pharmaceuticals for diabetes-related disorders that can be used in combination include a HMG-CoA reductase inhibitor, a squalene synthetase inhibitor, a bile acid adsorbent, an IBAT inhibitor, a CETP inhibitor, a CPT inhibitor, a vibrate pharmaceutical, an ACAT inhibitor, an MGAT inhibitor, a DRAT inhibitor, a cholesterol absorption inhibitor, a pancreatic lipase inhibitor, an MTP inhibitor, a nicotinic acid derivative, an LXR agonist, an LDL receptor accelerator, an angiotensin-converting enzyme inhibitor, an angiotensin II antagonist, a diuretic agent, a calcium antagonist, an endothelin-converting enzyme inhibitor, an endothelin receptor antagonist, an anorexigenic agent, a uric acid generation inhibitor and a uric acid excretion stimulator.
The compound of the present invention can be administered singly or in combination with a pharmaceutically or pharmacologically acceptable carrier or diluent. When the compound of the present invention is used as a GK activating substance or the like, the compound of the present invention may be directly administered orally or parenterally. Furthermore, an agent containing the compound of the present invention as an active ingredient may be administered orally or parenterally. Examples of parenteral administration include an intravenous administration, transnasal administration, transdermal administration, subcutaneous administration, intramuscular administration and sublingual administration.
The dose of the compound of the present invention varies depending upon an administration target, an administration route, a subject disorder and a symptom, etc. For example, when it is orally administered to an adult diabetic patient, one dose is usually about 0.01 to 100 mg/kg weight, preferably 0.05 to 30 mg/kg weight and further preferably 0.1 to 10 mg/kg weight. This dose is desirably administered once to three times per day.

The compound of the present invention can be synthesized by the process shown below; however, the following production process is an example of a general production process and will not limit the production process.

### Scheme 1: Production process for compound (1-e) from compound (1-a)

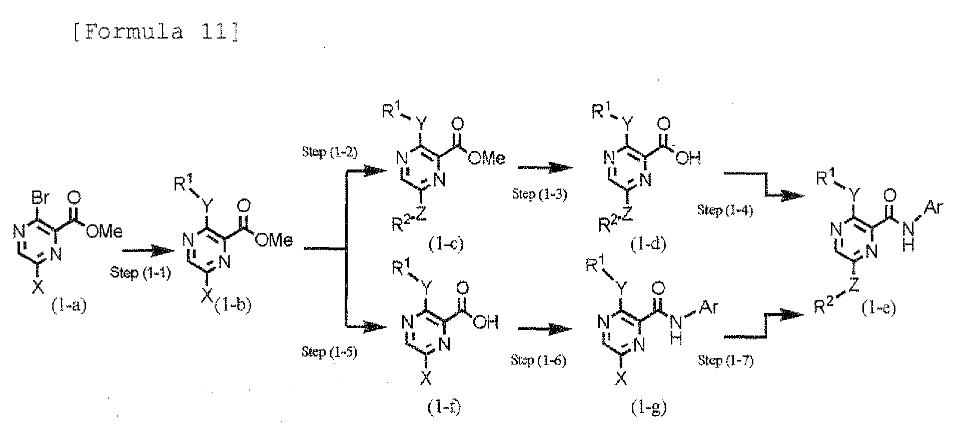

(In the Scheme, R¹, R², Y and Ar are the same as defined above; X represents Br or atom; and Z represents -S-or -O-).
Step (1-1): a when Y is -O- or -S-, a compound (1-a) and a compound represented by the formula R¹-Y-H are reacted in a solvent in the presence of a base such as potassium carbonate or sodium hydride to successfully produce a compound (1-b). When Y is -NH-, a compound (1-a) is reacted with a compound represented by the formula R¹-NH₂, which is also serves as a solvent, to successfully produce a compound (1-b).
Step (1-2): in a solvent, the compound (1-b) is reacted with a compound represented by the formula R²-Z-H in the presence of a base such as potassium carbonate to successfully produce a compound (1-c).
Step (1-3): in a solvent, the compound (1-c) is hydrolyzed by use of a base such as an aqueous sodium hydroxide solution to successfully produce a compound (1-d).
Step (1-4): in a solvent, the compound (1-d) is reacted with a compound represented by the formula Ar-NH₂ in the presence of a condensing agent such as dicyclohexylcarbodiimide, 1-(3-dimethylaminopropyl)-3-ethylcarbodiimide hydrochloride or 1-hydroxybenzotriazole and in the presence or absence of a base such as triethylamine or diisopropylethylamine to successfully produce the compound of a present invention (1-e).
In the case where a C₂₋₆ alkoxycarbonyl group is present in the Ar moiety, it is hydrolyzed by use of a base such as an aqueous sodium hydroxide solution to be successfully converted into a carboxy group. In the case where a C₁₋₆ alkoxy group is present in the R¹ moiety, it is reacted by use of a Lewis acid such as boron tribromide to be successfully converted into a hydroxy group.
Step (1-5): in a solvent, the compound (1-b) is hydrolyzed by use of a base such as an aqueous sodium hydroxide solution to successfully produce a compound (1-f).
Step (1-6): the compound (1-f) and a compound represented by the formula Ar-NH₂ are reacted in a solvent in the presence of a condensing agent such as dicyclohexylcarbodiimide, 1-(3-dimethylaminopropyl)-3-ethylcarbodiimide hydrochloride or 1-hydroxybenzotriazole and in the presence or absence of a base such as triethylamine or diisopropylethylam-ine to successfully produce a compound (1-g).
Step (1-7): in a solvent, the compound (1-g) and a compound represented by the formula R²-Z-H are reacted in the presence of a base such as potassium carbonate to successfully produce the compound of the present invention (1-e).

### Scheme 2: Production process for compound (2-g) from compound (2-a)

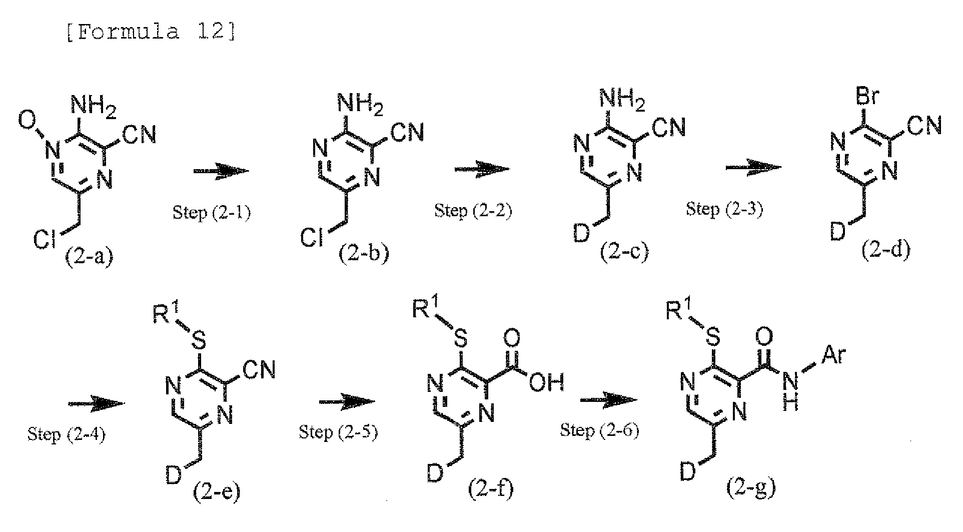

(In the Scheme, R¹ and Ar are the same as defined above; and D is a group represented by the following formula (α) : 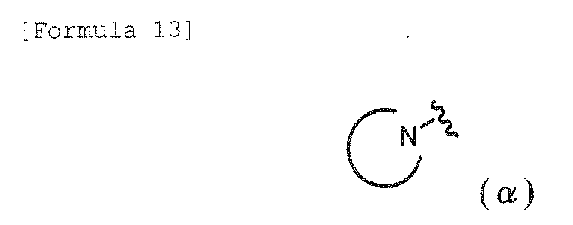 (the formula (α) represents a C₂₋₉ heterocyclyl group or a C₁₋₉ heteroaryl group)).

Step (2-1): in a solvent, a compound (2-a) is reacted with phosphorus trichloride to successfully produce a compound (2-b).
Step (2-2): in a solvent, the compound (2-b) is reacted with a compound represented by the formula D-H, in the presence of a base such as potassium carbonate or sodium hydride to successfully produce a compound (2-c).
Step (2-3): in a solvent, the compound (2-c) is reacted with sodium nitrite in the presence of an acid such as hydrobromic acid and subsequently reacted with bromine to successfully produce a compound (2-d).
Alternatively, in a solvent, the compound (2-c) is reacted with a nitrite compound such as t-butyl nitrite in the absence of an acid and subsequently reacted with a brominating agent such as copper (II) bromide to successfully produce compound (2-d).
Step (2-4): in a solvent, the compound (2-d) is reacted with a compound represented by the formula R¹-SH in the presence of a base such as potassium carbonate to successfully produce a compound (2-e).
Step (2-5): in a solvent, the compound (2-e) is hydrolyzed by use of a base such as an aqueous sodium hydroxide solution to successfully produce a compound (2-f).
Step (2-6): in a solvent, the compound (2-f) is reacted with a compound represented by the formula Ar-NH₂ in the presence of a condensing agent such as dicyclohexylcarbodiimide, 1-(3-dimethylaminopropyl)-3-ethylcarbodiimide hydrochloride or 1-hydroxybenzotriazole in the presence or absence of a base such as triethylamine or diisopropylethylamine to successfully produce the compound of the present invention (2-g).

### Scheme 3: Production process for compound (3-b) from compound (2-c)

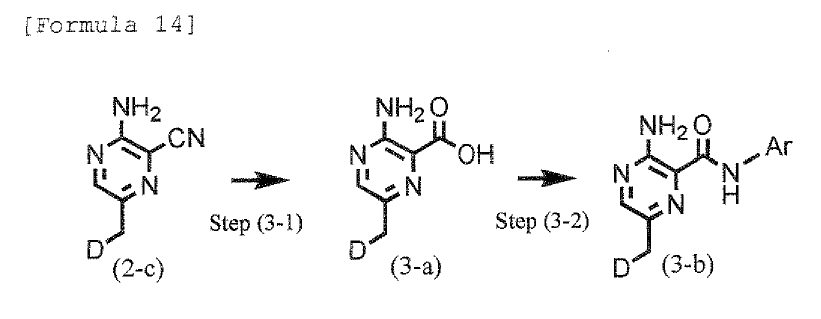

(In the Scheme, Ar is the same as defined above; and D represents a C₂₋₉ heterocyclyl group represented by the formula (α))
Step (3-1): in a solvent, the compound (2-c) is hydrolyzed by use of a base such as an aqueous sodium hydroxide solution to successfully produce a compound (3-a).
Step (3-2): in a solvent, the compound (3-a) and a compound represented by the formula Ar-NH₂ are reacted in the presence of a condensing agent such as dicyclohexylcarbodiimide, 1-(3-dimethylaminopropyl)-3-ethylcarbodiimide hydrochloride or 1-hydroxybenzotriazole and in the presence or absence of a base such as triethylamine or diisopropylethylamine to successfully produce the compound of the present invention (3-b).

### Scheme 4: Production process for compound (4-d) from compound (4-a)

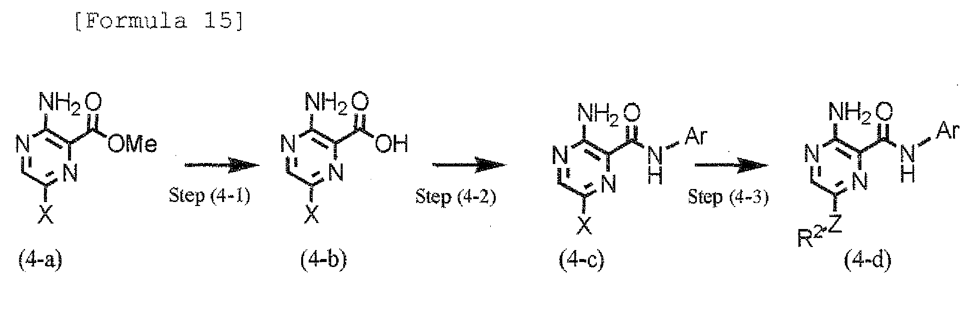

(In the Scheme, Ar and X are the same as defined above; R² represent a C₁₋₉ heteroaryl group; and Z represents -S- or -O-).
Step (4-1): in a solvent, the compound (4-a) is hydrolyzed by use of a base such as an aqueous sodium hydroxide solution to successfully produce a compound (4-b).
Step (4-2): in a solvent, the compound (4-b) and a compound represented by the formula Ar-NH₂ are reacted in the presence of a condensing agent such as dicyclohexylcarbodiimide, 1-(3-dimethylaminopropyl)-3-ethylcarbodiimide hydrochloride or 1-hydroxybenzotriazole and in the presence or absence of a base such as triethylamine or diisopropylethylamine to successfully produce a compound (4-c).
Step (4-3): in a solvent, the compound (4-c) and a compound represented by the formula R²-Z-H are reacted in the presence of a base such as potassium carbonate by use of a palladium reagent such as tris(dibenzylideneacetone)palladium and a phosphine ligand such as 4,5-bis(diphenylphosphino)-9,9-dimethylxanthene to successfully produce the compound of the present invention (4-d).

### Scheme 5: Production process for compound (5-e) from compound (5-a)

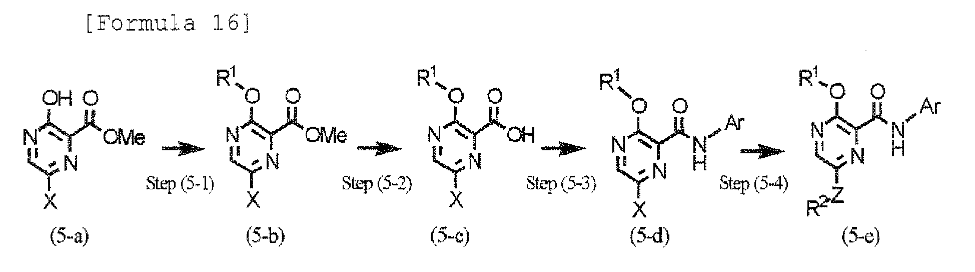

(In the Scheme, Ar and X are the same as defined above; Z represents -S- or -O-; 1 represents a C₂₋₉ heterocyclyl group; and R² represents a C₁₋₉ heteroaryl group).
Step (5-1): in a solvent, a compound (5-a), a phosphine reagent such as triphenylphosphine or tributylphosphine and an azo reagent such as diethylazodicarboxylate or diisopropylazodicarboxylate are reacted and subsequently, the Mitsunobu reaction is carried out by use of a compound represented by the formula R¹-OH to successfully produce a compound (5-b).
Step (5-2): in a solvent, the compound (5-b) is hydrolyzed by use of a base such as an aqueous sodium hydroxide solution to successfully produce a compound (5-c).
Step (5-3): in a solvent, the compound (5-c) and a compound represented by the formula Ar-NH₂ are reacted in the presence of a condensing agent such as dicyclohexylcarbodiimide, 1-(3-dimethylaminopropyl)-3-ethylcarbodiimide hydrochloride or 1-hydroxybenzotriazole and in the presence or absence of a base such as triethylamine or diisopropylethylamine to successfully produce a compound (5-d).
Step (5-4): in a solvent, the compound (5-d) and a compound represented by the formula R²-Z-H are reacted in the presence of a base such as potassium carbonate to successfully produce the compound of the present invention (5-e).
A general production process for a pharmaceutically acceptable salt of the compound of the present invention is as follows. In the case of a compound forming an acid addition salt, the compound is dissolved in an appropriate solvent and mixed with an acid such as hydrochloric acid and oxalic acid directly or in a dissolved state. The resultant precipitate or a precipitate obtained by adding a poor solvent is obtained by filtration as a desired product. In this manner, the corresponding acid addition salt can be produced. Furthermore, in the case of a compound of the present invention forming a base addition salt, the compound is dissolved in an appropriate solvent and mixed with a base such as sodium hydroxide, potassium hydroxide and arginine directly or in a dissolved state. The resultant precipitate or a precipitate obtained by adding a poor solvent is obtained by filtration as a desired product. In this manner, the corresponding base addition salt can be produced.

As the base according to a general process for producing the compound of the present invention, for example, alkali metal salts such as sodium carbonate, potassium carbonate, cesium carbonate, sodium hydrogen carbonate, potassium hydrogen carbonate, sodium hydroxide, sodium hydride, lithium hydride, sodium amide, potassium t-butoxide, n-butyl lithium and lithium diisopropylamide, amines such as triethylamine, diisopropylethylamine, pyrrolidine and piperidine, sodium acetate, and potassium acetate can be mentioned.
The solvent according to a general process for producing the compound of the present invention is not particularly limited as long as it is stable and labile in the reaction conditions and it does not inhibit the reaction. For example, water, alcohols such as methanol, ethanol, i-propyl alcohol, n-butanol and t-butyl alcohol, ethers such as dioxane and tetrahydrofuran, dimethylformamide, dimethylacetamide, dimethyl sulfoxide, pyridine, ethyl acetate, methylene chloride, chloroform, acetone, acetic acid, benzene, toluene and solvent mixtures of these can be mentioned.
As the reaction temperature according a general process for producing the compound of the present invention, an appropriate temperature can be selected within the range from -78°C to a boiling point of the solvent to be used in the reaction. The production process of the present invention can be performed under normal pressure, under pressure and irradiation of microwave etc.

### EXAMPLES

Examples and experimental examples will be shown below in order to specifically describe compounds of the present invention in detail; however, the present invention is not limited to these examples.

### (Referential Synthesis Example 1)

### Production of 3-bromo-6-iodopyrazine-2-carboxylic acid methyl ester and 3-hydoxy-6-iodopyrazine-2-carboxylic acid methyl ester

To a dimethylformamide (60 ml) suspension of copper (II) bromide (19.2 g, 86.0 mmol) raised in temperature to 50°C, t-butyl nitrite (5.7 ml, 43.0 mmol) was added and subsequently a dimethylformamide (60 ml) suspension of 3-amino-6-iodopyrazine-2-carboxylic acid methyl ester (see Patent Document: WO2006/015124)(12.0 g, 43.0 mmol) was added dropwise and the mixture was stirred for one hour. The reaction mixture was charged with t-butyl nitrite (11.4 ml, 86.0 mmol) and was further stirred for 30 minutes at an external temperature of 50°C. The reaction solution was poured into a 3 M aqueous hydrochloride solution (60 ml) of sulfamic acid (7.6 g) under ice cooling and extracted with ethyl acetate, dried (over anhydrous magnesium sulfate), filtered and concentrated to obtain a residue, which was purified by silica gel column chromatography [developing eluent: hexane: ethyl acetate = 5:1 to 1:1]. The resultant low polarity product was washed with hexane-ethyl acetate to obtain 3-bromo-6-iodopyrazine-2-carboxylic acid methyl ester (6.38 g) in the form of a white solid substance. A high-polarity product was washed with ethyl acetate to obtain 3-hydoxy-6-iodopyrazine-2-carboxylic acid methyl ester (2.70 g) in the form of a yellow solid substance.
3-Bromo-6-iodopyrazine-2-carboxylic acid methyl ester
MS (ESI): 342 (M+1)
1H NMR (300 MHz, CDC13) δ ppm 4.02 (d, J = 0.55 Hz, 3 H) 8.72 (s, 1 H)
3-Hydoxy-6-iodopyrazirie-2-carboxylic acid methyl ester
MS (ESI): (ES+) 281 (ES-) 279
1H NMR (300 MHz, DMSO) δ ppm 3.82 (s, 2.53 H) 3.84 (s, 0.47 H) 8.35 (s, 0.78 H) 8.39 (s, 0.22 H) 12.69 - 13.03 (br, 1 H)

### (Example 1)

### Production of 6-[(4-fluorophenyl)sulfanyl)-N-(5-methyl-1,3-thiazol-2-yl)-3-(4H-1,2,4-triazol-3-ylsulfanyl)pyrazine-2-carboxamide (Compound No. 1)

(1) To a dimethylformamide (15 ml) solution of 3,6-dibromopyrazine-2-carboxylic acid methyl ester (see Patent Document: BE662507)(780 mg, 2.64 mmol), potassium carbonate (364 mg, 2.64 mmol)) and 4-fluorothiophenol (0.253 ml, 2.37 mmol) were sequentially added at room temperature and the mixture was stirred at room temperature for one hour. The reaction solution was charged with water, extracted with chloroform and then dried (over anhydrous magnesium sulfate), filtered and concentrated to obtain a residue, which was purified by silica gel column chromatography [developing eluent: chloroform hexane = 1:2 to 10:0] and purified by recycle preparative HPLC (LC-908 type, Japan Analytical Industry Co. , Ltd.) [developing eluent: chloroform] to obtain a mixture of 6-bromo-3-[(4-fluorophenyl)sulfanyl]pyrazine-2-carboxylic acid methyl ester and 3-bromo-6-[(4-fluorophenyl)sulfanyl]pyrazine-2-carboxylic acid methyl ester (940 mg) in the form of a light yellow solid substance.
(2) To a dimethylformamide (8 ml) solution of the mixture (406 mg, 1.18 mmol) of 6-bromo-3-[(4-fluorophenyl)sulfanyl]pyrazine-2-carboxylic acid methyl ester and 3-bromo-6-[(4-flucrophenyl)sulfany-l]pyrazine-2-carboxylic acid methyl ester, potassium carbonate (326 mg, 2.36 mmol) and 3-mercapto-4H-1,2,4-triazole (238 mg, 2.36 mmol) were sequentially added at room temperature and the mixture was stirred at an external temperature of 50°C for 2.5 hours. The reaction solution was filtered to remove potassium carbonate. The residue obtained by concentrating the filtrate was charged with chloroform and extracted twice with water and then, the water phase was concentrated to obtain crude 6-[(4-fluorophenyl)sulfanyl]-3-(4H-1,2,4-triazol-3-ylsulfanyl)pyrazine-2-carboxylic acid.
(3) To a dimethylformamide (11.4 ml) solution of the 6-[(4-fluorophenyl)sulfanyl]-3-(4H-1,2,4-triazol-3-ylsulfanyl)pyrazine-2-carboxylic acid, 1-(3-dimethylaminopropyl)-3-ethylcarbodiimide hydrochloride (469 mg, 2.45 mmol), 1-hydroxybenzotriazole (331 mg, 2.45 mmol) and 2-amino-5-methylthiazole (280 mg, 2.45 mmol) were sequentially added at room temperature and the mixture was stirred at an external temperature of 100°C for 30 minutes. The reaction solution was charged with water, extracted with chloroform and then dried (over anhydrous magnesium sulfate), filtered and concentrated to obtain a residue. To the residue, chloroform was added and the mixture was washed with a 1 M aqueous hydrochloric acid solution and a saturated aqueous sodium hydrogen carbonate solution, dried (over anhydrous magnesium sulfate), filtered and concentrated to obtain a residue, which was purified by silica gel column chromatography [developing eluent: chloroform: methanol = 10:1]. The resultant solid substance was further washed with chloroform to obtain 6-[(4-fluorophenyl)sulfanyl]-N-(5-methyl-1,3-thiazol-2-yl)-3-(4H-1,2,4-triazol-3-ylsulfanyl)pyrazine-2-carboxamide (63.4 mg) in the form of a yellow solid substance.

### (Example 2)

### Production of 3-[(4-fluorophenyl)sulfanyl]-N-(1-methyl-1H-pyrazol-3-yl)-6-(4H-1,2,4-triazol-3-ylsulfanyl)pyrazine-2-carboxamide (Compound No. 2)

(1) To a dimethylformamide (300 ml) solution of 3,6-dibromopyrazine-2-carboxylic acid methyl ester (7.47 g, 25.2 mmol), potassium carbonate (3.48 g, 25.2 mmol) and 4-fluorothiophenol (2.42 ml, 22.7 mmol) were sequentially added at room temperature and the mixture was stirred at room temperature for 30 minutes. The reaction solution was charged with water, extracted with ethyl acetate and then washed (with water and brine in this order). The water phases were combined and re-extracted with chloroform. The organic phases were combined, dried (over anhydrous magnesium sulfate), filtered and concentrated to obtain a residue, which was purified by silica gel column chromatography [developing eluent: hexane: ethyl acetate = 25:1 to 10:1] to obtain a mixture (9.29 g) of 6-bromo-3-[(4-fluorophenyl)sulfanyl]pyrazine-2-carboxylic acid methyl ester, 3-bromo-6-1(4-fluorophenyl)sulfanyl]pyrazine-2-carboxylic acid methyl ester and 3,6-bis[(4-fluorophenyl)sulfanyl]pyrazine-2-carboxylic acid methyl ester.
(2) To a dimethylformamide (180 ml) solution of the above mixture (9.29 g), potassium carbonate (3.13 g, 22.7 mmol) and 3-mercapto-4H-1,2,4-triazole (2.29 g, 22.7 mmol) were sequentially added at room temperature and the mixture was stirred at an external temperature of 50°C for 3.5 hours. The reaction solution was charged with water, extracted with chloroform and then washed with (brine). The water phase was re-extracted with chloroform. The organic phases were combined and dried (over anhydrous magnesium sulfate), filtered and concentrated to obtain a residue, which was purified with silica gel column chromatography [developing eluent: chloroform: methanol =10:0 to 20:1] and washed with hexane-ethyl acetate (2:1) to obtain 3-[(4-fluorophenyl)sulfanyl]-6-(4H-1,2,4-triazol-3-ylsulfanyl)pyrazine-2-carboxylic acid methyl ester (3.06 g) in the form of a light yellow solid substance.
   MS (ESI): 364 (M+1), 362 (M-1)
   1H NMR (300 MHz, DMSO-D6) δ ppm 3.94 (s, 3H) 7.30 (t, J = 9.1 Hz, 2 H) 7.55 - 7.60 (m, 2 H) 8.35 (s, 1H), 8.63 - 8.78 (br, 1 H)
(3) To an ethanol (30 ml) solution of 3-[(4-fluarophenyl)sulfanyl]-6-(4H-1,2,4-triazol-3-ylsulfanyl)pyrazine-2-carboxylic acid methyl ester (3.06 g, 8.42 mmol), a 1 M aqueous sodium hydroxide solution (16.8 ml) was added at room temperature and the mixture was stirred at room temperature for one hour. To the reaction solution, a 1 M aqueous hydrochloric acid solution (16.8 ml) was added. The solid substance precipitated was filtered off and washed with ethanol and water to obtain 3-[(4-fluorophenyl)sulfanyl]-6-(4H-1,2,4-triazol-3-ylsulfanyl)pyrazine-2-carboxylic acid (2.41 g) in the form of a yellow solid substance. After the filtrate was distilled away, the residue was extracted with chloroform and then dried (over anhydrous magnesium sulfate), filtered and concentrated to obtain 3-[(4-fluorophenyl)sulfanyl]-6-(4H-1,2,4-triazol-3-ylsulfanyl)pyrazine-2-carboxylic acid (0.22 g)in the form of a yellow solid substance.
   MS (ESI): 350 (M+1), 348 (M-1)
   1H NMR (300 MHz, DMSO-D6) δ ppm 7.29 (t, J = 9.0 Hz, 2 H) 7.54 - 7.59 (m, 2 H) 8.32 (s, 1 H) 8.56 - 8.83 (br, 1 H) 13.89 - 14.30 (br, 1 H), 14.42 - 14.70 (br, 1 H)
(4) To a dimethylformamide (26ml) solution of 3-((4-fluorophenyl)sulfayl]-6-(4H-1,2,4-triazol-3-ylsulfanyl)pyrazine-2-carboxylic acid (2.63 g, 7.53 mmol), 1-(3-dimethylaminopropyl)-3-ethylcarbodiimide hydrochloride (2.17 g, 11.3 mmol), 1-hydroxybenzotriazole (1.53 g, 11.3 mmol), 3-amino-1-methylpyrazole hydrochloride (1.51 g, 11.3 mmol) and triethylamine (1.9 ml, 13.6 mmol) were sequentially added at room temperature, and the mixture was stirred at room temperature for one hour. The reaction solution was charged with water, extracted with ethyl acetate and then washed (with saturated aqueous ammonium chloride solution). The water phases were combined and extracted with chloroform. The organic phases were combined and dried (over anhydrous magnesium sulfate), filtered and concentrated to obtain a residue, which was purified with silica gel column chromatography [developing eluent: chloroform: methanol = 50:1] and dissolved in ethanol (160 ml) while heating. To the solution, water (160 ml) was added at 80°C, and then cooled to room temperature. The solid substance precipitated was filtered and washed with ethanol-water (1:1) to obtain 3-[(4-fluorophenyl)sulfanyl]-N-(1-methyl-1H-pyrazol-3-yl)-6-(4H-1,2,4-triazol-3-ylsulfanyl)pyrazine-2-carboxamide (3.00 g) in the form of a yellow solid substance.

### (Example 3)

According to the production process of Example 2, 3,6-dibromopyrazine-2-carboxylic acid methyl ester was used or 3-broma-6-iodopyrazine-2-carboxylic acid methyl ester was used in place; 3-amino-1-methylpyrazole was used or, 2-amino-5-methylthiazole, 6-aminonicotinic acid methyl ester, 2-(3-amino-1H-pyrazol-1-yl)acetic acid ethyl ester, 3-amino-1-(2-hydroxyethyl)pyrazole, 3-amino-1 (dimethylamino)ethyl]pyrazole, 3-amino-1-(2 methoxyethyl)pyrazole, 3-amino-1,5-dimethylpyrazole, 2-amino-2-thiazoline, 2-amino-5-chlorothiazole, 3-amino-5-methyl-isoxazole, 6-aminonicotinamide, or aminopyrazine was used in place; 4-fluorothiophenol was used or, in place, 2-propanethiol, cyclohexanethiol, 4-methylthiophenol, 4-methoxythiophenol, 4-(methylsulfanyl)thiophenol, benzylmercaptan, 4-(methylsulfonyl)thiophenol, (S)-1-phenylpropane-2-thiol, 4-(trifluoromethyl)thiophenol, tetrahydro-2H-pyran-4-thiol, 3,4-dimethoxythiophenol, 3-methoxythiophenol, 2-methoxythiophenol, 3-mercapto-6-methylpyridine or 4-mercaptopyridine was used; and 3-mercapto-4H-1,2,4-triazole was used or, in place, 3-mercapta-5-methyl-4H-1,2,4-triazole, 4-fluorothiophenol or 4-mercaptopyridine was used, to obtain the following compounds of the present invention:
3-[(4-fluorophenyl)sulfanyl]-N-(5-methyl-1,3-thiazol-2-yl)-6-(4H-1,2,4-triazol-3-ylsulfanyl)pyrazine-2-carboxamide (Compound No. 3),
3-[(1-methylethyl)sulfanyl]-N-(1-methyl-1H-pyrazol-3-yl)-6-(4H-1,2,4-triazol-3-ylsulfanyl)pyrazine-2-carboxamide (Compound No. 4),
3-(cyclohexylsulfanyl)-N-(1-methyl-1H-pyrazol-3-yl)-6-(4H-1,2,4-triazol-3-ylsulfanyl)pyrazine-2-carboxamide (Compound No. 5),
3-[(4-methylphenyl)sulfanyl]-N-(1-methyl-1H-pyrazol-3-yl)-6-(4H-1,2,4-triazol-3-ylsulfanyl)pyrazine-2-carboxamide (Compound No. 6),
3-[(4-methoxyphenyl)sulfanyl]-N-(1-methyl-1H-pyrazol-3-yl)-6-(4H-1,2,4-triazol-3-ylsulfanyl)pyrazine-2-carboxamide (Compound No. 7),
N-(1-methyl-1H-pyrazol-3-yl)-3-{[4-(methylsulfanyl)phenyl]sulfanyl}-6-(4H-1,2,4-triazol-3-ylsulfanyl)pyrazine-2-carboxamide (Compound No. 8),
3-(benzylsulfanyl)-N-(1-methyl-1H-pyrazol-3-yl)-6-(4H-1,2,4-triazol-3-ylsulfanyl)pyrazine-2-carboxamide (Compound No. 9),
N-(1-me-thyl-1H-pyrazol-3-yl)-3-([4-(methylsulfonyl)phenyl]sulfanyl}-6-(4H-1,2,4-triazol-3-ylsulfanyl)pyrazine-2-carboxamide (Compound No. 10),
3-{[(1S)-1-methy-2-phenylethyl]sulfanyl}-N-(1-methyl-1H-pyrazol-3-yl)-6-(4H-1,2,4-triazol-3-ylsulfanyl)pyrazine-2-carboxamide (Compound No. 11),
6-[({3-[(4-fluorophenyl)sulfanyl]-6-(4H-1,2,4-triazol-3-ylsulfanyl)pyrazin-2-yl}carbonyl)amino]pyridine-3-carboxylic acid methyl ester (Compound No. 12),
(3-[({3-[(4-fluorophenyl)sulfanyl]-6-(4H-1,2,4-triazol-3-ylsulfanyl)pyrazin-2-yl}carbonyl)amino]-1H-pyrazol-1-yl}acetic acid ethyl ester (Compound No. 13),
3-[(4-fluorophenyl)sulfanyl]-N-[1-(2-hydroxyethyl)-1H-pyrazol-3-yl]-6-(4H-1,2,4-triazol-3-ylsulfanyl)pyrazine-2-carboxamide (Compound No. 14),
N-{1-[2-(dimethylamino)ethyl]-1H-pyrazol-"3-" yl}-3-[(4-fluorophenyl)sulfanyl]-6-(4H-1,2,4-triazol-3-ylsulfanyl)pyrazine-2-carboxamide (Compound No. 15),
3-[(4-fluorophenyl)sulfanyl]-N-[1-(2-methoxyethyl)-1H-pyrazol-3-yl]-6-(4H-1,2,4-triazol-3-ylsulfanyl)pyrazine-2-carboxamide (Compound No. 16),
N-(1,5-dimethyl-1H-pyrazol-3-yl)-3-[(4-fluorophenyl)sulfanyl]-6-(4H-1,2,4-triazol-3-ylsulfanyl)pyrazine-2-carboxamide (Compound No. 17),
N-(1-methyl-1H-pyrazol-3-yl)-6-(4H-1,2,4-triazol-3-ylsulfanyl)-3-{([4-(trifluoromethyl)phenyl]sulfanyl}pyrazine-2-carboxamide (Compound No. 18),
N-(1-methyl-1H-pyrazol-3-yl)-3-(tetrahydro-2H-pyran-4-ylsulfanyl)-6-(4H-1,2,4-triazol-3-ylsulfanyl)pyrazine-2-carboxamide (Compound No. 19),
3-[(3,4-dimethoxyphenyl)sulfanyl]-N-(1-methyl-1H-pyrazol-3-yl)-6-(4H-1,2,4-triazol-3-ylsulfanyl)pyrazine-2-carboxamide (Compound No. 20),
3-[(3-methoxyphenyl)sulfanyl]-N-(1-methyl-1H-pyrazol-3-yl)-6-(4H-1,2,4-triazol-3-ylsulfanyl)pyrazine-2-carboxamide (Compound No. 21),
3-[(2-methoxyphenyl)sulfanyl]-N-(1-methyl-1H-pyrazol-3-yl)-6-(4H-1,2,4-triazol-3-ylsulfanyl)pyrazine-2-carboxamide (Compound No. 22),
N-(1-methyl-1H-pyrazol-3-yl)-3-[(6-methylpyridin-3-yl)sulfanyl]-6-(4H-1,2,4-triazol-3-ylsulfanyl)pyrazine-2-carboxamide (Compound No. 23),
N-(1-methyl-1H-pyrazol-3-yl)-3-(pyridin-4-ylsulfanyl)-6-(4H-1,2,4-triazol-3-ylsulfanyl)pyrazine-2-carboxamide (Compound No . 24),
N-(4,5-dihydro-1,3-thiazol-2-yl)-3-[(4-fluorophenyl)sulfanyl]-6-(4H-1,2,4-triazol-3-ylsulfanyl)pyrazine-2-carboxamide (Compound No. 25),
N-(5-chloro-1,3-thiazol-2-yl)-3-[(4-fluorophenyl)sulfanyl]-6-(4H-1,2,4-triazol-3-ylsulfanyl)pyrazine-2-carboxamide (compound No. 26),
3-[(4-fluorophenyl)sulfanyl-]-N-(5-methylisoxazol-3-yl)-6-(4H-1,2,4-triazol-3-ylsulfanyl)pyrazine-2-carboxamide (compound No. 27),
N-(5-carbamoylpyridin-2-yl)-3-[(4-fluorophenyl)sulfanyl]-6-(4H-1,2,4-triazol-3-ylsulfanyl)pyrazine-2-carboxamide (Compound No. 28),
3-[(4-fluorophenyl)sulfanyl]-N-(pyrazin-2-yl)-6-(4H-1,2,4-triazol-3-ylsulfanyl)pyrazine-2-carboxamide (Compound No. 29),
3-[(4-fluorophenyl)sulfanyl-N-[1-(2-methoxyethyl)-1H-pyrazol-3-yl]-6-[(5-methyl-4H-1,2,4-triazol-3-yl)sulfanyl]pyrazine-2-carboxamide (Compound No. 30),
3,6-bis[(4-fluorophenyl)sulfanyl]-N-(1-methyl-1H-pyrazol-3-yl)pyrazine-2-carboxamide (Compound No. 31),
3-[(4-methoxyphenyl)sulfanyl]-N-(5-methyl-1,3-thiazol-2-yl)-6-(4H-1,2,4-triazol-3-ylsulfanyl)pyrazine-2-carboxamide (Compound No. 32),
N-(1-methyl-1H-pyrazol-3-yl)-3,6-bis(pyridin-4-ylsulfanyl)pyrazine-2-carboxamide (Compound No. 33).

### (Example 4)

### Production of 3-[(4-hydroxyphenyl)sulfanyl]-N-(5-methyl-1,3-thiazol-2-yl)-6-(4H-1,2,4-triazol-3-ylsulfanyl)pyrazine-2-carboxamide (Compound No. 34)

To a dichloromethane (4 ml) solution of a 3-[(4-methoxyphenyl)sulfanyl]-N-(5-methyl-1,3-thiazol-2-yl)-6-(4H-1,2,4-triazol-3-ylsulfanyl)pyrazine-2-carboxamide (100 mg, 0.22 mmol) obtained in Example 3, a 1 M boron tribromide dichloromethane solution (0.33 ml) was added at -78°C and the mixture was stirred at room temperature for one hour and 20 minutes. Furthermore, a 1 M boron tribromide dichloromethane solution (0.66 ml) was added at -78°C and the mixture was stirred at room temperature for 2 hours. Moreover, the 1 M boron tribromide dichloromethane solution (0.66 ml) was added at -78°C and the mixture was stirred at room temperature for 2 hours. After methanol was added, the solvent was distilled away. The resultant residue was sequentially washed with ethyl acetate, chloroform and methanol to obtain 3-[(4-hydroxyphenyl)sulfanyl]-N-(5-methyl-1,3-thiazol-2-yl)-6-(4H-1,2,4-triazol-3-ylsulfanyl)pyrazine-2-carboxamide (68.5 mg).

### (Example 5)

### Production of 6-[({3-[(4-fluorophenyl)sulfanyl]-6-(4H-1,2,4-triazol-3-ylsulfanyl)pyrazin-2-yl}carbonyl)amino]pyridine-3-carboxylic acid (Compound No. 35)

To a methanol(3 ml) and tetrahydrofuran (3 ml) solution of 6-[({3-[(4-fluorophenyl)sulfanyl]-6"-(4H-1,2,4-triazol-3-ylsulfanyl)pyrazin-2-yl}carbonyl)amino]pyridine-3-carboxylic acid methyl ester (81.1 mg, 0.17 mmol) obtained in Example 3, a 1 M aqueous sodium hydroxide solution (0.37 ml) was added at room temperature and the mixture was stirred at room temperature for one hour and further at an external temperature of 50°C for 2 hours. To the reaction solution a 1 M aqueous hydrochloric acid solution (0.37 ml) was added. After the reaction solution was extracted with chloroform, the reaction solution was allowed to stand still for one day. A solid substance precipitated was filtered off and further washed with methanol to obtain 6-[({3"[(4-fluorophenyl)sulfanyl]-6-(4H-1,2,4-triazol-3-ylsulfanyl)pyrazin-2-yl}carbonyl)amino]pyridine-3-carboxylic acid (9.4 mg) in the form of a yellow solid substance.

### (Example 6)

According to the production process of Example 5, {3-[({3-[(4-fluorophenyl)sulfanyl]-6-(4H-1,2,4-triazol-3-yls-ylsulfanyl)pyrazin-2-yl}carbonyl)amino]-1H-pyrazol-1-yl}acetic acid ethyl ester was used in place of 6-[(13-[(4-fluorophenyl)sulfanyl]-6-(4H-1,2,4-triazol-3-ylsulfanyl)pyrazin-2-yl}carbonyl)amino]pyridine-3-carboxylic acid methyl ester to obtain the following compound of the present invention:
{3-[({3-[(4-fluorophenyl)sulfanyl]-6-(4H-1,2,4-triazol-3-ylsulfanyl)pyrazin-2-yl}carbonyl)amino]-1H-pyrazol-1-yl}acetic acid (Compound No. 36)

### (Example 7)

### Production of 3-[(4-fluorophenyl)sulfanyl]-N-(1-methyl-1H-pyrazol-3-yl)-6-[(4-methyl-4H-1,2,4-triazol-3-yl)sulfanyl]pyrazine-2-carboxamide (Compound No. 37)

(1) To a dimethylformamide (160 ml) solution of 3,6-dibromopyrazine-2-carboxylic acid methyl ester (4.0 g, 13.5 mmol), 4-fluorothiophenol(1.36 ml, 12.8 mmol)) and potassium carbonate (1.86 g, 13.5 mmol) were added and the mixture was stirred for 2 hours at room temperature. The reaction solution was poured into water, extracted with ethyl acetate and washed (with water and brine, in this order), dried (over anhydrous magnesium sulfate), filtered and concentrated to obtain a residue, which was purified by silica gel column chromatography [developing eluent: hexane: ethyl acetate = 20:1 to 10:1] to obtain 6-bromo-3-[(4-fluorophenyl)sulfanyl]pyrazine-2-carboxylic acid methyl ester (1.62 g) in the form of colorless powder.
   MS (ESI/APCI Dual): 343 (M+1)
   1H NMR (300 MHz, CDC13) δ ppm 4.06 (s, 3 H) 6.99 - 7.25 (m, 2 H) 7.41 - 7.59 (m, 2 H) 8.43 (s, 1 H)
(2) To a methanol (7 ml) and tetrahydrofuran (7 ml) solution of 6-bromo-3-[(4-fluorophenyl)sulfanyl]pyrazine-2-carboxylic acid methyl ester (700 mg, 2.03 mmol), a 1 M aqueous sodium hydroxide solution (4.06 ml) was added under ice cooling and the mixture was stirred for 2 hours under ice cooling. To the reaction solution, a 1 M aqueous hydrochloric acid solution was added to render the reaction system acidic. The reaction solution was extracted with chloroform and then washed (with water and brine in this order), dried (over anhydrous magnesium sulfate), filtered and concentrated to obtain crude 6-bromo-3-[(4-fluorophenyl)sulfanyl]pyrazine-2-carboxylic acid.
(3) To a dimethylformamide (15 ml) solution of the 6-bromo-3-[(4-fluorophenyl)sulfanyl]pyrazine-2-carboxylic acid, 1-(3-dimethylaminopropyl)-3-ethylcarbodiimide hydrochloride (583 mg, 3.045 mmol), 1-hydroxybenzotriazole (420 mg, 3.045 mmol), 3-amino-1-methylpyrazole hydrochloride (406 mg, 3.045 mmol) and triethylamine (0.425 ml, 3.045 mmol) were sequentially added and the mixture was stirred at room temperature for 15 hours. The reaction solution was charged with water, extracted with ethyl acetate, washed (with water and brine in this order), dried (over anhydrous magnesium sulfate), filtered and concentrated to obtain a residue, which was purified by silica gel column chromatography [developing eluent: hexane: ethyl acetate = 9:1 to 1:1] to obtain 6-bromo-3-[(4-fluorophenyl)sulfanyl]-N-(1-methyl-1H-pyrazol-3-yl)pyrazirie-2-carboxamide (788 mg) in the form of colorless powder.
   MS (ESI/APCI Dual): 408 (M+1)
   1H NMR (300 MHz, CDC13) δ ppm 3.87 (s, 3 H) 6.91 (d, J = 2.3 Hz, 1H) 7.09 - 7.18 (m, 2 H) 7.33 (d, J = 2.3 Hz, 1 H) 7.47 - 7.58 (m, 2 H) 8.43 (s, 1 H) 9.97 - 9.98 (br, 1 H)
(4) A dimethylformamide (6 ml) suspension of 6-bromo-3-[(4-fluorophenyl)sulfanyl]-N-(1-methyl-1H-pyrazol-3-yl)pyrazine-2-carboxamide (300 mg, 0.734 mmol)), 3-mercapto-4-methyl-4H-1,2,4-triazole (169 mg, 1.47 mmol), potassium carbonate (152 mg, 1.10 mmol) was stirred at an external temperature of 50°C for 2 hours. The reaction solution was poured into water and extracted with ethyl acetate, washed (with water and brine in this order), dried (over anhydrous magnesium sulfate), filtered and concentrated to obtain a residue, which was purified by silica gel column chromatography [developing eluent: chloroform: methanol = 100:1 to 10:1] to obtain 3-[(4-fluorophenyl)sulfanyl]-N-(1-methyl-1H-pyrazol-3-yl)-6-[(4-methyl-4H-1,2,4-triazol-3-yl)sulfanyl]pyrazine-2-carboxamide (193 mg) in the form of light yellow powder.

### (Example 8)

According to the production process of Example 7, 3,6-dibromopyrazine-2-carboxylic acid methyl ester was used or, in place, 3-bromo-6-iodopyrazine-2-carboxylic acid methyl ester was used; 3-amino-1-methylpyrazole was used or, in place, aminopyrazine was used; in place of 3-mercapto-4-methyl-4H-1,2,4-triazole, 3-mercapto-4H-1,2,4-triazole, 3-mercapto-5-methyl-4H-1,2,4-triazole, 5-ethyl-3-mercapto-4H-1,2,4-triazole, 5-hydoxy-4H-1,2,4-triazole-3-thiol, 5-amino-3-mercapto-4H-1,2,4-triazole, 2-mercapto-1,3,4-thiadiazole, 2-mercaptopyridine or (S)-2-methoxy-1-methylethanol was used; 4-fluorothiophenol was used or, in place, 4-methoxythiophenol, 4-(methylsulfanyl)thiophenol, 4-(methylsulfonyl)thiophenol, 4-mercaptopyridine, 4-fluorophenol or 4-fluoroaniline was used to obtain the following compounds of the present invention:
3-[(4-fluorophenyl)sulfanyl]-N-(1-methyl-1H-pyrazol-3-yl)-6-[(5-methyl-4H-1,2,4-triazol-3-yl)sulfanyl]pyrazine-2-carboxamide (Compound No. 38),
3-[(4-fluorophenyl)sulfanyl]-6-[(5-hydoxy-4H-1,2,4-triazol-3-yl)sulfanyl]-N-(1-methyl-1H-pyrazol-3-yl)pyrazine-2-carboxamide (Compound No. 39),
6-[(5-amino-4H-1,2,4-triazol-3-yl)sulfanyl]-3-[(4-fluorophenyl)sulfanyl]-N-(1-methyl-1H-pyrazol-3-yl)pyrazine-2-carboxamide (Compound No. 40),
3-[(4-fluorophenyl)sulfanyl]-N-(1-methyl-1H-pyrazol-3-yl)-6-(1,3,4-thiadiazol-2-ylsulfanyl)pyrazine-2-carboxamide (Compound No. 41),
3-[(4-fluorophenyl)sulfanyl]-N-(1-methyl-1H-pyrazol-3-yl)-6-(pyridin-2-ylsulfanyl)pyrazine-2-carboxamide (Compound No. 42),
3-[(4-methoxyphenyl)sulfanyl]-N-(1-methyl-1H-pyrazol-3-yl-)-6-[(5-methyl-4H-1,2,4-triazol-3-yl)sulfanyl]pyrazine-2-carboxamide (Compound No. 43),
6-[(5-ethyl-4H-1,2,4-triazol-3-yl)suilfanyl]-3-[(4-methoxyphenyl)sulfanyl]-N-(1-methyl-1H-pyrazol-3-yl)pyrazine-2-carboxamide (Compound No. 44),
6-[(5-amino-4H-1,2,4-triazol-3-yl)sulfanyl]-3-[(4-methoxyphenyl)sulfanyl]-N-(1-methyl-1H-pyrazol-3-yl)pyrazine-2-carboxamide (Compound No. 45),
3-[(4-methoxyphenyl)sulfanyl]-N-pyrazin-2-yl-6-(4H-1,2,4-triazol-3-ylsulfanyl)pyrazine-2-carboxamide (Compound No. 46),
3-[(4-methoxyphenyl)sulfanyl]-6-[(5-methyl-4H-1,2,4-trlazol-3-yl)sulfanyl]-N-pyrazin-2-ylpyrazine-2-carboxamide (Compound No. 47),
6-[(5-amino-4H-1,2,4-triazol-3-yl)sulfanyl]-3-[(4-methoxyphenyl)sulfanyl]-N-pyrazin-2-ylpyrazine-2-carboxamide (Compound No. 48),
3-([4-(methylsulfanyl)phenyl]sulfanyl}-N-pyrazin-2-yl-6-(4H-1,2,4-triazol-3-ylsulfanyl)pyrazine-2-carboxamide, (Compound No. 49),
3-{[4-(methylsulfanyl)phenyl]sulfanyl}-6-[(5-methyl-4H-1,2,4-triazol-3-yl)sulfanyl]-N-pyrazin-2-ylpyrazine-2-carboxamide (Compound No. 50),
6-[(5-amino-4H-1,2,4-triazol-3-yl)sulfanyl]-3-{(4-(methylsulfanyl)phenyl)sulfanyl}-N-pyrazin-2-ylpyrazine-2-carboxamide (Compound No. 51),
N-(1-methyl-1H-pyrazol-3-yl)-3-{[4-(methylsulfonyl)phenyl]sulfanyl}-6-[(5-methyl-4H-1,2,4-triazol-3-yl)sulfanyl]pyrazine-2-carboxamide (Compound No. 52),
6-[(5-amino-4H-1,2,4-triazol-3-yl)sulfanyl]-N-(1-methyl-1H-pyrazol-3-yl)-3-([4-(methylsulfonyl)phenyl]sulfanyl}pyrazine-2-carboxamide (Compound No. 53),
6-[(1S)-2-methoxy-1-methylethoxy]-N-(1-methyl-1H-pyrazol-3-yl)-3-{[4-(methylsulfonyl)phenyl]sulfanyl}pyrazine-2-carboxamide (Compound No. 54),
N-(1-methyl-1H-pyrazol-3-yl)-6-[(5-methyl-4H-1,2,4-triazol-3-yl)sulfanyl]-3-(pyridin-4-ylsulfanyl)pyrazine-2-carboxamide (Compound No. 55),
6-[(5-amino-4H-1,2,4-triazol-3-yl)sulfanyl]-N-(1-methyl-1H-pyrazol-3-yl)-3-(pyridin-4-ylsulfanyl)pyrazine-2-carboxamide (Compound No. 56),
3-(4-fluorophenoxy)-N-(1-methyl-1H-pyrazol-3-yl)-6-(4H-1,2,4-triazol-3-ylsulfanyl)pyrazine-2-carboxamide (Compound No. 57),
3-[(4-fluorophenyl)amino]-N-(1-methyl-1H-pyrazol-3-yl)-6-(4H-1,2,4-triazol-3-ylsulfanyl)pyrazine-2-carboxamide (Compound No. 58)

### (Example 9)

### Production of 3-[(4-fluorophenyl)sulfanyl]-N-(5-methyl-1,3-thiazol-2-yl)-6-[(6-oxopyridazin-1(6H)-yl)methyl]pyrazine-2-carboxamide (Compound No. 59)

(1) To a tetrahydrofuran (200 ml) solution of 3-amino-6-chloromethyl-2-pyrazinecarbonitrile-4-oxide (5. g, 27.1 mmol)), phosphorus trichloride (4.73 ml, 54.1 mmol)) was added under ice cooling and the mixture was stirred at room temperature for 40 minutes. The reaction solution was distilled away up to about 20 ml and water was added dropwise under ice cooling. The solid substance precipitated was filtered and washed with water to obtain 3-amino-6-chloromethyl-2-pyrazinecarbonitrile (3.65 g) in the form of a light brown solid substance.
   MS (ESI): 169 (M+1)
   1H NMR (300 MHz, CDC13) δ ppm 4.59 (s, 2 H) 5.23 - 5.48 (br, 2 H) 8.37 (s, 1 H)
(2) Under nitrogen gas flow, to a dimethylformamide (20 ml) solution of 3(2H)-pyridazinone (684 mg, 7.12 mmol), sodium hydride (45% of liquid paraffin was added) (311 mg, 7.12 mmol)) 3-amino-6-chloromethyl-2-pyrazinecarbonitrile (1 g, 5.93 nimol)were sequentially added under ice cooling and the mixture was stirred at room temperature for 10 minutes. The reaction solution was charged with water and chloroform, filtered by Celite, extracted five times with chloroform, dried (over anhydrous magnesium sulfate), filtered and concentrated to obtain a solid substance, which was washed with chloroform to obtain 3-amino-6-[(6-oxopyridazin-1(6H)-yl)methyl]pyrazine-2-carbonitrile (627 mg) in the form of a yellow solid substance.
   MS (ESI): 229 (M+1)
   1H NMR (300 MHz, CDC13) δ ppm 5.19 (s, 2 H) 6.93 - 6.97 (m, 1 H) 7.32 (s, 2 H) 7.39 - 7.44 (m, 1 H) 7.90 - 7.92 (m, 1 H) 8.30 (s, 1H)
(3) To an acetic acid (6.6 ml) solution of 3-amino-6-[(6-oxopyridazin-1(6H)-yl)methyl]pyrazine-2-carbonitrile (600 mg, 2.63 mmol, a 48% aqueous hydrobromic acid solution (4.2 m1), an acetic acid (0.6 ml) solution of bromine (0.3 ml) and an aqueous (1.2 ml) solution of sodium nitrite (545.1 mg, 7.9 mmol) were sequentially added under ice cooling and the mixture was stirred at room temperature for 1.5 hours. The reaction solution was charged with a 20% aqueous potassium hydrogensulfite solution (10 ml) and a 20% aqueous sodium hydroxide solution (20 ml), extracted with chloroform and then dried (over anhydrous magnesium sulfate), filtered and concentrated to obtain a residue, which was purified by silica gel column chromatography [developing eluent: hexane: ethyl acetate = 2:1 to 1:1] to obtain 3-promo-6-[(6-oxopyridazin-1(6H)-yl)methyl]pyrazine-2-carbonitrile (314.4 mg) in the form of a white amorphous substance.
   MS (ESI): 292 (M+1)
   1H NMR (300 MHz, CDCl3) δ ppm 5.48 (s, 2 H) 6.99 (dd, J = 1.7, 9.4 Hz, 1 H) 7.28 (dd, J = 3.9, 9.4 Hz, 1 H) 7.85 (dd, J = 1.7, 3.8 Hz, 1H ) 8.62 (s, 1 H)
(4) To a dimethylformamide (7 ml) solution of 3-bromo-6-[(6-oxopyridazin-1-(6H)-yl)methyl]pyrazine-2-carbonitrile (314.4 mg, 1.08 mmol), potassium carbonate (223.9 mg, 1.62 mmol) and 4-fluorothiophenol (0.173 ml, 1.62 mmol) were sequentially added at room temperature and the mixture was stirred at room temperature for 30 minutes. The reaction solution was charged with water and a saturated aqueous sodium chloride solution, extracted with chloroform and then dried (over anhydrous magnesium sulfate), filtered and concentrated to obtain a residue, which was purified by silica gel column chromatography [developing eluent: chloroform: ethyl acetate = 10:0 to 4:1] to obtain 3-[(4-fluorophenyl)sulfanyl]-6-[(6-oxopyridazin-1(6H)-yl)methyl]pyrazine-2-carbonitrile (300 mg) in the form of a yellow amorphous substance.
   MS (ESI): 340 (M+1), 338 (M-1)
   1H NMR (300 MHz, CDCl3) δ ppm 5.42 (s, 2 H) 6.95 (dd, J = 1.7, 9.4 Hz, 1H) 7.12 - 7.25 (m, 3 H) 7.50 - 7.57 (m, 2 H) 7.81 (dd, J = 1.7, 3.9 Hz, 1 H) 8.45 (s, 1H)
(5) To an ethanol (1.6 ml) solution of 3-[(4-fluorophenyl)sulfanyl]-6-[(6-oxopyridazin-1(6H)-yl)methyl]pyrazine-2-carbonitrile (326 mg, 0.96 mmol), an 1 M aqueous sodium hydroxide solution (4.8 ml) was added at room temperature and the mixture was stirred at an external temperature of 90°C for 4 hours. The reaction solution was charged with a 1 M aqueous hydrochloric acid solution (4.8 ml), extracted with chloroform and then dried (over anhydrous magnesium sulfate), filtered and concentrated to obtain crude 3-[((4-fluorophenyl)sulfanyl]-6-[(6-oxopyridazin-1(6H)-yl)methy]lpyrazine-2-carboxylic acid (237 mg).
(6) To a dimethylformamide (1 ml) solution, the 3-((4-fluorophenyl)snlfanyl]-6-[(6-oxopyridazin-1(6H)-yl)methyl]pyrazine-2-carboxylic acid (37 mg), 1-(3-dimethylaminopropyl)-3-ethylcarbodiimide hydrochloride (23.0 mg, 0.12 mmol), 1-hydroxybenzotriazole (16.2 mg, 0.12 mmol) and 2-amino-5-methylthiazole (13.7 mg, 0.12 mmol) were sequentially added at room temperature and the mixture was stirred at room temperature for one hour. The reaction solution was charged with water, extracted with chloroform and then dried (over anhydrous magnesium sulfate), filtered and concentrated to obtain a residue, which was purified by silica gel column chromatography [developing eluent: chloroform: methanol = 50:1]. The resultant solid substance was dissolved in chloroform and methanol was added thereto to recrystallize. The resultant solid substance was washed with methanol to obtain 3-[(4-fluorophenyl)sulfanyl]-N-(5-methyl-1,3-thiazol-2-yl)-6-[(6-caxopyridazin-1(6H)-yl)methyl]pyrazine-2-carboxamide (9.0 mg) in the form of a white solid substance.

### (Example 10)

According to the production process of Example 9, 3(2H)-pyridazinone or, in place, 1,2,4-triazole was used; in place of 2-amino-5-methylthiazole, 3-amino-1-methylpyrazole was used to obtain the following compounds of the present invention:
3-[(4-fluorophenyl)sulfanyl]-N-(1-methyl-1H-pyrazol-3-yl)-6-[(6-oxopyridazin-1(6H)-yl)methyl]pyrazine-2-carboxamide (Compound No. 60),
3-[(4-fluorophenyl)sulfanyl]-N-(1-methyl-1H-pyrazol-3-'yl)-6-(1H-1,2,4-triazol-1-ylmethyl)pyrazine-2-carboxamide (Compound No. 61).

### (Example 11)

### Production of 3-amino-N-(5-methyl-1,3-thiazol-2-yl)-6-[(6-oxopyridazin-1(6H)" yl)methyl]pyrazine-2-carboxamide (Compound No. 62)

(1) To an ethanol (4.3 ml) solution of 3-amino-6-[(6-oxopyridazin-1(6H)-yl)methyl]pyrazine-2-carbonitrile (431 mg, 1.89 mmol)), a 1 M aqueous sodium hydroxide solution (9.5 ml) was added at room temperature and the mixture was stirred for 3 hours at an external temperature of 90°C. The reaction solution was charged with a 1 M aqueous hydrochloric acid solution (10 ml) and extracted with ethyl acetate and then washed (with brine), dried (over anhydrous sodium sulfate), filtered and concentrated to obtain crude 3-amino-6-[(6-oxopyridazin-1(6H)-yl) methyl]pyrazine-2-carboxylic acid (381 mg) .
(2) To a dimethylformamide (1 ml) solution of the 3-amino-6-[(6-oxopyridazin-1(6H)-yl)methyl]pyrazine-2-carboxylic acid (50 mg, 0.20 mmol), 1-(3-dimethylaminopropyl)-3-ethylcarbodiimide hydrochloride (46.5 mg, 0.24 mmol), 1-hydroxybenzotriazole (32.8 mg, 0.24 mmol) and 2-amino-5-methylthiazole (27.7 mg, 0.24 mmol) were sequentially added at room temperature and the mixture was stirred at room temperature for one hour. The reaction solution was charged with water and extracted with ethyl acetate, washed (with brine), dried (over anhydrous magnesium sulfate), filtered and concentrated to obtain a residue, which was washed with chloroform and methanol to obtain 3-amino-N-(5-methyl-1,3-thiazol-2-yl)-6-[(6-oxopyridazin-l(6H)-yl)methyl]pyrazine-2-carboxamide (27.8 mg) in the form of a yellow solid substance.

### (Example 12)

### Production of 3-amino-N-(1-methyl-1H-pyrazol-3-yl)-6-(4H-1,2,4-triazol-3-ylsulfanyl)pyrazine-2-carboxamide (Compound No. 63)

(1) To a methanol (10 ml) and tetrahydrofuran (10 ml) solution of 3-amino-6-iodopyrazine-2-carboxylic acid methyl ester (see Patent Document: WO2006/015124)(2.0 g, 7.16 mmol), a 1 M aqueous sodium hydroxide solution (14.3 ml) was added under ice cooling and the mixture was stirred for 2 hours under ice cooling. The reaction solution was charged with a 1 M aqueous hydrochloric acid solution to render the reaction system acidic and concentrated to obtain crude 3-amino-6-iodopyrazine-2-carboxylic acid.
(2) To a dimethylformamide (20 ml) solution of the 3-amino-6-iodopyrazine-2-carboxylic acid, 1-(3-dimethylaminopropyl)-3-ethylcarbodiimide hydrochloride (2.05 g, 10.74 mmol), 1-hydroxybenzotriazole (1.48 g, 10.74 mmol), 3-amino-1-methylpyrazole hydrochloride (1.43 g, 10.74 mmol) and triethylamine (1.99 ml, 14.32 mmol) were sequentially added at room temperature and the mixture was stirred at room temperature for 15 hours. The reaction solution was charged with water, extracted with ethyl acetate, washed (with water and brine in this order), dried (over anhydrous magnesium sulfate), filtered and concentrated to obtain a residue, which was purified by silica gel column chromatography [developing eluent: chloroform: ethyl acetate = 9:1 to 2:1] to obtain 3-amino-6-iodo-N-(1-methyl-1H-pyrazol-3-yl)pyrazine-2-carboxamide (1.05 g) in the form of light yellow powder.
   MS (ESI/APCI Dual): 344 (M+1)
   1H NMR (300 MHz, DMSO-D6) δ ppm 3.78 (s, 3 H) 6.55 (d, J = 2.3 Hz, 1 H) 7. 47 - 7.85 (m, 3 H) 8.48 (s, 1 H) 9.92 - 10.24 (br, 1 H)
(3) To a 1,4-dioxane (3 ml) solution of 3-amino-6-iodo-N-(1-methyl-1H-pyrazol-3-yl)pyrazine-2-carboxamide (200 mg, 0.581 mmol), 3-mercapto-4H-1,2,4-triazole (117 mg, 1.162 mmol), tris(dibenzylideneacetone)palladium (12.8 mg, 0.014 mmol), 4,5-bis(diphenylphosphino)-9,9-dimethylxanthene (16 mg, 0.029 mmol) and cesium carbonate (378 mg, 1.162 mmol) were sequentially added and the mixture was stirred at an external temperature of 100°C for 5 hours. The reaction solution was charged with chloroform and filtered by Celite. The filtrate was concentrated to obtain a residue, which was purified by silica gel column chromatography [developing eluent: chloroform: methanol = 50:1 to 10:1] to obtain 3-amino-N-(1-methyl-1H-pyrazol-3-yl)-6-(4H-1,2,4-triazol-3-ylsulfanyl)pyrazine-2-carboxamide (35 mg) in the form of light yellow powder.

### (Example 13).

### Production of 3-[(1-methylpiperidin-4-yl)oxy]-N-(1-methyl-1H-pyrazol-3-yl)-6-(4H-1,2,4-triazol-3-ylsulfanyl)pyrazine-2-carboxamide (Compound No. 64)

(1) To a tetrahydrofuran (20 ml) solution of 3-hydoxy-6-iodopyrazine-2-carboxylic acid methyl ester (1.0 g, 3.57 mmol), 4-hydoxy-1-methylpiperidine (822.3 mg, 7.14 mmol) and triphenylphosphine (1.87 g, 7.14 mmol), a 1.9 M diisopropyl azodicarboxylate toluene solution (3.76 ml, 7.14 mmol) was added at room temperature and the mixture was stirred at room temperature for 4 hours. The reaction solution was concentrated and dissolved in ethanol (20 ml). To the reaction solution, a 1 M aqueous sodium hydroxide solution (7.2 ml) was added at room temperature and the mixture was stirred at room temperature for one hour. The reaction solution was charged with water, extracted with ethyl acetate. The water phase was charged with a 1 M aqueous hydrochloric acid solution (7.2 ml) and concentrated. To the resultant residue, methanol-tetrahydrofuran (1:1) was added, insoluble matter was filtered off, and the filtrate was concentrated to obtain crude 6-iodo-3-[(1-methylpiperidin-4-yl)oxy]pyrazine-2-carboxylic acid.
(2) To a dimethylformamide (24 ml) solution of the 6-iodo-3-[(1-methylpiperidin-4-yl)oxy]pyrazine-2-carboxylic acid, 1-(3-dimethylaminopropyl)-3-ethylcarbodiimide hydrochloride (1.37 g, 7.14 mmol), 1-hydroxybenzotriazole (965 mg, 7.14 mmol), 3-amino-1-methylpyrazole hydrochloride (954 mg, 7.14 mmol) and triethylamine (0.995 ml, 7.14 mmol) were sequentially added at room temperature and the mixture was stirred at room temperature for one hour. The reaction solution was distilled away and the residue was washed with ethyl acetate. The resultant solid substance was recrystallized with ethanol to obtain 6-iodo-3-[(1-methylpiperidin-4-yl)oxy]-N-(1-methyl-1H-pyrazol-3-yl)pyrazine-2-carboxamide hydrochloride (395.3 mg) in the form of a white solid substance.
   MS (ESI): 443 (M+1)
   1H NMR (300 MHz, CDCl3) δ ppm 2.15 - 2.21 (m, 2 H) 2.65 - 2.73 (m, 2 H) 2.85 (d, J = 3.7 Hz, 3 H) 3.34 - 3.49 (m, 4 H) 3.87 (s, 3 H) 5.60 (s, 1 H) 6.75 (d, J = 2.5 Hz, 1 H) 7.33 (d, J = 2.0 Hz, 1 H) 8.41 (s, 1 H) 9.81 (s, 1 H) 12.35 - 12.53 (br, 1 H)
(3) To a dimethylformamide (3 ml) solution of 6-iodo-3-[(1-methylpiperidin-4-yl)oxy]-N-(1-methyl-1H-pyrazol-3-yl)pyrazine-2-carboxamide hydrochloride (150 mg, 0.31 mmol), potassium carbonate (70.4 mg, 0.51 mmol) and 3-mercapto-4H-1,2,4-triazole (51.6 mg, 0.51 mmol) were sequentially added at room temperature and the mixture was stirred at an external temperature of 150°C for 7 hours. The reaction solution was filtered to remove potassium carbonate and the filtrate was concentrated to obtain a residue, which was purified by NH-silica gel column chromatography [developing eluent: chloroform: methanol = 5:1] to obtain 3-[(1-methylpiperidin-4-yl)oxy]-N-(1-methyl-1H-pyrazol-3-yl)-6-(4H-1,2,4-triazol-3-ylsufanyl)pyrazine-2-carboxamide (63.8 mg) in the form of a white solid substance.

### (Example 14)

### Production of N-(1-methyl-1H-pyrazol-3-yl)-3-[(6-methylpyridin-3-yl)sulfanyl]-6-(4H-1,2,4-triazol-3-ylsulfanyl)pyrazine-2-carboxamide hydrochloride (Compound No. 65)

To an ethanol (10 ml) solution of the N-(1-methyl-1H-pyrazol-3-yl)-3-[(6-methylpyridin-3-yl)sulfanyl]-6-(4H-1,2,4-triazol-3-ylsulfanyl)pyrazine-2-carboxamide (526 mg, 1.24 mmol) obtained in Example 3, a 4 M hydrochloric acid/ethyl acetate solution (465 µl) and water (2 ml) were added at room temperature and the mixture was stirred at 100°C. Filtration was performed while heating and the filtrate was cooled. The crystals generated were filtered, washed with cold ethanol and dried under reduced pressure to obtain N-(1-methyl-1H-pyrazol-3-yl)-3-[(6-methylpyridin-3-yl)sulfanyl]-6-(4H-1,2,4-triazol-3-ylsulfanyl)pyrazine-2-carboxamide hydrochloride (434 mg) in the form of yellow powder.

### (Example 15)

### Production of 3-[(4-fluorophenyl)sulfanyl]-N-(1-methyl-1H-pyrazol-3-yl)-6-[(4H-1,2,4-triazol-3-yl)sulfanyl]pyrazine-2-carboxamide potassium salt (Compound No. 66)

To an ethanol (3 ml) suspension of the 3-[(4-fluorophenyl)sulfanyl]-N-(1-methyl-1H-pyrazol-3-yl)-6-[(4H-1,2,4-triazol-3-yl)sulfanyl]pyrazine-2-carboxamide (128 mg, 0.3 mmol) obtained in Example 2, a 1 M methanol solution (0.3 ml) of potassium hydroxide was gradually added at room temperature and the mixture was stirred for 2 hours. To the reaction solution, diisopropyl ether (6 ml) was added and the crystals precipitated were filtered and washed with ethanol-diisopropyl ether (1:2) to obtain a 3-[(4-fluorophenyl)sulfanyl]-N-(1-methyl-1H-pyrazol-3-yl)-6-[(4H-1,2,4-triazol-3-yl)sulfanyl]pyrazine-2-carboxamide potassium salt (109 mg) in the form of a yellow solid substance.

### (Example 16)

According to the production process of Example 15, in place of 3-[(4-fluorophenyl)sulfanyl]-N-(1-methyl-1H-pyrazol-3-yl)-6-[(4H-1,2,4-triazol-3-yl)sulfanyl]pyrazine-2-carboxamide, 3-[(4-fluorophenyl)sulfanyl]-N-(1-methyl-1H-pyrazol-3-yl)-6-[(5-methyl-4H-1,2,4-triazol-3-yl)sulfanyl]pyrazine-2-carboxamide or 3-[(4-methoxyphenyl)sulfanyl]-N-(1-methyl-1H-pyrazol-3-yl)-6-(4H-1,2,4-triazol-3-ylsulfanyl)pyrazine-2-carboxamide was used to obtain the following compounds of the present invention:
3-[(4-fluorophenyl)sulfanyl]-N-(1-methyl-1H-pyrazol-3-yl)-6-[(5-methyl-4H-1,2,4-triazol-3-yl)sulfanyl]pyrazine-2-carboxamide potassium salt (Compound No. 67)
3-[(4-methoxyphenyl)sulfanyl]-N-(1-methyl-1H-pyrazol-3-yl)-6-(4H-1,2,4-triazol-3-ylsulfanyl)pyrazine-2-carboxamide potassium salt (Compound No. 68).

Compound structures, NMR data and MS data obtained in Examples are shown in Tables 1-1 to 1-6.

**[Table 1-1]**

| | | | | | |
|---|---|---|---|---|---|
| 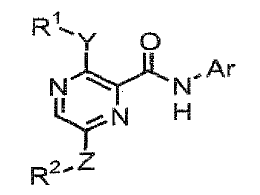 | | | | | |

| Compound No. | R¹-Y- | R²-Z- | -Ar | NMR | MS |
|---|---|---|---|---|---|
| 1 | 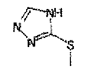 | 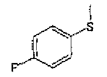 | 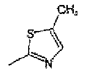 | 1H NMR (300 MHz, DMSO-D6) δ ppm 2.39 (s, 3 H) 7.26 (d, J=1.3 Hz, 1 H) 7.34 (t, J=8.9 Hz, 2 H) 7.71 - 7.76 (m, 2H) 8.19 (d, J=1.0 Hz, 1H) 8.50 - 8.63 (br, 1H) (d, J=1.0 | MS (ESI)_{:} 446 (M+1), 444 (M-1) |
| 2 | 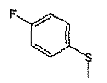 | 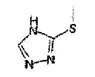 | 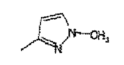 | 1 H NMR (300 MHz, DMSO-D6) δ ppm 3.81 (s, 3 H) 6.61 (d, J=2.3 Hz, 1 H) 7.26 - 7.32 (m, 2 H) 7.55 - 7.59 (m, 2 H) 7.68 (d, J=2.3 Hz, 1 H) 6.37 (a, 1 H) 8.63 - 8.84 (br, 1 H), 10.54 (s, 1 H) 14.56 - 14.72 (br, 1 H) | MS (ESI): 429 (M+1), 427 (M-1) |
| 3 | 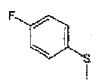 | 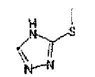 | 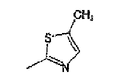 | 1H NMR (300 MHz, DMSO-D6) δ ppm 2.41 (s, 3 H) 7,27-7-32 (m, 3 H) 7,56 - 7.61 (m, 2 H) 8.37 (s. 1 H) 8,72 (s, 1 H) | MS (ESI): 446 (M+1). 444 (M-1) |
| 4 | 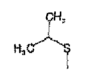 | 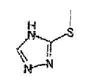 | 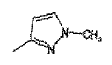 | 1H NMR (300 MHz, CDCl3) δ ppm 1.40 (d, J=6.7 Hz, 6 H) 3,83 (s, 3 H) 3.84 - 4.09 (m, 1 H) 6.87 (d, J=2.3 Hz. 1 H) 7.28 (d, J=2.3 Hz, 1 H) 8.29 - 8.41 (br, 1H) 8.60 (s, 1 H) 9.84 - 10.05 (br. 1 H) | MS (ESI/APCI Dual): 377 (M+1), 375 (M-1) |
| 5 | 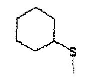 | 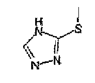 | 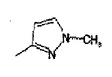 | 1H NMR (300 MHz. CDCl3) δ ppm 1.15 -1.38 (m, 2 H) 1.36 - 1.57 (m, 4 H) 1.69 -1.85 (m, 2 H) 1.98-2.15 (m, 2 H) 3.73-3.98 (m, 1 H) 3.83 (s, 3 H) 6.86 (d, J=2.3 Hz. 1 H) 7.27 (d, J=2.3 Hz, 1 H) 8.19 - 8.47 (br. 1 H 8.57 (s, 1 H) 9.88 - 10.09 (br, 1 H) | MS (ESI/APCI Dual): 417 (M+1), 415 (M-1) |
| 6 | 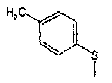 | 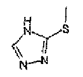 | 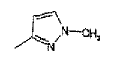 | 1H NMR (300 MHz, DMSO-D6) δ ppm 2.34 (s, 3 H) 3.80 (s, 3 H) 6.60 (d, J=2.1 Hz, 1 H) 7.25 (d, J=7.8Hz, 2 H) 7,39 (d. J=7.8Hz, 2 H) 7.67 (d, J=2.7 Hz, 1H) 8.35 (s. 1 H) 8.60 - 8.80 (br, 1 H), 10.54 (s, 1 H) 14.55 - 14.65 (br, 1 H) | MS (ESI): 425 (M+1), 423 (M-1) |
| 7 | 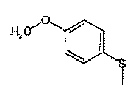 | 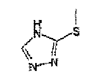 | 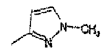 | 1H NMR (300 MHz, DMSO-D6) δ ppm 3.80 (s, 3 H) 3.81 (s, 3 H) 8.61 (s, 1 H) 7.00 (d. J=8.4Hz, 2 H) 7.43 (d, J=8.1Hz, 2 H) 7.68 (s, 1H) 8.36 (s, 1 H) 8.60 - 8.85 (br, 1 H). 10.52 (s, 1 H) 14.50 - 14.70 (br, 1 H) | MS (ESI): 441 (M+1), 438 (M-1) |
| 8 | 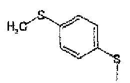 | 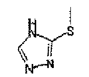 | 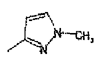 | 1H NMR (300 MHz, DMSO-D6) δ ppm 3.10 - 3.54 (br, 3 H) 3.80 (s, 3 H) 6.61 (d, J=2.3 Hz, 1 H) 7.23 - 7.37 (m, 2 H) 7.40 - 7.46 (m, 2 H) 7,67 (d, J=2.3 Hz, 1 H) 8.36 (s, 1 H) 8.83 - 8.78 (br, 1 H) 10.45 - 10.58 (br, 1 H) | MS (ESI/APCI Dual): 457 (M+1), 455 (M-1) |
| 9 | 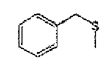 | 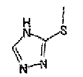 | 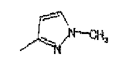 | 1H NMR (300 MHz, DMSO-D6) δ ppm 3,78 (s, 3 H) 4.35 (s, 2 H) 6.54 (d, J=2.2 Hz, 1 H) 7.18 - 7.34 (m, 3 H) 7.37 - 7.46 (m, 2 H) 7.64 (d. J=2.2 Hz, 1 H) 8.64 (s, 1 H) 8.70 -8.80 (br, 1 H) 10.32 - 10.49 (br, 1 H) | MS (ESI/APCI Dual); 425 (M+1), 423 (M-1) |
| 10 | 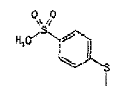 | 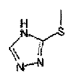 | 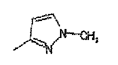 | 1H NMR (300 MHz, DMSO-D6) δ ppm 3.28 (s, 3 H) 3.61 (s, 3 H) 6.60 (d, J=2.2 Hz, 1 H) 7.68 (d, J=2.2 Hz, 1 H) 7.78 (d, J=8.7 Hz, 2 H) 7.95 (d, J=8.7 Hz, 2 H) 8.38 (s, 1 H) 8.68 - 8.79 (br, 1 H) 10.55 - 10.64 (br, 1 H) | MS (ESI/APCI Dual): 489 (M+1), 487 (M-1) |
| 11 | 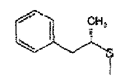 | 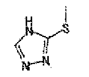 | 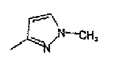 | 1H NMR (300 MHz, CDCl3) δ ppm 1.32 (d, J=6.8 Hz, 3 H) 2.70 (dd, J=13.5, 9.3 Hz, 1 H) 3.19 (dd, J=13.5, 5.2 Hz, 1 H) 3.83 (s, 3 H) 4,10 - 4.23 (m, 1 H) 6.87 (d. J=2.3 Hz, 1 H) 722 - 7.31 (m, 6 H) 8.32 - 6.39 (br, 1 H) 8.62 (s, 1 H) 8.93 - 9.99 (br, 1 H) | MS (ESI/APCI Dual): 453 (M+1), 451 (M-1) |

**[Table 1-2]**

| | | | | | |
|---|---|---|---|---|---|
| 12 | 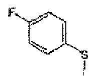 | 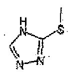 | 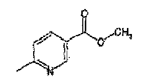 | 1H NMR (300 MHz, DMSO-D6) δ ppm 3.88 (d, J=1.2 Hz. 3 H) 7.27 - 7.32 (m, 2 H) 7.56 - 7.60 (m, 2 H) 8.32 - 8.47 (m, 2 H) 8.47 (d. J=1.6 Hz, 1 H) 8.72 - 8.80 (br. 1 H) 8,93 (z, 1 H) 10.51 - 10.64 (br, 1 H) | MS (ESI): 484 (M+1), 482 (M-1) |
| 13 | 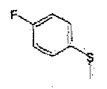 | 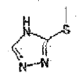 | 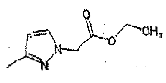 | 1H NMR (300 MHz, DMSO-D6) δ ppm 1.22 (t, J=7.1 Hz, 3 H) 4.17 (q, J=7.1 Hz, 2 H) 5.02 (s, 2 H) 0.69 (d, J=2.2 Hz, 1 H 7.19 - Dual): 501 7.40 (m*,* 2 H) 7.51 - 7,64 (m, 2 H) 7.75 (d, J=2.2 Hz, 1 H) 8,35 (s, 1 H) 8.57 - 8.81 (br, 1 H) 10.60 - 10,79 (br, 1 H) | MS (ESI/APCI (M+1), 499 (M-1) |
| 14 | 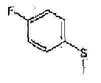 | 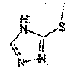 | 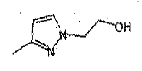 | 1H NMR (300 MHz, DMSO-D6) δ ppm 3.67 - 3.83 (m, 2 H) 4.09 (t, J=5.6 Hz, 2 H) 4.91 (t, J=5.2 Hz, 1 H) 6.61 (d, J=2.3 Hz, 1 H) Dual): 722 - 7.37 (m, 2 H) 7.51 - 7.63 (m, 2 H) 7.69 (d, J=2.3 Hz, 1 H) 8.36 (s, 1 H) 8,64 -8.80 (br, 1 H) 10.53 - 10.63 (br, 1 H) | MS (ESI/APCI 481 (M+23), 457 (M-1) |
| 15 | 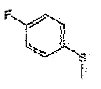 | 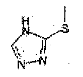 | 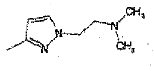 | 1H NMR (300 MHz, DMSO-D6) δ ppm 220 (s, 6 H) 2.61 - 2.74 (m, 2 H) 4.02 - 4.26 (m, 2 H) 6.61 (d, J=2.3 Hz, 1 H) 7.19 - 7.37 (m, 2 H) 7.52 - 7.62 (m, 2 H) 7.73 (d, J=2.3 Hz, 1 H) 8.36 (s, 1 H) 8.71 (s, 1 H) 10.40 - 10.62 (br, 1 H). | MS (ESI/APCI Dual): 486 (M+1), 484 (M-1) |
| 16 | 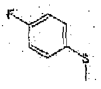 | 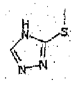 | 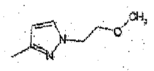 | 1H NMR (300 MHz. DMSO-D6) δ ppm 3.25 (s, 3 H) 3.69 (t, J=5:3 Hz, 2 H) 4.21 (t, J=5.3 Hz, 2 H) 6.62 (d, J=2.3 Hz, 1 H) 7.20 7.36 (m, 2 H) 7.50 7,64 (m, 2 H) 7.70 (d J=2.3 Hz, 1 H) 8.36 (s, 1 H) 8.59 - 8.79 (br, 1 H) Hz, 1 H) 8,36 (s, 1 H) 8.59 - 8.79 (br, 1 H) 10.46 - 10.72 (br, 1 H) | MS (ESI/APOI (Dual): 473 (M+1), 471 (*M*-1) |
| 17 | 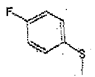 | 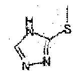 | 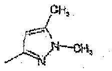 | 1H NMR (300 MHz, DMSO-D6) δ ppm 2.27 (s, 3 H) 3.68 (s, 3 H) 6.46 (s, 1 H) 721 - 7.35 (m, 2 H) 7.50 - 7.66 (m, 2 H) 8.36 (s, 1 H) 8.63 - 8.80 (br, 1 H) 10.33 - 10,45 (br, 1 H) | MS (ESI/APCI Dual): 443 (M+1), 441 (M-1) |
| 16 | 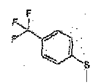 | 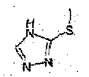 | 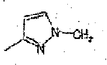 | 1H NMR (300 MHz, DMSO-D6) δ ppm 3.81 (s, 3 H) 6.61 (d, J=2.4 Hz, 1 H) 7.68 (d, J=2.4Hz, 1 H) 7.74 (d; J=8.4Hz, 2 H) 7.79 (d, J=8,7 Hz. 2 H) 8.38 (s, 1 H) 8.68 - -.8.90 (br, 1 H) 10.60 (s, 1 H) 14.55 - 14.70 (br, 1 H) | MS (ESI): 479 (M+1), 477 (M-1) |
| 19 | 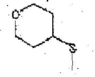 | 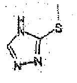 | 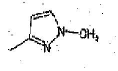 | 1H NMR (300 MHz, DMSO-D6) δ ppm 1.50 - 1.75 (m, 2 H) 1.85 - 2.07 (m, 2 H) 3.37 - 3.53 (m, 2 H) 3.79 (s, 3 H) 3.20 - 3.90 (m, H) 3.01 - 4.06 (m, 1 H) 6.56 (d, J=2.2 Hz, 1 H) 7.65 (d, J=2.2 Hz, 1 H) 8.58 (s, 1 H) 8.69 - 8.78 (br, 1 H) 10.43 - 10.54 (br, 1 H) | MS (ESI/APCI 2 Dual): 419 (M+1), 417 (M-1) |
| 20 | 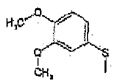 | 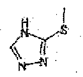 | 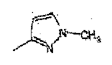 | 1H NMR (300 MHz, DMSO-D6) δ ppm 3.72 (s, 3 H) 3.79 (s, 3 H) 3.80 (s, 3 H) 6.61 (d, J=2,3 Hz, 1 H) 6.94 - 7.19 (m, 3 H) 7.67 (d, Dual): J=2,3 Hz, 1 H) 6.38 (s, 1 H) 8.62 - 8.79 (br, 1 H) 10.41 - 10.60 (br, 1 H) | MS (ESI/APCI 471 (M+1), 469 (M-1) |
| 21 | 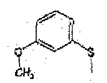 | 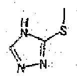 | 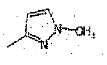 | 1H NMR (300 MHz, DMSO-D6) δ ppm 3.75 (s, 3 H) 3.80 (s, 3 H) 6.60 (d, J=2.2 Hz, 1 H) 6.95 - 7.05 (m, 1 H) 7.06 - 714 (m, 2 H) 7.29 - 7.44 (m, 1 H) 7.67 (d, J=2.2 Hz, 1 H) 8.39 (s, 1 H) 8.64 - 8.75 (br, 1 H) 10.49 - 10.59 (br, 1 H) | MS (ESI/APCI Dual): 441 (M+1), 439 (M-1) |
| 22 | 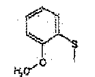 | 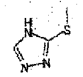 | 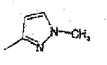 | 1H NMR (300 MHz, DMSO-D6) δ ppm 3.69 (s, 3 H) 3.81 (s, 3 H) 6.61 (d, J=2.2 Hz, 1 H) 6.92 - 7.05 (m, 1 H) 7.06 7.17 (m, 1 H) 7.39 - 7.52 (m, 2 H) 7.68 (d, J=2.2 Hz, 1 H) 8,35 (s, 1 H) 8.57 - 8,91 (br, 1 H) 10,38 - 10.68 (br, 1 H) | MS (ESI/APCI Dual): 441 (M+1), 439 (M-1) |
| 23 | 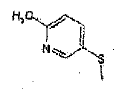 | 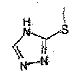 | 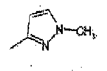 | 1H NMR (300 MHz, DMSO-d6) δ ppm 3.24 - 3.47 (m, 3 H) 3.81 (s, 3 H) 6.61 (d, J=2.2 Hz, 1 H) 7,34 (d, J=7,6 Hz, 1 H) 7,68 (d, J=2.2 Hz, 1 H) 7.82 (dd, J=8.0, 2.2 Hz, 1 H) 8.36 (s, 1 H) 8.50 (d, J=2.2 Hz, 1 H) 8.85 - 8.75 (br, 1 H) 10.44 - 10.58 (br, 1 H) | MS (ESI/APCI Dual); 426(M+1), 424 (M-1) |
| 24 | 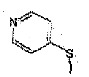 | 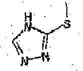 | 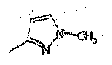 | 1H NMR (300 MHz, DMSO-D6) δ ppm 3.80 (s, 3 H) 6.59 (s, 1 H) 7.51 (d, J=6.1 Hz, 2 H) 7.67 (s, 1 H) 8.43 (s, 1 H) 8.57 (d, J=6.1 Hz, 2 H) 5.68 - 8.80 (br, 1 H) 10.63 (s, 1 H) | MS (ESI): 412 (M+1), 410 (M-1) |

**[Table 1-3]**

| | | | | | |
|---|---|---|---|---|---|
| 25 | 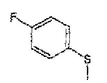 | 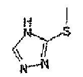 | 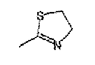 | 1H NMR (300 MHz. DMSO-D6) δ ppm 3.32 (t, J=7.6 Hz, 2 H) 3.69 (t, J=7.6 Hz, 2 H) 7.25 (t, J=8.6 Hz, 2 H) 7.52 (dd, J=5.6. 8.6 Hz. 2 H) 8.21 (s, 1 H) 8.65 (s, 1 H) | MS (ESI): 434 (M+1), 432 (M-1) |
| 26 | 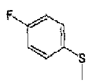 | 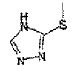 | 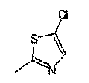 | 1H NMR (300 MHz DMSO-06) δ ppm 7.2B (t, J=8.6 Hz, 2 H) 7.57 (dd, J=5.6, 8.3 Hz, 2 H) 7.86 (s, 1 H) 3.35 (s, 1 H) 8.68 - 8.83 (br. 1 H), 12.60 - 12.80 (br, 1 H). 14.50 - 14.72 (br, 1 H) | MS (ESI): 486 (M+1), 464 (M-1) |
| 27 | 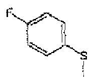 | 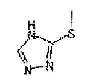 | 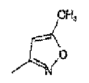 | 1H NMR (300 MHz. DMSO-D6) δ ppm 2.44 (s, 3 H) 6.78 (s, 1 H), 7.31 (dd, J=8.7, 9.0 Hz, 2 H) 7.58 (dd, J=7.8. 8.1Hz, 2 H) 8,37 (s, 1 H) 8.68 - 8.82 (br. 1 H) 11.21 (s, 1 H) 14.55 - 14.70 (br, 1 H) | MS (ESI): 430 (M+1), 428 (M-1) |
| 28 | 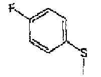 | 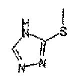 | 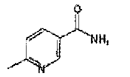 | 1H NMR (300 MHz, DMSO-D6) δ ppm 7.29 (t, J=8.6 Hz, 2 H) 7.53 - 7.60 (m, 3 H) 8.11 (s, 1 H) 8.25 - 8.36 (m, 2 H) 8.45 (s, 1 H) 8.80 s, 1 H) 8.88 (t, J=1.0 Hz, 1 H), 10.44 (s. 1 H), 14.62 (s. 1 H) | MS (ESI); 469 (M+1), 467 (M-1) |
| 29 | 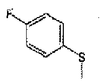 | 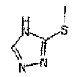 | 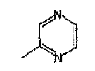 | 1H NMR (300 MHz, DMSO-D6) δ ppm 7.28 - 7.34 (m, 2 H) 7.57 - 7.61 (m, 2 H) 8.46 (s, 1 H) 8.50 - 8.53 (m. 2 H) 8.69 - 8.81 (br, 1 H) 9.44 (d, J=1.2 Hz, 1 H), 10,60 (s. 1 H). 14.57 - 14.71 (br. 1 H) | MS (ESI): 427 (M+1), 425 (M-1) |
| 30 | 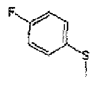 | 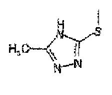 | 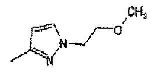 | 1H NMR (300 MHz. CDCl3) δ ppm 2,54 (s. 3 H) 3.35 (s, 3 H) 3.74 (t, J=5.0 Hz. 2 H) 4.22 (t, J=5.0 Hz. 2 H) 6.91(d, J=2.3 Hz, 1 H) 7.08 - 7.14 (m, 2 H) 7.41 (d. J=2.3 Hz, 1 H) 7.47 - 7.53 (m, 2 H) 8.32 (s, 1 H) 10.05 (s, 1 H) 11.56 - 11.95 (br, 1 H) | MS (ESI): 487 (M+1), 485 (M-1) |
| 31 | 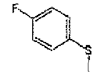 | 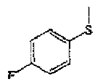 | 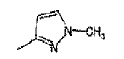 | 1 H NMR (300 MHz, CDCl3) δ ppm 3.85 (s, 3 H) 8,88 (d, J=2.0 Hz, 1 H) 7.05 - 7.28 (m, 4 H) 7.30 (d, J=2.3 Hz, 1 H) 7.48 (dd, J=5 8.3 Hz, 2 H) 7.56 - 7.60 (m, 2 H) 7.94 (d. J=0.7 Hz, 1H), 9.87 (s, 1 H) | MS (ESI): 3, 456 (M+1) |
| 32 | 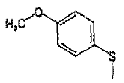 | 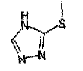 | 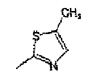 | 1H NMR (300 MHz DMSO-D6) δ ppm 2.42 (s, 3 H) 3.80 (s. 3 H) 7.01 (d. J=8.9 Hz 2 H) 7.28 (d J=1.4 Hz, 1 H) 7.44 (d, J=8.9 Hz, 2 H) 8.36 (s. 1 H) 6.79 - 8.80 (br. 1 H) | MS (ESI): 458 (M+1), 456 (M-1) |
| 33 | 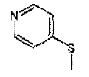 | 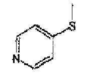 | 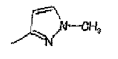 | 1H NMR (300 MMz. DMSO-D6) δ ppm 3.78 (s, 3 H) 6.59 (d. J=2.2 Hz, 1 H) 7.50 (d, J=6.6 Hz, 2 H) 7.58 (d. J=6.1 Hz, 2 H) 7.67 (d. J=2.2 Hz, 1 H) 8.53 (d, J=6.3 Hz, 2 H) 8.61 (d. J=6.1 Hz, 2 H) 8.69 (s. 1 H) 10.51 - 10.57 (br, 1 H) | MS (ESI); 422 (M+1), 420 (M-1) |
| 34 | 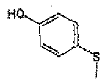 | 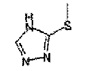 | 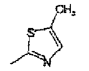 | 1H NMR (300 MHz. DMSO-D6) δ ppm 2.42 (s, 3 H) 8.83 (d, J=8.5 Hz. 2 H) 7,25-7.35 (m. 3 H) 8.37 (s, 1 H) 8.70-8.76 (br, 1 H) | MS (ESI): 444 (M+1), 442 (M-1) |
| 35 | 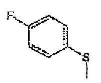 | 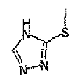 | 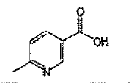 | 1H NMR (300 MHz, DMSO-D6) δ ppm 7.29 (t, J=7.8 Hz, 2 H) 7.57 (t J=7.3 Hz, 2 H) 8.29 - 8.38 (m, 2H) 8.47 (s. 1 H) 8.70 - 8.85 (br, 1 H) 8.90 (s, 1 H) 10.51 (s, 1 H) 14,54 - 14.76 (br. 1 H) | MS (ESI): 470 (M+1), 468 (M-1) |
| 36 | 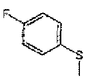 | 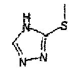 | 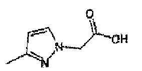 | 1H NMR (300 MHz. DMSO-D6) δ ppm 4.91 (s. 2 H) 6,67 (d. J=2.3 Hz, 1 H) 7.21 - 7.37 (m, 2 H) 7.61 - 7.62 (m, 2 H) 7.73 (d, J=2,3 Hz, 1 H) 8.35 (s, 1 H) 8.89 - 8.83 (br, 1 H) 10,57 - 10.72 (br, 1 H) | MS (ESI/APCI Dual): 473 (M+1), 471 (M-1) |
| 37 | 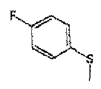 | 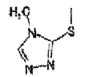 | 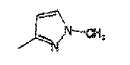 | 1H NMR (300 MHz, DMSO-D6) δ ppm 3.67 (s, 3 H) 3.81 (s. 3 H) 6.59 (d. J=2.3 Hz, 1 H) 7.21 - 7.38 (m, 2 H)7.49 - 7.82 (m, 2 H) 7-67 (d. J=2.3 Hz, 1 H) 8.28 (s. 1 H) 8.83 (s, 1 H) 10.19 - (0.47 (br. 1 H) | MS (ESI/APCI Dual): 443 (M+1). 441 (M-1) |
| 38 | 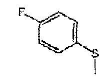 | 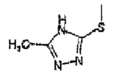 | 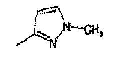 | 1H NMR (300 MHz, DMSO-D6) δ ppm 2.42 (s. 3 H) 3.80 (s. 3 H) 6.61 (d, J=2.2 Hz, 1 H) 7.11 - 7.43 (m, 2 H) 7.48 - 7.63 (m. 2 H) 7,68 (d. J=2.2 Hz, 1 H) 8.38 (s, 1 H) 10.25 - 10.67 (br. 1 H) | MS (ESI/APCI Dual): 443 (M+1), 441 (M-1) |

**[Table 1-4]**

| | | | | | |
|---|---|---|---|---|---|
| 39 | 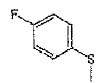 | 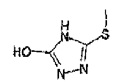 | 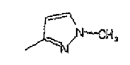 | 1 H NMR (300 MHz, DMSO-D6) δ ppm 3.81 (s, 3 H) 6.62 (d. J=2.2 Hz, 1 H) 7.19 - 7.42 (m, 2 H) 7.51 - 7.64 (m, 2 H) 7.69 (d, J=2.2 Hz, 1 H) 8.54 (s, 1 H) 10.54 - 10,74 (br, 1 H) 11.88 - 12.03 (br. 1 H) 12.05 - 12.19 (br, 1 H) | MS (ESI/APCI Dual): 445 (M+1). 443 (M-1) |
| 40 | 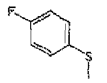 | 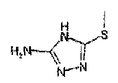 | 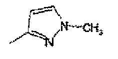 | 1H NMR (300 MHz, DMSO-D6) δ ppm 3.80 (s, 3 H) 6.18 - 6.43 (br, 2 H) 6.61 (d, J=2.2 Hz, 1 H) 7.19 - 7.38 (m, 2 H) 7.49 - 7.64 (m, 2 H) 7.68 (d. J=2.2 Hz, 1 H) 8,40 (s, 1 H) 10.59 - 10.75 (br, 1 H) | MS (ESI/APOI Dual): 444 (M+1), 442 (M-1) |
| 41 | 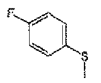 | 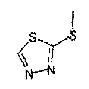 | 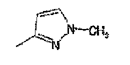 | 1H NMR (300 MHz. DMSO-d6) δ ppm 3.80 (s. 3 H) 6.62 (d, J=2.0 Hz, 1 H) 7.32 (t. J=.8.9 Hz, 2 H) 7.58 - 7.33 (m, 2 H) 7.68 (d, J=2.3 Hz, 1 H) 8.76 (s. 1 H) 9.74 (s. 1 H) 10.64 - 10.70 (br, 1 H) | MS (ESI): 446 (M+1), 444 (M-1) |
| 42 | 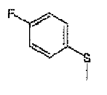 | 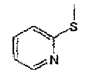 | 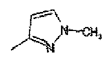 | 1H NMR (300 MHz. DMSO-D6) δ ppm 3-79 (s, 3 H) 6.61 (d, J=2.3 Hz. 1 H) 7.24 - 7.38 (m, 3 H) 7.51 (d. J=7.9 Hz, 1 H) 7.56 - 7.65 (m, 2 H) 7.67 (d. J=2.3 Hz. 1 H) 7.73 - 7,85 (m, 1 H) 8.42 - 8.52 (m. 1 H) 8.62 (s. 1 H) 10.47 - 10.56 (br, 1 H) | MS (ESI/APOI Dual): 439 (M+1) 437 (M-1) |
| 43 | 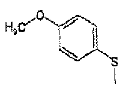 | 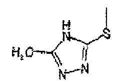 | 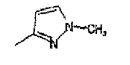 | 1H NMR (300 MHz. DMSO-D6) δ ppm 2.41 (s, 3 H) 3,80 (s, 3 H) 3.80 (s. 3 H) 6.61 (d, J=2.3 Hz, 1 H) 7.00 (d, J=8.8 Hz. 2 H) 7.42 (d, J=8.8Hz, 2 H) 7.67 (d. J=2.3 Hz, 1 H) 8.36 (s, 1 H) 10.44 - 10.48 (br, 1H) | MS (ESI/APCI Dual): 455(M+1). 453 (M-1) |
| 44 | 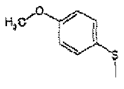 | 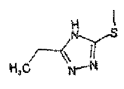 | 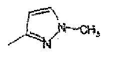 | 1H NMR (300 MHz, CDCl3) δ pom 1.36 (t. J=7.6 Hz, 3 H) 2.87 (q, J=7.6 Hz. 2 H) 3.83 (s, 3 H) 3.84 (s, 3 H) 6.90 (d. J=2.3 Hz, 1 H) 6.94 (d, J=8,8 Hz, 2 H) 7.30 (d, J=2.3 Hz, 1 H) 7.44 (d. J=8.8 Hz. 2 H) 8.32 (s. 1 H) 9.91 - 10.05 (br, 1 H) | MS (ESI/APCI Dual): 469(M+1), 407 (M-1) |
| 45 | 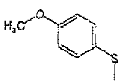 | 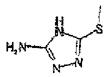 | 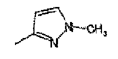 | 1H NMR (300 MHz, DMSO-D6) δ ppm 3.80 (s. 3 H) 3.80 (s, 3 H) 6.27 - 6.32 (br, 2 H) 6.61 (d. J=2.3 Hz. 1 H) 7.00 (d. J=8.8 Hz, 2 H) 7.42 (d. J=8.8 Hz, 2 H) 7.67 (d. J=2.3 Hz, 1 H) 8.39 (s, 1 H) 10.63 - 10.67 (br, 1 H) | MS (ESI/APCI Dual): 456(M+1), 454 (M-1) |
| 46 | 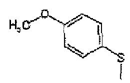 | 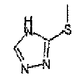 | 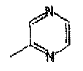 | 1H NMR (300 MHz, CDCl3) δ ppm 3.85 (s, 3 H) 8.96 (d, J=8.9 Hz. 2 H) 7.45 (d, J=8.9 Hz, 2 H) 8.33 - 9,43 (m, 4 H) 9,78 (s, 1 H) 10.05 - 10.15 (br, 1 H) | (ESI): 436 (M+1), 437 (M-1) 437 (M-1) |
| 47 | 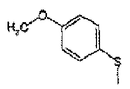 | 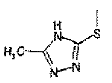 | 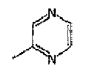 | 1H NMR (300 MHz, DMSO-d6) δ ppm 2.42 (s, 3 H) 3.80 (s. 3 H) 7.01 (d, J=8.6 Hz, 2 H) 7.44 (d, J=8.6 Hz, 2 H) 8.45 (s, 1 H) 8,49 - 8.52 (m, 2 H) 9.42 - 9.47 (br, 1 H) | MS (ESI): 453 (M+1), 451 (M-1) |
| 48 | 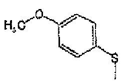 | 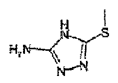 | 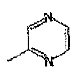 | 1H NMR (300 MHz, DMSO-d6) δ ppm 3.80 (s, 3 H) 6.31 (s, 2 H) 7.01 (d, J=8.6 Hz, 2 H) 7.44 (d, J=8.6 Hz, 2 H) 8.46 - 8.52 (m, 3 H) 9.44 (s, 1 H) 10.85 - 10.85 (br, 1 H) 12.40 - 12.60 (br. 1 H) | MS (ESI): 454 (M+1), 452 (M-1) |
| 49 | 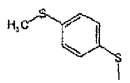 | 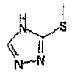 | 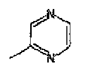 | 1H NMR (300 MHz. DMSO-d6) δ ppm 2.49 (s, 3 H) 7.29 (d, J=8.6 Hz. 2 H) 7.43 (d. J=8.3 Hz, 2 H) 8.44 (s. 1 H) 8.48 - 8.50 (m. 2 H) 8.68 - 8.83 (br. 1 H) 0.41 (d, J=1.3 Hz, 1 H) 10.57 (s, 1 H) 14.53 - 14.71 (br, 1 H) | MS (ESI): 455 (M+1), 453 (M-1) |
| 50 | 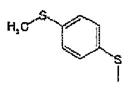 | 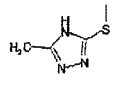 | 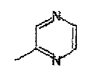 | 1H NMR (300 MHz, CDCl3) δ ppm 2.51 (s, 3 H) 2.59 (s, 3 H) 7.26 - 7.29 (m. 2 H) 7.42 - 7.46 (m, 2 H) 8.31 - 8.33 (m, 1 H) 8.39 (s, 1 H) 8.42 (d, J=2.3 Hz, 1 H) 9.77 (d. J=1.7 Hz, 1 H) 10.04 - 10,08 (br, 1 H) 10.79 - 10.91 (br. 1 H) | MS (ESI): 489 (M+1). 467 (M-1) |
| 51 | 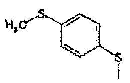 | 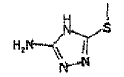 | 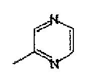 | 114 NMR (300 MHz. DMSO-d6) δ ppm 2,49 (s, 3 H) 6.29 (s, 2 H) 7.29 (d, J=7.9 Hz, 2 H) 7.42 (d, J=8,3 Hz, 2 H) 8.46 (s, 1 H) 8.48 - 8.50 (m, 2 H) 9.42 (s, 1 H) 10,64 - 10,86 (br, 1 H) 12.37 - 12.62 (br, 1 H) | MS (ESI): 470 (M+1). 468 (M-1) |

**[Table 1-5]**

| | | | | | |
|---|---|---|---|---|---|
| 52 | 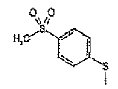 | 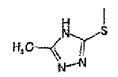 | 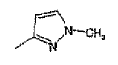 | 1H NMR (300 MHz, DMSO-d6) δ ppm 2.42 (s, 3 H) 3.27 (s, 3 H) 3.79 (s, 3 H) 6.59 (d, J=2.0 Hz, 1 H) 7.67 (d, J=2.0 Hz, 1 H) 7.77 (d, J=7.9 Hz. 2 H) 7.94 (d, J=8.3 Hz, 2 H) 8.38 (s, 1 H) 10.54 (s, 1 H) 14.15 - 14.23 (br, 1 H) | MS (ESI): 503 (M+1), 501 (M-1) |
| 53 | 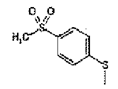 | 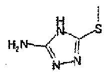 | 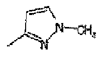 | 1H NMR (300 MHz, DMSO-d6) δ ppm 3.27 (s, 3 H) 3.79 (s, 3 H) 6.33 (s, 2 H) 6.60 (s, 1 H) 7.67 (s, 1 H) 7.77 (d, J=7.3 Hz, 2 H) 7.94 (d, J=8.3 Hz, 2 H) 8.42 (s, 1 H) 10.73 (s, 1 H) 12.51 (s, 1 H) | MS (ESI): 504 (M+1), 502 (M-1) |
| 54 | 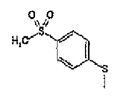 | 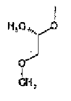 | 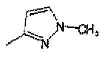 | 1H NMR (300 MHz, CDCl3) δ ppm 1.45 (d, J=6.5 Hz, 3 H) 3.07 (s, 3 H) 3.44 (s, 3 H) 3.60 (dd, J=4.1, 10.2 Hz, 1 H) 3.77 (dd, J=7.0, 10.2 Hz, 1 H) 3.81 (s, 3 H) 5.54 - 5.59 478 (m, 1 H) 6.84 (d, J=2.0 Hz, 1 H) 7.27 (s, 1 H) 7.52 - 7.55 (m, 2 H) 7.90 - 7.92 (m, 2 H) 8.34 (s, 1 H) 9.77 (s, 1 H) | MS (ESI): (M+1). 476 (M-1) |
| 55 | 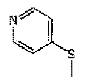 | 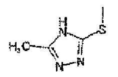 | 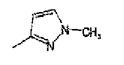 | 1H NMR (300 MHz, DMSO-d6) δ ppm 2.44 (s, 3 H) 3.80) (s. 3 H) 6.59 (d, J=2.4 Hz, 1 H) 7.52 (d, J=6.1 Hz, 2 H) 7.67 (d, J=2.0 Hz, 1 H) 8.43 (s, 1 H) 8.57 (d, J=6.1 Hz, 2 H) 10.55 - 10.63 (br, 1 H) | MS (ESI): 426 (M+1), 424 (M-1) |
| 56 | 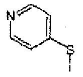 | 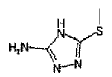 | 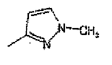 | 1H NMR (300 MHz, DMSO-d6) δ ppm 3.80 (s, 3 H) 6.25 - 6.45 (br, 2 H) 6.59 (d. J=1.7 Hz, 1 H) 7.50 (d, J=5.1 Hz, 2 H) 7.67 (s, 1 H) 8.47 (d, J=1.0 Hz. 1 H) 8.57 (d, J=5.1 Hz, 2 H) 10.68 - 10.90 (br, 1 H) | MS (ESI): 427 (M+1), 425 (M-1) |
| 57 | 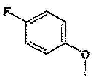 | 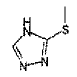 | 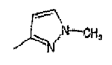 | 1H NMR (300 NHz, DMSO-D6) δ ppm 3.77 (s, 3 H) 6.58 (d, J=2.0 Hz, 1 H) 7.28 (d, J=6.5 Hz, 4 H) 7.64 (d, J=2.0 Hz, 1 H) 8.26 (s, 1 H) 8.80 - 8.76 (br, 1 H) 11.01-11.05 (br, 1H) | MS (ESI): 413 (M+1), 411 (M-1) |
| 58 | 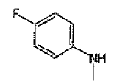 | 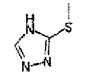 | 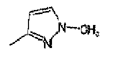 | 1H NMR (300 MHz, DMSO-D6) δ ppm 3.79 (s, 3 H) 6.59 (d, 2.5 Hz, 1 H) 7.16 - 7.22 (m, 2 H) 7.64 - 7.69 (m, 3 H) 8.48 (s, 1 H) 8.52 - 8.75 (br, 1 H) 10.28 - 10.43 (br, 1 H) 10.76 (s, 1 H) 14.32 - 14.50 (br, 1 H) | MS (ESI): 412 (M+1). 410 (M-1) |
| 59 | 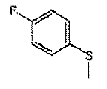 | 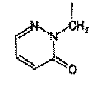 | 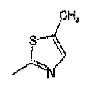 | 1H NMR (300 MHz, CDCl3) δ ppm 2.85 (d, J=6.5 Hz. 3 H) 5.46 (s, 2 H) 6.96 (dd, J=1.6, 9.4 Hz, 1 H) 7.09 - 7.32 (m, 4 H) 7.46 - 7.53 (m, 2 H) 7.81 (dd, J=1.6, 4.1 Hz, 1 H) 8.44 (s, 1 H) 10.69 - 10.81 (br, 1 H) | MS (ESI): 455 (M+1), 453 (M-1) |
| 60 | 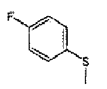 | 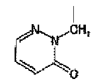 | 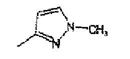 | 1H NMR (300 MHz, CDCl3) δ ppm 3.87 (s, 3 H) 5.45 (s, 2 H) 6.92 - 6.98 (m, 2 H) 7.08 - 7.15 (m, 2 H) 7.20 - 7.32 (m, 2 H) 7.48 - 7.54 (m, 2 H) 7.80 (dd, J=1.8, 3.9 Hz, 1 H) 8.37 (s, 1 H) 10.08 (s, 1 H) | MS (ESI): 438 (M+1), 436 (M-1) |
| 61 | 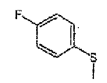 | 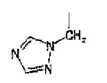 | 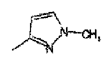 | 1H NMR (300 MHz, ODCl3) δ ppm 3.87 (s, 3 H) 5.47 (s, 2 H) 6.91 (d, J=2.0 Hz, 1 H) 7.09 - 7.15 (m, 2 H) 7.33 (d. J=2.5 Hz, 1 H) 7.47 - 7.52 (m, 2 H) 7.98 (s, 1 H) 8.26 (s, 1 H) 8.32 (s, 1 H) 9.99 (s, 1 H) | MS (ESI): 411 (M+1). 409 (M-1) |
| 62 | 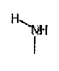 | 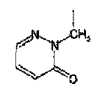 | 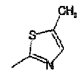 | 1H NMR (300 MHz, DMSO-D6) δ ppm 2.39 (s, 3 H), 3.32 (s, 2 H), 5.34 (s, 2 H), 7.01 (d, J=9.5 Hz, 1 H). 7.23 (s, 1 H), 7.46 (dd, J=9.5, 3.8 Hz, 1 H), 7.54-7.64 (br, 1 H), 7.96 (d, J=3.8 Hz 1 H), 8.33 (s, 1 H) | MS (ESI): 344 (M+1), 342 (M-1) |
| 63 | 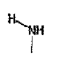 | 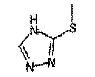 | 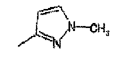 | 1H NMR (300 MHz, DMSO-D6) δ ppm 3.78 (s, 3 H) 6.55 (d. J=2.3 Hz, 1 H) 7.65 (d. J=2.3 Hz, 1 H) 7.75 - 7.83 (br, 2 H) 8.39 (s, 1 H) 8.47 - 8.61 (br, 1 H) 9.87 - 10.18 (br, 1 H) | MS (ESI/APCI Dual): 318 (M+1), 318 (M-1) |
| 64 | 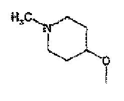 | 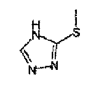 | 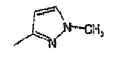 | 1H NMR (300 MHz, DMSO-D6) δ ppm 1.67 - 1.72 (m, 2 H) 1.90 - 1.97 (m, 2 H) 2.07 - 2.20 (m, 2 H) 2.13 (s, 3 H) 2.49 - 2.54 (m, 2 H) 3.75 (d, J=0.7 Hz, 3 H) 5.03 - 5.05 (m, 1 H) 6.53 (q, J=1.0 Hz, 1 H) 7.61 (d, J=1.3 Hz, 1 H) 8.33 (d, J=1.0 Hz. 1 H) 8.59 (d, J=1.0 Hz, 1 H) 10.73 (s, 1 H) | MS (ESI): 416 (M+1), 414 (M-1) |

**[Table 1-6]**

| | | | | | |
|---|---|---|---|---|---|
| 65 | 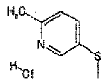 | 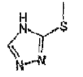 | 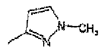 | 1H NMR (300 MHz, DMSO-D6) δ ppm 2.64 (s, 3 H) 3.81 (s, 3 H) 6.61 (d, J=2.3 Hz. 1 H) 7.59 - 7.72 (m. 2 H) 8.21 (dd, J=8.0, 2.6 Hz, 1 H) 8.37 (s, 1 H) 8.66 - 8.81 (m, 2 H) 10.51 - 10.64 (br, 1 H) | MS (ESI/APCI Dual): 429(M+1), 424 (M-1) |
| 66 | 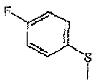 | 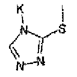 | 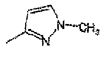 | 1H NMR (300 MHz, DMSO-d6) δ ppm 3.81 (d, J= 1.0 Hz, 3 H) 6.61 (t, J=2.0 Hz, 1H) 7.20 - 7.25 (m. 2 H) 7.48 - 7.52 (m, 2 H) 7.67 (s, 1 H) 7.60 - 7.83 (m, 2H) 10.49 - 10.64 (br, 1 H) | MS (ESI): 429 (M+1), 427 (M-1) |
| 67 | 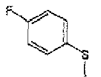 | 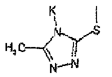 | 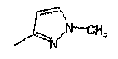 | 1H NMR (300 MHz, DMSO-d6) δ ppm 2.16 (s, 3 H) 3.79 (s, 3 H) 6.61 (d, J=2.2 Hz, 1 H) 7.12 - 7.36 (m, 2 H) 7.43 - 7.58 (m, 2 H) 7.58 - 7.70 (m, 1 H) 7.80 - 7.95 (m, 1 H) 10.31 - 10.81 (br, f H) | MS (ESI/APCI Dual): 443 (M+1), 441 (M-1) |
| 68 | 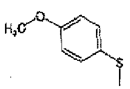 | 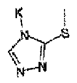 | 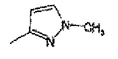 | 1H NMR (300 MHz, DMSO-d6) δ ppm 3.77 (s, 3 H) 3.81 (s, 3 H) 6.61 (d, J=2.3 Hz, 1 H) 6.91 - 7.01 (m, 2 H) 7.37 (d, J=8.9 Hz, 2 H) 7.67 (d, J=2.3 Hz, 1 H) 7.81 (s, 2 H) 10.44 -10.63 (br, 1 H) | MS (ESI/APCI Dual): 441 (M+1), 439 (M-1) |

GK activation action of the compound of the present invention can be evaluated in accordance with a known method, for example, a method described in an experimental example.

GK activation action of the compound of the present invention was measured by the method described in the following experimental example.

### (Experimental Example 1) - GK activation test -

A GK activation by a test compound was tested by partially modifying the method of Irwin A. Rose et al. (J. Biol. Chem. 1964 Jan; 239: 12-7).
The enzyme source used in this assay was human liver GK, which was allowed to express by Escherichia coli as a fusion protein having GST (Glutathione S-transferase) added to the amino terminal and purified by Glutathione Sepharose 4B (Amersham Biosciences).
The test was performed by using flat-bottom 96-well plates (Sumitomo Bakelite Co., Ltd). To each of the wells of plates, a dimethyl sulfoxide (DMSO) solution of a test compound and DMSO serving as a control were added so as to obtain a final DMSO concentration of 1%. Furthermore, 25 mM Hepes-KOH (pH = 7.1), 25 mM KCl, 2 mM MgCl₂, 2 mM ATP, 4 mM ¹⁴C-labeled glucose (Amersham Pharmacia) and 1 mM DTT (dithiothreitol) (these concentrations were final concentrations) were added. Finally, human liver GK was added so as to obtain 0.24 µg/well. The reaction was initiated and carried out at room temperature.
Twenty minutes later, AG1-X4 resin (BioRad), which was suspended with 25 mM Hepes-KOH (pH = 7.1), was added and allowed to bind a reaction product, a labeled glucose-6-phosphate. The whole quantity was transferred to a multi-screen plate (Millipore) and the resin was washed with water. Thereafter, labeled glucose-6-phosphate bound to the resin was eluted with a 0.5 M NaCl solution. The activity of the labeled glucose-6-phosphate eluted was measured and used as an index of GK activity.
Regarding the GK activity of a control group in a well in which DMSO alone was added as 100%, the GK activity activated by the test compound to a maximum was indicated as the maximum activation value (Emax value). Furthermore, the concentration of a test compound required for activating GK activity to 50% of the maximum activation value was indicated as an EC₅₀ value.
The results are shown in Table 2 below.

**[Table 2]**

| Compound No. | Emax % | EC₅₀ nM |
|---|---|---|
| 2 | 1267 | 104 |
| 5 | 1246 | 171 |
| 24 | 1130 | 110 |
| 25 | 1302 | 365 |
| 29 | 1280 | 89.5 |
| 30 | 1256 | 397 |
| 35 | 1358 | 72.4 |
| 38 | 1317 | 117 |
| 41 | 1416 | 311 |
| 46 | 1204 | 59.4 |
| 51 | 1083 | 131 |
| 52 | 883 | 128 |

### (Experimental Example 2) - Blood glucose level-lowering effect -

The effect of the compound of the present invention to decrease blood glucose level was investigated by use of SD rats.
SD rats were raised by free-feeding. From the caudal vein of 8-week old mice, blood was taken and centrifugally separated to obtain the plasma. A blood glucose level of the plasma was measured by glucose CII test Wako. The rats were divided into groups of 6 rats so as to have the same average blood glucose level. In an agent administration group, a compound suspended in a 0.5% aqueous methyl cellulose solution was orally administered to the rats; whereas, in a control group, a 0.5% aqueous methylcellulose solution was orally administered. At 30 minutes, 60 minutes, 120 minutes, 240 minutes, 360 minutes and 480 minutes after the administration, blood was taken from the caudal vein of each rat. The plasma was centrifugally recovered and a blood glucose level was measured by use of glucose CII test Wako. Furthermore, a plasma insulin concentration was measured by an ultrasensitive rat insulin measuring kit (Morinaga Institute of Biological Science, Inc.). The blood glucose levels at individual time points were plotted to obtain a graph showing a change of blood glucose level.
Regarding the blood glucose level of the 0.5% methyl cellulose administration group as 100%, the blood glucose level of SD rats administrated with Compound No. 2 was indicated. In a 3 mg/kg administration group, the blood glucose level was 59% at 1 hour, 71% at 2 hours and 76% at 4 hours. Whereas, in a 10 mg/kg administration group, the blood glucose level was 53% at 1 hour, 68% at 2 hours, and 75% at 4 hours.
Preparation Examples of the compound of the present invention will be described below.

### Preparation Example 1

Granules containing the following components are manufactured.

| Components | |
|---|---|
| Compound represented by the formula (1) | 10 mg |
| Lactose | 700 mg |
| Cornstarch | 274 mg |
| HPC-L | 16 mg |
| | 1000 mg |

A compound represented by the formula (1) and lactose are passed through a 60-mesh sieve. Cornstarch is passed through a 120 mesh sieve. These are mixed by a V-shape mixer. To the mixed powder, a low-viscosity aqueous hydroxypropyl cellulose (HPC-L) solution is added, kneaded, granulated (extruding granulation, pore size: 0.5 to 1 mm) and then dried. The resultant dried granules are passed through a shaking sieve (12/60 mesh) to obtain granules.

### Preparation Example 2

Powder for encapsulation containing the following components is manufactured.

| Component | |
|---|---|
| Compound represented by the formula (1) | 10 mg |
| Lactose | 79 mg |
| Cornstarch | 10 mg |
| Magnesium stearate | 1 mg |
| | 100 mg |

A compound represented by the formula (1) and lactose are passed through a 60-mesh sieve. Cornstarch is passed through a 120 mesh sieve. These and magnesium stearate are mixed by a V-shape mixer. Then, 10 triturated powder (100 mg) is encapsulated in a No. 5 hard gelatin capsule.

### Preparation Example 3

Granules for encapsulation containing the following components are manufactured.

| Component | |
|---|---|
| Compound represented by the formula (1) | 15 mg |
| Lactose | 90 mg |
| Cornstarch | 42 mg |
| HPC-L | 3 mg |
| | 150 mg |

A compound represented by the formula (1) and lactose are passed through a 60-mesh sieve. Cornstarch is passed through a 120 mesh sieve. These are mixed by a V-shape mixer. To the powder mixture, a low-viscosity aqueous hydroxypropyl cellulose (HPC-L) solution is added, kneaded, granulated and then dried. The resultant dried granules are passed through a shaking sieve (12/60 mesh) to obtain granules. The granules (150 mg) are encapsulated in a No. 4 hard gelatin capsule.

### Preparation Example 4

### Tablets containing the following components are manufactured.

| Component | |
|---|---|
| Compound represented by the formula (1) | 10 mg |
| Lactose | 90 mg |
| Microcrystalline cellulose | 30 mg |
| Magnesium stearate | 5 mg |
| CMC-Na | 15 mg |
| | 150 mg |

A compound represented by the formula (1), lactose, microcrystalline cellulose and CMC-Na (sodium salt of carboxymethylcellulose) were passed through a 60-mesh sieve and mixed. To the powder mixture, magnesium stearate is added to obtain powder mixture for a preparation. The powder mixture was directly tableted to obtain 150 mg tablets.

### Preparation Example 5

A preparation for intravenous administration is manufactured as follows.

| | |
|---|---|
| Compound represented by the formula (1) | 100 mg |
| Saturated fatty acid glyceride | 1000 ml |

A solution containing the above components is intravenously administered to a patient generally at a rate of 1 ml/minute.

### INDUSTRIAL APPLICABILITY

The compound of the present invention has excellent GK activation action and can provide therapeutic and prophylactic agents not only for diabetes but also for diabetes-related disorders such as obesity and hyperlipemia or for diabetic chronic complications such as retinopathy, nephropathy and arteriosclerosis.

## Claims

1. A pyrazinamide compound represented by formula (1), a tautomer or stereo isomer of the compound or a pharmaceutically acceptable salt thereof or a solvate thereof 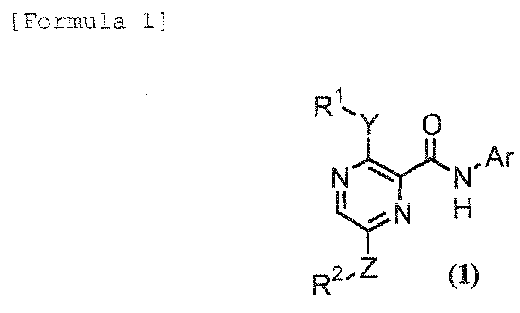 (in the formula (1),
R¹ represents a hydrogen atom, a C₁₋₈ alkyl group, a C₃₋₈ cycloalkyl group (the C₃₋₈ cycloalkyl group is unsubstituted or substituted with one hydroxy group), a C₂₋₉ heterocyclyl group (the C₂₋₉ heterocyclyl group is unsubstituted or substituted with one to three groups which may be the same or different and selected from the group consisting of a C₁₋₆ alkyl group and an oxo group), a phenyl group (the phenyl group is unsubstituted or substituted with one to three groups which may be the same or different and selected from the group consisting of a halogen atom, a hydroxy group, a trifluoromethyl group, a C₁₋₆ alkyl group, a hydoxy C₁₋₈ alkyl group, a C₁₋₆ alkoxy group, a C₁₋₆ alkylthio group, a C₁₋₆ alkylsulfonyl group, a carboxy group and a C₂₋₆ alkoxycarbonyl group), a C₁₋₉ heteroaryl group (the C₁₋₉ heteroaryl group is unsubstituted or substituted with one to three groups which may be the same or different and selected from the group consisting of a halogen atom, a C₁₋₆ alkyl group and a hydoxy C₁₋₈ alkyl group) or a C₇₋₁₄ arylalkyl group (the C₇₋₁₄ arylalkyl group is unsubstituted or substituted with one to three groups which may be the same or different and selected from the group consisting of a halogen atom, a C₁₋₆ alkyl group, a hydroxy group and a C₁₋₆ alkoxy group),
R² represents a C₁₋₈ alkyl group (the C₁₋₈ alkyl group is unsubstituted or substituted with one to three groups which may be the same or different and selected from the group consisting of a halogen atom, a hydroxy group and a C₁₋₆ alkoxy group), a C₂₋₉ heterocyclyl group (the C₂₋₉ heterocyclyl group is unsubstituted or substituted with one or two oxo groups), a phenyl group (the phenyl group is unsubstituted or substituted with one to three halogen atoms) or a C₁₋₉ heteroaryl group (the C₁₋₉ heteroaryl group is unsubstituted or substituted with one to three groups which may be the same or different and selected from the group consisting of a hydroxy group, an amino group and a C₁₋₆ alkyl group),
Ar represents a monocyclic or fused polycyclic C₁₋₉ heteroaryl group represented by formula (2) or a C₂₋₉ heterocyclyl group represented by formula (2): 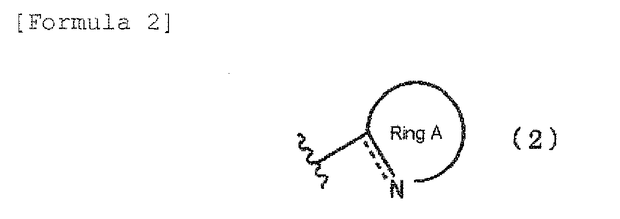 (in the formula (2), ring A is unsubstituted or substituted with one to three groups which may be the same or different and selected from the group consisting of a C₁₋₆ alkyl group, a carboy group, a carboxy C₁₋₆ alkyl group, a carbamoyl group, a carbamoyl C₁₋₆ alkyl group, a hydoxy C₁₋₈ alkyl group, a C₁₋₆ alkoxy C₁₋₈ alkyl group, a halogen atom, a C₂₋₆ alkoxycarbonyl group, a C₂₋₆ alkoxycarbonyl C₁₋₆ alkyl group and a di(C₁₋₆ alkyl)amino C₁₋₈ alkyl group),
Y represents -S-, -O- or -NH-, and
Z represents -S-, -O- or -CH₂-).

2. The pyrazinamide compound represented by the formula (1), a tautomer or stereo isomer of the compound or a pharmaceutically acceptable salt thereof or a solvate thereof according to claim 1 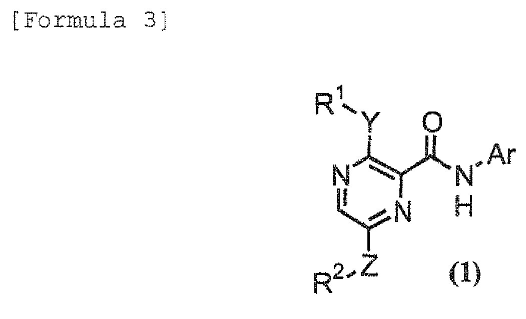 (in the formula (1),
R¹ represents a hydrogen atoms, a C₁₋₈ alkyl group, a C₃₋₈ cycloalkyl group, a C₂₋₉ heterocyclyl group (the C₂₋₉ heterocyclyl group is unsubstituted or substituted with one C₁₋₆ alkyl group), a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group or a C₇₋₁₄ arylalkyl group (the C₇₋₁₄ arylalkyl group is unsubstituted or substituted with one C₁₋₆ alkyl group),
R² represents a C₂₋₉ heterocyclyl group (the C₂₋₉ heterocyclyl group is unsubstituted or substituted with one oxo group), a phenyl group (the phenyl group is unsubstituted or substituted with one halogen atom) or a C₁₋₉ heteroaryl group (the C₁₋₉ heteroaryl group is unsubstituted or substituted with one group selected from the group consisting of a hydroxy group, an amino group and a C₁₋₆ alkyl group),
Ar represents a monocyclic C₁₋₆ heteroaryl group represented by the formula (2) or a C₂₋₉) heterocyclyl group represented by formula (2): 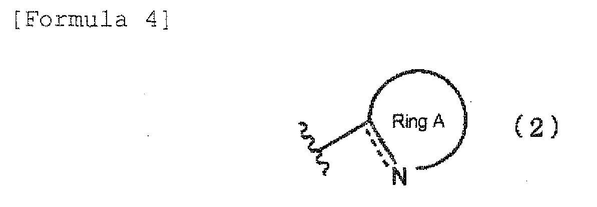 (in the formula (2), ring A is unsubstituted or substituted with one or two groups which may be the same or different and selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl group, a C₂₋₆ alkoxycarbonyl C₁₋₆ alkyl group and a di(C₁₋₆ alkyl) amino C₁₋₈ alkyl group),
represents -S-, -O- or -NH-, and
Z represents -S- or -CH₂-).

3. The pyrazinamide compound represented by the formula (1), a tautomer or stereo isomer of the compound or a pharmaceutically acceptable salt thereof or a solvate thereof according to claim 1 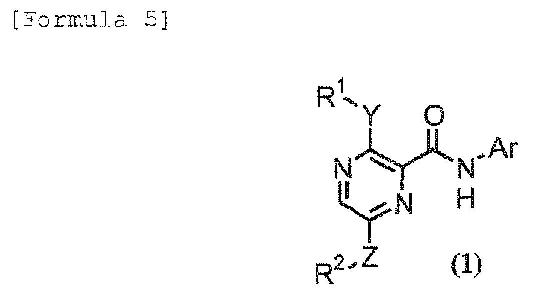 (in the formula (1),
R¹ represents a C₁₋₈ alkyl group, a C₃₋₈ cycloalkyl group, a C₂₋₉ heterocyclyl group (the C₂₋₉ heterocyclyl group is unsubstituted or substituted with one C₁₋₆ alkyl group), a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group (the C₁₋₉ heteroaryl group is unsubstituted or substituted with one C₁₋₆ alkyl group) or a C₇₋₁₄ arylalkyl group (the C₇₋₁₄ arylalkyl group is unsubstituted or substituted with one C₁₋₆ alkyl group),
R² represents a C₂₋₉ heterocyclyl group (the C₂₋₉ heterocyclyl group is unsubstituted or substituted with one oxo group), a phenyl group (the phenyl group is unsubstituted or substituted with one halogen atom) or a C₁₋₉ heteroaryl group (the C₁₋₉ heteroaryl group is unsubstituted or substituted with one group selected from the group consisting of a hydroxy group, an amino group and a C₁₋₆ alkyl group),
Ar represents a monocyclic C₁₋₆ heteroaryl group represented by formula (2) or a C₂₋₉ heterocyclyl group represented by formula (2): 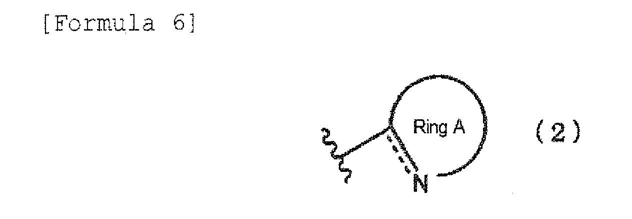 (in the formula (2), ring A is unsubstituted or substituted with one or two groups which may be the same or different and selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl group, a C₂₋₆ alkoxycarbonyl C₁₋₆ alkyl group and a di (C₁₋₆ alkyl)amino C₁₋₈ alkyl group),
Y represents -S- or -O- and
Z represents -S- or -CH₂-,
with the proviso that, when Z represents -CH₂-, the monocyclic C₁₋₆ heteroaryl group represented by the formula (2) does not represent a pyridin-2-yl group).

4. The pyrazinamide compound, a tautomer or stereo isomer of the compound or a pharmaceutically acceptable salt thereof or a solvate thereof according to claim 2, wherein R² is a C₁₋₉ heteroaryl group (the C₁₋₉ heteroaryl group is unsubstituted or substituted with one group selected from the group consisting of a hydroxy group, an amino group and a C₁₋₆ alkyl group) and Z is -S-.

5. The pyrazinamide compound, a tautomer or stereo isomer of the compound or a pharmaceutically acceptable salt thereof or a solvate thereof according to claim 3, wherein R² is a C₁₋₉ heteroaryl group (the C₁₋₉ heteroaryl group is unsubstituted or substituted with one group selected from the group consisting of a hydroxy group, an amino group and a C₁₋₆ alkyl group) and Z is -S-.

6. The pyrazinamide compound, a tautomer or stereo isomer of the compound or a pharmaceutically acceptable salt thereof or a solvate thereof according to claim 4, wherein R² is a triazolyl group (the triazolyl group is unsubstituted or substituted with one group selected from the group consisting of an amino group and a C₁₋₆ alkyl group).

7. The pyrazinamide compound, a tautomer or stereo isomer of the compound or a pharmaceutically acceptable salt thereof or a solvate thereof according to claim 5, wherein R² is a triazolyl group (the triazolyl group is unsubstituted or substituted with one group selected from the group consisting of an amino group and a C₁₋₆ alkyl group).

8. The pyrazinamide compound, a tautomer or stereo isomer of the compound or a pharmaceutically acceptable salt thereof or a solvate thereof according to claim 2, wherein R² is a 1,6-dihydropyridazinyl group (the 1,6-dihydropyridazinyl group is substituted with one oxo group) and Z is -CH₂-.

9. The pyrazinamide compound, a tautomer or stereo isomer of the compound or a pharmaceutically acceptable salt thereof or a solvate thereof according to claim 3, wherein R² is a 1,6-dihydropyridazinyl group (the 1,6-dihydropyridazinyl group is substituted with one oxo group) and Z is -CH₂-.

10. The pyrazinamide compound, a tautomer or stereo isomer of the compound or a pharmaceutically acceptable salt thereof or a solvate thereof according to any one of claims 2, 4, 6 and 8, wherein
R¹ is a C₁₋₃ alkyl group, a C₃₋₆ cycloalkyl group, a C₂₋₉ heterocyclyl group, a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group or a C₇₋₁₄ arylalkyl group (the C₇₋₁₄ arylalkyl group is unsubstituted or substituted with one C₁₋₆ alkyl group) and
Y is -S-or -NH-.

11. The pyrazinamide compound, a tautomer or stereo isomer of the compound or a pharmaceutically acceptable salt thereof or a solvate thereof according to any one of claims 3, 5, 7 and 9, wherein
R¹ is a C₁₋₈ alkyl group, a C₃₋₈ cycloalkyl group, a C₂₋₉ heterocyclyl group, a phenyl group (the phenyl group is unsubstituted or substituted with one or two groups which may be the same or different and selected from the group consisting of a halogen atom, a hydroxy group, a trifluoromethyl group, a C₁₋₆ alkyl group, a C₁₋₆ alkoxy group, a C₁₋₆ alkylthio group and a C₁₋₆ alkylsulfonyl group), a C₁₋₉ heteroaryl group (the C₁₋₉ heteroaryl group is unsubstituted or substituted with one C₁₋₆ alkyl group) or a C₇₋₁₄ arylalkyl group (the C₇₋₁₄ arylalkyl group is unsubstituted or substituted with one C₁₋₆ alkyl group), and
Y is -S-

12. The pyrazinamide compound, a tautomer or stereo isomer of the compound or a pharmaceutically acceptable salt thereof or a solvate thereof according to claim 10, wherein Y is -S-.

13. The pyrazinamide compound, a tautomer or stereo isomer of the compound or a pharmaceutically acceptable salt thereof or a solvate thereof according to any one of claims 2 to 12, wherein Ar is a monocyclic C₁₋₆ heteroaryl group represented by the formula (2) 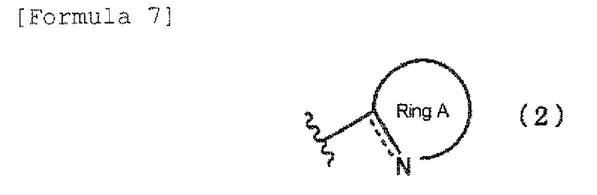 (in the formula (2), ring A is unsubstituted or substituted with one group selected from the group consisting of a C₁₋₆ alkyl group, a carboxy group, a carboxy C₁₋₆ alkyl group, a carbamoyl group, a hydoxy C₁₋₈ alkyl group, a C₁₋₆ alkoxy C₁₋₈ alkyl group, a halogen atom, a C₂₋₆ alkoxycarbonyl; group and a C₂₋₆ alkoxycarbonyl C₁₋₆ alkyl group) or a C₂₋₉ heterocyclyl group.

14. The pyrazinamide compound, a tautomer or stereo isomer of the compound or a pharmaceutically acceptable salt thereof or a solvate thereof according to claim 13, wherein
Ar is a 1H-pyrazol-3-yl group, a 1,3-thiazol-2-yl group, a pyridin-2-yl group, a pyrazin-2-yl group, an isoxazol-3-yl group (the 1H-pyrazol-3-yl group, 1,3-thiazol-2-yl group, pyridin-2-yl group, pyrazin-2-yl group and isoxazol-3-yl group are unsubstituted or substituted with one group selected from the group consisting of a C₁₋₆ alkyl group, a carboxy group, a carboxy C₁₋₆ alkyl group, a carbamoyl group, a hydroxy C₁₋₈ alkyl group, a C₁₋₆ alkoxy C₁₋₈ alkyl group, a halogen atom, a C₂₋₆ alkoxycarbonyl group and a C₂₋₆ alkoxycarbonyl C₁₋₆ alkyl group) or a 4,5-dihydro-1,3-thiazol-2-yl group.

15. The following pyrazinamide compounds, a tautomer or stereo isomer of the compound or a pharmaceutically acceptable salt thereof or a solvate thereof:
3-[(4-fluorophenyl)sulfanyl]-N-(1-methyl-1H-pyrazol-3-yl)-6-(4H-1,2,4-triazol-3-ylsulfanyl)pyrazine-2-carboxamide,
3-[(4-fluorophenyl)sulfanyl]-N-(5-methyl-1,3-thiazol-2-yl)-6-(4H-1,2,4-triazol-3-ylsulfanyl)pyrazine-2-carboxamide,
3-[(1-methylethyl)sulfanyl]-N-(1-methyl-1H-pyrazol-3-yl)-6-(4H-1,2,4-triazol-3-ylsulfanyl)pyrazine-2-carboxamide,
3-(cyclohexylsulfanyl)-N-(1-methyl-1H-pyrazol-3-yl)-6-(4H-1,2,4-triazol-3-ylsulfanyl)pyrazine-2-carboxamide,
3-[(4-methylphenyl)sulfanyl]-N-(1-methyl-1H-pyrazol-3-yl)-6-(4H-1,2,4-triazol-3-ylsulfanyl)pyrazine-2-carboxamide,
3-[(4-methoxyphenyl)sulfanyl]-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-{[4-(methylsulfanyl)phenyl]sulfanyl}-6-(4H-1,2,4-triazol-3-ylsulfanyl)pyrazine-2-carboxamide,
3-(benzylsulfanyl)-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)--3-{[4-(methylsulfonyl)phenyl]sulfanyl}-6-(4H-1,2,4-triazol-3-ylsulfanyl)pyrazine-2-carboxamide,
3-{[(1S)-1-methyl-2-phenylethyl]sulfanyl}-N-(1-methyl-1H-pyrazol-3-yl)-6-(4H-1,2,4-triazol-3-ylsulfanyl)pyrazine-2-carboxamide,
6-[({3-[(4-fluorophenyl)sulfanyl]-6-(4H-1,2,4-triazol-3-ylsulfanyl)pyrazin-2-yl}carbonyl)amino]pyridine-3-carboxylic acid methyl ester,
{3-[({3-L(4-fluorophenyl)sulfanyl]-6-(4H-1,2,4-triazol-3-ylsulfanyl)pyrazin-2-yl}carbonyl)amino]-1H-pyrazol-1-yl}acetic acid ethyl ester,
3-[(4-fluorophenyl)sulfanyl]-N-[1-(2-hydroxyethyl)-1H-pyrazol-3-yl]-6-(4H-1,2,4-triazol-3-ylsulfanyl)pyrazine-2-carboxamide,
3-[(4-fluorophenyl)sulfanyl]-N-[1-(2-methoxyethyl)-1H-pyrazol-3-yl]-6-(4H-1,2,4-triazol-3-ylsulfanyl)pyrazine-2-carboxamide,
N-(1,5-dimethyl-1H-pyrazol-3-yl)-3-[(4-fluorophenyl)sulfanyl]-6-(4H-1,2,4-triazol-3-ylsulfanyl)pyrazine-2-carboxamide,
N-(1-methyl-1H-pyrazol-3-yl)-6-(4H-1,2,4-triazol-3-ylsulfanyl)-3-{[4-(trifluoromethyl)phenyl]sulfanyl}pyrazine-2-carboxamide,
N-(1-methyl-1H-pyrazol-3-yl)-3-(tetrahydro-2H-pyran-4-ylsulfanyl)-6-(4H-1,2,4-triazol-3-ylsulfanyl)pyrazine-2-carboxamide,
3-[(3,4-dimethoxyphenyl)sulfanyl]-N-(1-methyl-1H-pyrazol-3-yl)-6-(4H-1,2,4-triazol-3-ylsulfanyl)pyrazine-2-carboxamide,
3-[(3-methoxyphenyl)sulfanyl]-N-(1-methyl-1H-pyrazol-3-yl)-6-(4H-1,2,4-triazal-3-ylsulfanyl)pyrazine-2-carboxamide,
3-[(2-methoxyphenyl)sulfanyl]-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-[(6-methylpyridin-3-yl)sulfanyl]-6-(4H-1,2,4-triazol-3-ylsulfanyl)pyrazine-2-carboxamide,
N-(1-methyl-1H-pyrazol-3-yl)-3-(pyridin-4-ylsulfanyl)-6-(4H-1,2,4-triazol-3-ylsulfanyl)pyrazine-2-carboxamide,
N-(4,5-dihydro-1,3-thiazol-2-yl)-3-[(4-fluorophenyl)sulfanyl]-6-(4H-1,2,4-triazol-3-ylsulfanyl)pyrazine-2-carboxamide,
N-(5-chloro-l,3-thiazol-2-yl)-3-[(4-fluorophenyl)sulfanyl]-6-(4H-1,2,4-triazol-3-ylsulfanyl)pyrazine-2-carboxamide,
3-[(4-fluorophenyl)sulfanyl]-N-(5-methylisoxazol-3-yl)-6-(4H-1,2,4-triazol-3-ylsulfanyl)pyrazine-2-carboxamide,
N-(5-carbamoylpyridin-2-yl)-3-[(4-fluorophenyl)sulfanyl]-6-(4H-1,2,4-triazol-3-ylsulfanyl)pyrazine-2-carboxamide,
3-[(4-fluorophenyl)sulfanyl]-N-(pyrazin-2-yl)-6-(4H-1,2,4-triazol-3-ylsulfanyl)pyrazine-2-carboxamide,
3-[(4-fluorophenyl)sulfanyl]-N-[1-(2-methoxyethyl)-1H-pyrazol-3-yl]-6-[(5-methyl-4H-1,2,4-triazol-3-yl)sulfanyl]pyrazine-2-carboxamide;
3-[(4-methoxyphenyl)sulfanyl]-N-(5-methyl-1,3-thiazol-2-yl)-6-(4H-1,2,4-triazol-3-ylsulfanyl)pyrazine-2-carboxamide,
3-[(4-hydroxyphenyl)sulfanyl]-N-(5-methyl-1,3-thiazol-2-yl)-6-(4H-1,2,4-triazol-3-ylsulfanyl)pyrazine-2-carboxamide,
6-[({3-[(4-fluorophenyl)sulfanyl]-6-(4H-1,2,4-triazol-3-ylsulfanyl)pyrazin-2-yl}carbonyl)amino]pyridine-3-carboxylic acid,
{3-[({3-[(4-fluorophenyl)sulfanyl]-6-(4H-1,2,4-triazol-3-ylsuifanyl)pyrazin-2-yl}carbonyl)amino]-1H-pyrazol-1-yl}acetic acid,
3-[(4-fluorophenyl)sulfanyl]-N-(1-methyl-1H-pyrazol-3-yl)-6-[(4-methyl-4H-1,2,4-triazol-3-yl)sulfanyl]pyrazine-2-carboxamide,
3-[(4-fluorophenyl)sulfanyl]-N-(1-methyl-1H-pyrazol-3-yl)-6-[(5-methyl-4H-1,2,4-triazol-3-yl)sulfanyl]pyrazine-2-carboxamide,
3-[(4-fluorophenyl)sulfanyl]-6-[(5-hydroxy-4H-1,2,4-triazol-3-yl)sulfanyl]-N-(1-methyl-1H-pyrazol-3-yl)pyrazine-2-carboxamide,
6-[(5-amino-4H-1,2,4-triazol-3-yl)sulfanyl]-3-[(4-fluorophenyl)sulfanyl]-N-(1-methyl-1H-pyrazol-3-yl)pyrazine-2-carboxamide,
3-[(4-fluorophenyl)sulfanyl]-N-(1-methyl-1H-pyrazol-3-yl)-6-(1,3,4-thiadiazol-2-ylsulfanyl)pyrazine-2-carboxamide,
3-[(4-fluorophenyl)sulfanyl]-N-(1-methyl-1H-pyrazol-3-yl)-6-(pyridin-2-ylsulfanyl)pyrazine-2-carboxamide,
3-[(4-methoxyphenyl)sulfanyl]-N-(1-methyl-1H-pyrazol-3-yl)-6-[(5-methyl-4H-1,2,4-triazol-3-yl)sulfanyl]pyrazine-2-carboxamide,
6-[(5-ethyl-4H-1,2,4-triazol-3-yl)sulfanyl]-3-[(4-methoxyphenyl)sulfanyl]-N-(1-methyl-1H-pyrazol-3-yl)pyrazine-2-carboxamide,
6-[(5-amino-4H-1,2,4-triazol-3-yl)sulfanyl]-3-[(4-methoxyphenyl)sulfanyl]-N-(1-methyl-1H-pyrazol-3-yl)pyrazine-2-carboxamide,
3-[(4-methoxyphenyl)sulfanyl]-N-pyrazin-2-yl-6-(4H-1,2,4-triazol-3-ylsulfanyl)pyrazine-2-carboxamide,
3-[(4-methoxyphenyl)sulfanyl]-6-[(5-methyl-4H-1,2,4-triazol-3-yl)sulfanyl]-N-pyrazin-2-ylpyrazine-2-carboxamide,
6-[(5-amino-4H-1,2,4-triazol-3-yl)sulfanyl]-3-[(4-methoxyphenyl)sulfanyl]-N-pyrazin-2-ylpyrazine-2-carboxamide,
3-([4-(methylsulfanyl)phenyl]sulfanyl)-N-pyrazin-2-yl-6-(4H-1,2,4-triazol-3-ylsulfanyl)pyrazine-2-carboxamide,
3-{[4-(methylsulfanyl)phenyl]sulfanyl}-6-[(5-methyl-4H-1,2,4-triazol1-3-yl)sulfanyl]-N-pyrazin-2-ylpyrazine-2-carboxamide,
6-[(5-amino-4H-1,2,4-triazol-3-yl)sulfanyl]-3-{[4-(methylsulfanyl)phenyl]sulfanyl}-N-pyrazin-2-ylpyrazine-2-carboxamide,
N-(1-methy-1H-pyrazol-3-yl)-3-{[4-(methylsulfonyl)phenyl]sulfanyl}-6-[(5-methyl-4H-1,2,4-triazol-3-yl)sulfanyl]pyrazine-2-carboxamide,
6-[(5-amino-4H-1,2,4-triazol-3-yl)sulfanyl]-N-(1-methyl-1H-pyrazol-3-yl)-3-{[4-(methylsulfonyl)phenyl]sulranyl}pyrazine-2-carboxamide,
N-(1-methyl-1H-pyrazol-3-yl)-6-[(5-methyl-4H-1,2,4-triazol-3-yl)sulfanyl]-3-(pyridin-4-ylsulfanyl)pyrazine-2-carboxamide,
6-[(5-amino-4H-1,2,4-triazol-3-yl)sulfanyl]-N-(1-methyl-1H-pyrazol-3-yl)-3-(pyridin-4-ylsulfanyl)pyrazine-2-carboxamide,
3-(4-fluorophenoxy)-N-(1-methyl-1H-pyrazol-3-yl)-6-(4H-1,2,4-triazol-3-ylsulfanyl)pyrazine-2-carboxamide,
3-[(4-fluorophenyl)amino]-N-(1-methyl-1H-pyrazol-3-yl)-6-(4H-1,2,4-triazol-3-ylsulfanyl)pyrazine-2-carboxamide,
3-[(4-fluorophenyl)sulfanyl]-N-(5-methyl-1,3-thiazol-2-yl)-6-[(6-oxopyridazin-1(6H)-yl)methyl]pyrazine-2-carboxamide,
3-[(4-fluorophenyl)sulfanyl]-N-(1-methyl-1H-pyrazol-3-yl)-6-[(6-oxopyridazin-1(6H)-yl)methyl]pyrazine-2-carboxamide,
3-[(4-fluorophenyl)sulfanyl]-N-(1-methyl-1H-pyrazol-3-yl)-6-(1H-1,2,4-triazol-1-yimethyl)pyrazine-2-carboxamide.

16. The following pyrazinamide compounds, a tautomer or stereo isomer of the compound or a pharmaceutically acceptable salt thereof or a solvate thereof:
3-[(4-fluorophenyl)sulfanyl]-N-(1-methyl-1H-pyrazol-3-yl)-6-(4H-1,2,4-triazol-3-ylsulfanyl)pyrazine-2-carboxamide,
3-[(4-fluorophenyl)sulfanyl]-N-(5-methyl-1,3-thiazol-2-yl)-6-(4H-1,2,4-triazol-3-ylsulfanyl)pyrazine-2-carboxamide,
3-[(1-methylethyl)sulfanyl]-N-(1-methyl-1H-pyrazol-3-yl)-6-(4H-1,2,4-triazol-3-ylsulfanyl)pyrazine-2-carboxamide,
3-(cyclohexylsulfanyl)-N-(1-methyl-1H-pyrazol-3-yl)-6-(4H-1,2,4-triazol-3-ylsulfanyl)pyrazine-2-carboxamide,
3-[(4-methylphenyl)sulfanyl]-N-(1-methyl-1H-pyrazol-3-yl)-6-(4H-1,2,4-triazol-3-ylsulfanyl)pyrazine-2-carboxamide,
3-[(4-methoxyphenyl)sulfanyl]-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-{[4-(methylsulfanyl)phenyl]sulfanyl}-6-(4H-1,2,4-triazol-3-ylsulfanyl)pyrazine-2-carboxamide,
3-(benzylsulfanyl)-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-{[4-(methylsulfonyl)phenyl]sulfanyl}-6-(4H-1,2,4-triazol-3-ylsulfanyl)pyrazine-2-carboxamide,
3-{[(1S)-1-methyl-2-phenylethyl]sulfanyl}-N-(1-methyl-1H-pyrazol-3-yl)-6-(4H-1,2,4-triazol-3-ylsulfanyl)pyrazine-2-carboxamide,
6-[({3-[(4-fluorophenyl)sulfanyl]-6-(4H-1,2,4-triazol-3-ylsulfanyl)pyrazin-2-yl}carbonyl)amino]pyridine-3-carboxylic acid methyl ester,
{3-[({3-[(4-fluorophenyl)sulfanyl]-6-(4H-1,2,4-triazol-3-ylsulfanyl)pyrazin-2-yl}carbonyl)amino]-1H-pyrazol-1-yl}acetic acid ethyl ester,
3-[(4-fluorophenyl)sulfanyl]-N-[1-(2-hydroxyethyl)-1H-pyrazol-3-yl]-6-(4H-1,2,4-triazol-3-ylsulfanyl)pyrazine-2-carboxamide,
3-[(4-fluorophenyl)sulfanyl]-N-[1-(2-methoxyethyl)-1H-pyrazol-3-yl]-6-(4H-1,2,4-triazol-3-ylsulfanyl)pyrazine-2-carboxamide,
N-(1,5-dimethyl-1H-pyrazol-3-yl)-3-[(4-fluorophenyl)sulfanyl]-6-(4H-1,2,4-triazol-3-ylsulfanyl)pyrazine-2-carboxamide,
N-(1-methyl-1H-pyrazol-3-yl)-6-(4H-1,2,4-triazol-3-ylsulfanyl)-3-{[4-(trifluoromethyl)phenyl]sulfanyl}pyrazine-2-carboxamide,
N-(1-methyl-1H-pyrazol-3-yl)-3-(tetrahydro-2H-pyran-4-ylsulfanyl)-6-(4H-1,2,4-triazol-3-ylsulfanyl)pyrazine-2-carboxamide,
3-[(3,4-dimethoxyphenyl)sulfanyl]-N-(1-methyl-1H-pyrazol-3-yl)-6-(4H-1,2,4-triazol-3-ylsulfanyl)pyrazine-2-carboxamide,
3-[(3-methoxyphenyl)sulfanyl]-N-(1-methyl-1H-pyrazol-3-yl)-6-(4H-1,2,4-triazol-3-ylsulfanyl)pyrazine-2-carboxamide,
3-[(2-methoxyphenyl)sulfanyl]-N-(1-methyl-1H-pyrazol-3-yl)-6-(4H-1,2,4-triazol-3-ylsulfanyl)pyrazine-2-carboxamide,
N-(1-methyl-1H-pyrazol-3-yl)-3-(pyridine-4-ylsulfanyl)-6-(4H-1,2,4-triazol-3-ylsulfanyl)pyrazine-2-carboxamide,
N-(4,5-dihydro-1,3-thiazol-2-yl)-3-[(4-fluorophenyl)sulfanyl]-6-(4H-1,2,4-triazol-3-ylsulfanyl)pyrazine-2-carboxamide,
N-(5-chloro-1,3-thiazol-2-yl)-3-[(4-fluorophenyl)sulfanyl]-6-(4H-1,2,4-triazol-3-ylsulfanyl)pyrazine-2-carboxamide,
3-[(4-fluorophenyl)sulfanyl]-N-(5-methylisoxazol-3-yl)-6-(4H-1,2,4-triazol-3-ylsulfanyl)pyrazine-2-carboxamide,
N-(5-carbamoylpyridin-2-yl)-3-[(4-fluorophenyl)sulfanyl]-6-(4H-1,2,4-triazol-3-ylsulfanyl)pyrazine-2-carboxamide,
3-[(4-fluorophenyl)sulfanyl]-N-(pyrazin-2-yl)-6-(4H-1,2,4-triazol-3-ylsulfanyl)pyrazine-2-carboxamide,
3-[(4-methoxyphenyl)sulfanyl]-N-(5-methyl-1,3-thiazol-2-yl)-6-(4H-1,2,4-triazol-3-ylsulfanyl)pyrazine-2-carboxamide,
3-[(4-hydroxyphenyl)sulfanyl]-N-(5-methyl-1,3-thiazol-2-yl)-6-(4H-1,2,4-triazol-3-ylsulfanyl)pyrazine-2-carboxamide,
6-[({3-[(4-fluorophenyl)sulfanyl]-6-(4H-1,2,4-triazol-3-ylsulfanyl)pyrazin-2-yl}carbonyl)amino]pyridine-3-carboxylic acid,
{3-[({3-[(4-fluorophenyl)sulfanyl]-6-(4H-1,2,4-triazol-3-ylsulfanyl)pyrazin-2-yl}carbonyl)amino]-1H-pyrazol-1-yl}acetic acid,
3-[(4-fluorophenyl)sulfanyl]-N-(1-methyl-1H-pyrazol-3-yl)-6-[(4-methyl-4H-1,2,4-triazol-3-yl)sulfanyl]pyrazine-2-carboxamide,
3-[(4-fluorophenyl)sulfanyl]-N-(1-methyl-1H-pyrazol-3-yl)-6-[(5-methyl-4H-1,2,4-triazol-3-yl)sulfanyl]pyrazine-2-carboxamide,
3-[(4-fluorophenyl)sulfanyl]-6-[(5-hydoxy-4H-1,2,4-triazol-3-yl)sulfanyl]-N-(1-methyl-1H-pyrazol-3-yl)pyrazine-2-carboxamide,
6-[(5-amino-4H-1,2,4-triazol-3-yl)sulfanyl]-3-[(4-fluorophenyl)sulfanyl]-N-(1-methyl-1H-pyrazol-3-yl)pyrazine-2-carboxamide,
3-[(4-fluarophenyl)sulfanyl]-N-(1-methyl-1H-pyrazol-3-yl)-6-(pyridin-2-ylsulfanyl)pyrazine-2-carboxamide,
3-(4-fluorophenoxy)-N-(1-methyl-1H-pyrazol-3-yl)-6-(4H-1,2,4-triazol-3-ylsulfanyl)pyrazine-2-carboxamide,
3-[(4-fluorophenyl)amino]-N-(1-methyl-1H-pyrazol-3-yl)-6-(4H-1,2,4-triazol-3-ylsulfanyl)pyrazine-2-carboxamide,
3-[(4-fluorophenyl)sulfanyl]-N-(5-methyl-1,3-thiazol-2-yl)-6-[(6-oxopyridazin-1(6H)-yl)methyl]pyrazine-2-carboxamide,
3-[(4-fluorophenyl)sulfanyl]-N-(1-methyl-1H-pyrazol-3-yl)-6-[(6-oxopyrldazin-1(6H)-yl)methyl]pyrazine-2-carboxamide,
3-[(4-fluorophenyl)sulfanyl]-N-(1-methyl-1H-pyrazol-3-yl)-6-(1H-1,2,4-triazol-1-ylmethyl)pyrazine-2-carboxamide.

17. A pharmaceutical having, as an active ingredient, the pyrazinamide compound, a tautomer or stereo isomer of the compound or a pharmaceutically acceptable salt thereof or a solvate thereof according to any one of claims 1 to 16.

18. The pharmaceutical according to claim 17 for preventing or treating a disorder or state that may be improved by a glucokinase activating effect.

19. The pharmaceutical according to claim 18 which is a prophylactic/therapeutic agent for diabetes or obesity.
